# EUROPEAN PATENT APPLICATION

(11) **EP 2 322 626 A1**
(43) Date of publication of application: **18.05.2011**
(21) Application number: 10180900.2
(22) Date of filing: 18.09.2002
(51) Int. Cl.: C12N 15/48, C12N 15/09, C12N 15/49, A61P 31/18, C12N 15/869

(54) **HIV-GAG codon-optimised DNA vaccines**

(30) Priority: 11.12.2001 GB 0129604; 19.03.2002 GB 0206462; 20.09.2001 WO PCT/GB01/04207
(62) Divisional of application: 02798748.6
(71) Applicant: Glaxo Group Limited, Middlesex UB6 0NN (GB)
(72) Inventor: Beaton, Andrew, Stevenage, Hertfordshire SG1 2NY (GB); Ertl, Peter, Franz, Stevenage, Hertfordshire SG1 2NY (GB); Gough, Gerald Wayne, Stevenage, Hertfordshire SG1 2NY (GB); Lear, Andrew, Stevenage, Hertfordshire SG1 2NY (GB); Tite, John, Philip, Stevenage, Hertfordshire SG1 2NY (GB); Van Wely, Catherine, Ann, Stevenage, Hertfordshire SG1 2NY (GB)
(74) Representative: Lonergan, Nerissa Elizabeth

(57) **Abstract**

The invention provides a nucleotide sequence that encodes an HIV-1 gag protein or fragment thereof containing a gag epitope, an HIV-1 Nef protein or a fragment containing a Nef epitope thereof, and an RT protein or a fragment containing an RT epitope thereof, operably linked to a heterologous promoter, wherein the order of the sequence is Gag, RT, Nef.

## Description

### Field of the Invention

The present invention relates to nucleic acid constructs, host cells comprising such constructs and their use in nucleic acid vaccines. The invention further relates to vaccine formulations comprising such constructs and the use of such formulations in medicine. The invention in particular relates to DNA vaccines that are useful in the prophylaxis and treatment of HIV infections, more particularly when administered by particle mediated delivery.

### Background to the Invention

HIV-1 is the primary cause of the acquired immune deficiency syndrome (AIDS) which is regarded as one of the world's major health problems. Although extensive research throughout the world has been conducted to produce a vaccine, such efforts thus far have not been successful.

Non-envelope proteins of HIV-1 have been described and include for example internal structure proteins such as the products of the gag and pol genes and, other non-structural proteins such as Rev, Nef, Vif and Tat (Green et al., New England J. Med, 324, 5, 308 et seq (1991) and Bryant et al. (Ed. Pizzo), Pediatr. Infect. Dis. J., 11, 5, 390 et seq (1992).

The Gag gene is translated from the full-length RNA to yield a precursor polyprotein which is subsequently cleaved into 3 - 5 capsid proteins; the matrix protein, capsid protein and nucleic acid binding protein and protease. (1. Fundamental Virology, Fields BN, Knipe DM and Howley M 1996 2. Fields Virology vol 2 1996).

The gag gene gives rise to the 55-kilodalton (kD) Gag precursor protein, also called p55, which is expressed from the unspliced viral mRNA. During translation, the N terminus of p55 is myristoylated, triggering its association with the cytoplasmic aspect of cell membranes. The membrane-associated Gag polyprotein recruits two copies of the viral genomic RNA along with other viral and cellular proteins that triggers the budding of the viral particle from the surface of an infected cell. After budding, p55 is cleaved by the virally encoded protease (a product of the pol gene) during the process of viral maturation into four smaller proteins designated MA (matrix [p17]), CA (capsid [p24]), NC (nucleocapsid [p9]), and p6.(4)

In addition to the 3 major Gag protein, all Gag precursors contain several other regions, which are cleaved out and remain in the virion as peptides of various sizes. These proteins have different roles e.g. the p2 protein has a proposed role in regulating activity of the protease and contributes to the correct timing of proteolytic processing.

The MA polypeptide is derived from the N-terminal, myristoylated end of p55. Most MA molecules remain attached to the inner surface of the virion lipid bilayer, stabilizing the particle. A subset of MA is recruited inside the deeper layers of the virion where it becomes part of the complex which escorts the viral DNA to the nucleus.(5) These MA molecules facilitate the nuclear transport of the viral genome because a karyophilic signal on MA is recognized by the cellular nuclear import machinery. This phenomenon allows HIV to infect nondividing cells, an unusual property for a retrovirus.

The p24 (CA) protein forms the conical core of viral particles. Cyclophilin A has been demonstrated to interact with the p24 region of p55 leading to its incorporation into HIV particles. The interaction between Gag and cyclophilin A is essential because the disruption of this interaction by cyclosporine A inhibits viral replication.

The NC region of Gag is responsible for specifically recognizing the so-called packaging signal of HIV. The packaging signal consists of four stem loop structures located near the 5' end of the viral RNA, and is sufficient to mediate the incorporation of a heterologous RNA into HIV-1 virions. NC binds to the packaging signal through interactions mediated by two zinc-finger motifs. NC also facilitates reverse transcription.

The p6 polypeptide region mediates interactions between p55 Gag and the accessory protein Vpr, leading to the incorporation of Vpr into assembling virions. The p6 region also contains a so-called late domain which is required for the efficient release of budding virions from an infected cell

The Pol gene encodes two proteins containing the two activities needed by the virus in early infection, the RT and the integrase protein needed for integration of viral DNA into cell DNA. The primary product of Pol is cleaved by the virion protease to yield the amino terminal RT peptide which contains activities necessary for DNA synthesis (RNA and DNA directed DNA polymerase, ribouclease H) and carboxy terminal integrase protein.

HIV RT is a heterodimer of full-length RT (p66) and a cleavage product (p51) lacking the carboxy terminal Rnase integrase domain.

RT is one of the most highly conserved proteins encoded by the retroviral genome. Two major activities of RT are the DNA Pol and Ribonuclease H. The DNA Pol activity of RT uses RNA and DNA as templates interchangeably and like all DNA polymerases known is unable to initiate DNA synthesis de novo, but requires a pre existing molecule to serve as a primer (RNA).

The Rnase H activity inherent in all RT proteins plays the essential role early in replication of removing the RNA genome as DNA synthesis proceeds. It selectively degrades the RNA from all RNA - DNA hybrid molecules. Structurally the polymerase and ribo H occupy separate, non-overlapping domains with the Pol covering the amino two thirds of the Pol.

The p66 catalytic subunit is folded into 5 distinct subdomains. The amino terminal 23 of these have the portion with RT activity. Carboxy term to these is the Rnase H Domain.

After infection of the host cell, the retroviral RNA genome is copied into linear ds DNA by the reverse transcriptase that is present in the infecting particle. The integrase (reviewed in Skalka AM '99 Adv in Virus Res 52 271-273) recognises the ends of the viral DNA, trims them and accompanies the viral DNA to a host chromosomal site to catalyse integration. Many sites in the host DNA can be targets for integration. Although the integrase is sufficient to catalyse integration in vitro, it is not the only protein associated with the viral DNA in vivo - the large protein - viral DNA complex isolated from the infected cells has been denoted the pre integration complex. This facilitates the acquisition of the host cell genes by progeny viral genomes.

The integrase is made up of 3 distinct domains, the N terminal domain, the catalytic core and the c terjminal domain. The catalytic core domain contains all of the requirements for the chemistry of polynucleotidyl transfer.

The Nef protein is known to cause the removal of CD4, the HIV receptor, from the cell surface, but the biological importance of this function is debated. Additionally Nef interacts with the signal pathway of T cells and induces an active state, which in turn may promote more efficient gene expression. Some HIV isolates have mutations in this region, which cause them not to encode functional protein and are severely compromised in their replication and pathogenesis in vivo.

DNA vaccines usually consist of a bacterial plasmid vector into which is inserted a strong promoter, the gene of interest which encodes for an antigenic peptide and a polyadenylation/transcriptional termination sequences. The gene of interest may encode a full protein or simply an antigenic peptide sequence relating to the pathogen, tumour or other agent which is intended to be protected against. The plasmid can be grown in bacteria, such as for example E.coli and then isolated and prepared in an appropriate medium, depending upon the intended route of administration, before being administered to the host. Following administration the plasmid is taken up by cells of the host where the encoded peptide is produced. The plasmid vector will preferably be made without an origin of replication which is functional in eukaryotic cells, in order to prevent plasmid replication in the mammalian host and integration within chromosomal DNA of the animal concerned.

There are a number of advantages of DNA vaccination relative to traditional vaccination techniques. First, it is predicted that because of the proteins which are encoded by the DNA sequence are synthesised in the host, the structure or conformation of the protein will be similar to the native protein associated with the disease state. It is also likely that DNA vaccination will offer protection against different strains of a virus, by generating cytotoxic T lymphcyte response that recognise epitopes from conserved proteins. Furthermore, because the plasmids are taken up by the host cells where antigenic protein can be produced, a long-lasting immune response will be elicited. The technology also offers the possibility of combing diverse immunogens into a single preparation to facilitate simultaneous immunisation in relation to a number of disease states.

Helpful background information in relation to DNA vaccination is provided in Donnelly et al "DNA vaccines" Ann. Rev Immunol. 1997 15: 617-648, the disclosure of which is included herein in its entirety by way of reference.

### Summary of the Invention

The present invention provides novel constructs for use in nucleic acid vaccines for the prophylaxis and treatment of HIV infections and AIDS.

Accordingly, in a first aspect, there is provided a nucleic acid molecule comprising a nucleotide sequence encoding HIV gag protein or fragment thereof linked to a nucleotide sequence encoding a further HIV antigen or fragment thereof and operably linked to a heterologous promoter. The fragment of said nucleotide sequence will encode an HIV epitope and typically encode a peptide of at least 8 amino acids. The nucleotide sequence is preferably a DNA sequence and is preferably contained within a plasmid without an origin of replication. Such nucleic acid molecules are formulated with pharmaceutically acceptable excipient, carriers, diluents or adjuvants to produce pharmaceutical composition suitable for the treatment and/or prophylaxis of HIV infection and AIDS.

In a preferred embodiment the DNA sequence is formulated onto the surface of inert particles or beads suitable for particle mediated drug delivery. Preferably the beads are gold.

In a preferred embodiment of the invention there is provided a DNA sequence that highly expressed codes for gag protein which sequence is optimised to resemble the codon usage of genes in mammalian cells. In particular, the gag protein is optimised to resemble that of highly expressed human genes.

The DNA code has 4 letters (A, T, C and G) and uses these to spell three letter "codons" which represent the amino acids the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilisation of codons may be markedly different in a single species between genes which are expressed at high and low levels. This bias is different in viruses, plants, bacteria and mammalian cells, and some species show a stronger bias away from a random codon selection than others. For example, humans and other mammals are less strongly biased than certain bacteria or viruses. For these reasons, there is a significant probability that a mammalian gene expressed in E.coli or a foreign or recombinant gene expressed in mammalian cells will have an inappropriate distribution of codons for efficient expression. It is believed that the presence in a heterologous DNA sequence of clusters of codons or an abundance of codons which are rarely observed in the host in which expression is to occur, is predictive of low heterologous expression levels in that host.

In an embodiment of the present invention provides a gag polynucleotide sequence which encodes an amino acid sequence, wherein the codon usage pattern of the polynucleotide sequence resembles that of highly expressed mammalian genes. Preferably the polynucleotide sequence is a DNA sequence. Desirably the codon usage pattern of the polynucleotide sequence is typical of highly expressed human genes.

In the polynucleotides of the present invention, the codon usage pattern is altered from that typical of human immunodeficiency viruses to more closely represent the codon bias of the target organism, e.g. a mammal, especially a human. The "codon usage coefficient" is a measure of how closely the codon pattern of a given polynucleotide sequence resembles that of a target species. Codon frequencies can be derived from literature sources for the highly expressed genes of many species (see e.g. Nakamura et.al. Nucleic Acids Research 1996, 24:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the anti-log). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

According to the present invention, the codon usage pattern of the polynucleotide will preferably exclude codons with an RSCU value of less than 0.2 in highly expressed genes of the target organism. Alternatively, the codon usage pattern will exclude codons representing <10% of the codons used for a particular amino acid. A relative synonymous codon usage (RSCU) value is the observed number of codons divided by the number expected if all codons for that amino acid were used equally frequently. A polynucleotide of the present invention will generally have a codon usage coefficient (or RSCU) for highly expressed human genes of greater than 0.3, preferably greater than 0.4, most preferably greater than 0.5 Codon usage tables for human can also be found in Genebank.

In comparison, a highly expressed beta actin gene has a RSCU of 0.747. The codon usage table for a homo sapiens is set out below:

**Codon Usage Table 1:**

| ***Homo sapiens* [gbpri]: 27143 CDS's (12816923 codons)** | | | | | | | |
|---|---|---|---|---|---|---|---|
| fields: [triplet] [frequency: **per thousand**] ([number]) | | | | | | | |
| | | | | | | | |
| UUU | 17.0(217684) | UCU | 14.8(189419) | UAU | 12.1(155645) | UGU | 10.0(127719) |
| UUC | 20.5(262753) | UCC | 17.5(224470) | UAC | 15.8(202481) | UGC | 12.3(157257) |
| UUA | 7.3 (93924) | UCA | 11.9(152074) | UAA | 0.7 ( 9195) | UGA | 1.3( 16025) |
| UUG | 12.5(159611) | UCG | 4.5 ( 57572) | UAG | 0.5 ( 6789) | UGG | 12.9(165930) |
| | | | | | | | |
| CUU | 12.8(163707) | CCU | 17.3(222146) | CAU | 10.5(134186) | CGU | 4.6( 59454) |
| CUC | 19.3(247391) | CCC | 20.0(256235) | CAC | 14.9(190928) | CGC | 10.8(137865) |
| CUA | 7.0 ( 89078) | CCA | 16.7(214583) | CAA | 12.0(153590) | CGA | 6.3( 80709) |
| CUG | 39.7(509096) | CCG | 7.0 ( 89619) | CAG | 34.5(441727) | CGG | 11.6(148666) |
| | | | | | | | |
| AUU | 15.8(202844) | ACU | 12.9(165392) | AAU | 17.0(218508) | AGU | 12.0(154442) |
| AUC | 21.6(277066) | ACC | 19.3(247805) | AAC | 19.8(253475) | AGC | 19.3(247583) |
| AUA | 7.2( 92133) | ACA | 14.9(191518) | AAA | 24.0(308123) | AGA | 11.5(147264) |
| AUG | 22.3(285776) | ACG | 6.3( 80369) | AAG | 32.6(418141) | AGG | 11.3(145276) |
| GUU | 10.9(139611) | GCU | 18.5(236639) | GAU | 22.4(286742) | GGU | 10.8(138606) |
| GUC | 14.6(187333) | GCC | 28.3(362086) | GAC | 26.1(334158) | GGC | 22.7(290904) |
| GUA | 7.0( 89644) | GCA | 15.9(203310) | GAA | 29.1(373151) | GGA | 16.4(210643) |
| GUG | 28.8(369006) | GCG | 7.5( 96455) | GAG | 40.2(515485) | GGG | 16.4(209907) |

Coding GC 52.51% 1st letter GC 56.04% 2nd letter GC 42.35% 3rd letter GC 59.13%

**Codon Usage Table 2 (preferred):**

| Codon usage for human (highly expressed) genes 1/24/91 (human_high.cod) | | | | |
|---|---|---|---|---|
| AmAcid | Codon | Number | /1000 | Fraction .. |
| | | | | |
| Gly | GGG | 905.00 | 18.76 | 0.24 |
| Gly | GGA | 525.00 | 10.88 | 0.14 |
| Gly | GGT | 441.00 | 9.14 | 0.12 |
| Gly | GGC | 1867.00 | 38.70 | 0.50 |
| | | | | |
| Glu | GAG | 2420.00 | 50.16 | 0.75 |
| Glu | GAA | 792.00 | 16.42 | 0.25 |
| Asp | GAT | 592.00 | 12.27 | 0.25 |
| Asp | GAC | 1821.00 | 37.75 | 0.75 |
| | | | | |
| Val | GTG | 1866.00 | 38.68 | 0.64 |
| Val | GTA | 134.00 | 2.78 | 0.05 |
| Val | GTT | 198.00 | 4.10 | 0.07 |
| Val | GTC | 728.00 | 15.09 | 0.25 |
| | | | | |
| Ala | GCG | 652.00 | 13.51 | 0.17 |
| Ala | GCA | 488.00 | 10.12 | 0.13 |
| Ala | GCT | 654.00 | 13.56 | 0.17 |
| Ala | GCC | 2057.00 | 42.64 | 0.53 |
| | | | | |
| Arg | AGG | 512.00 | 10.61 | 0.18 |
| Arg | AGA | 298.00 | 6.18 | 0.10 |
| Ser | AGT | 354.00 | 7.34 | 0.10 |
| Ser | AGC | 1171.00 | 24.27 | 0.34 |
| | | | | |
| Lys | AAG | 2117.00 | 43.88 | 0.82 |
| Lys | AAA | 471.00 | 9.76 | 0.18 |
| Asn | AAT | 314.00 | 6.51 | 0.22 |
| Asn | AAC | 1120.00 | 23.22 | 0.78 |
| | | | | |
| Met | ATG | 1077.00 | 22.32 | 1.00 |
| Ile | ATA | 88.00 | 1.82 | 0.05 |
| Ile | ATT | 315.00 | 6.53 | 0.18 |
| Ile | ATC | 1369.00 | 28.38 | 0.77 |
| | | | | |
| Thr | ACG | 405.00 | 8.40 | 0.15 |
| Thr | ACA | 373.00 | 7.73 | 0.14 |
| Thr | ACT | 358.00 | 7.42 | 0.14 |
| Thr | ACC | 1502.00 | 31.13 | 0.57 |
| | | | | |
| Trp | TGG | 652.00 | 13.51 | 1.00 |
| End | TGA | 109.00 | 2.26 | 0.55 |
| Cys | TGT | 325.00 | 6.74 | 0.32 |
| Cys | TGC | 706.00 | 14.63 | 0.68 |
| | | | | |
| End | TAG | 42.00 | 0.87 | 0.21 |
| End | TAA | 46.00 | 0.95 | 0.23 |
| Tyr | TAT | 360.00 | 7.46 | 0.26 |
| Tyr | TAC | 1042.00 | 21.60 | 0.74 |
| | | | | |
| Leu | TTG | 313.00 | 6.49 | 0.06 |
| Leu | TTA | 76.00 | 1.58 | 0.02 |
| Phe | TTT | 336.00 | 6.96 | 0.20 |
| Phe | TTC | 1377.00 | 28.54 | 0.80 |
| | | | | |
| Ser | TCG | 325.00 | 6.74 | 0.09 |
| Ser | TCA | 165.00 | 3.42 | 0.05 |
| Ser | TCT | 450.00 | 9.33 | 0.13 |
| Ser | TCC | 958.00 | 19.86 | 0.28 |
| | | | | |
| Arg | CGG | 611.00 | 12.67 | 0.21 |
| Arg | CGA | 183.00 | 3.79 | 0.06 |
| Arg | CGT | 210.00 | 4.35 | 0.07 |
| Arg | CGC | 1086.00 | 22.51 | 0.37 |
| | | | | |
| GIn | CAG | 2020.00 | 41.87 | 0.88 |
| GIn | CAA | 283.00 | 5.87 | 0.12 |
| His | CAT | 234.00 | 4.85 | 0.21 |
| His | CAC | 870.00 | 18.03 | 0.79 |
| | | | | |
| Leu | CTG | 2884.00 | 59.78 | 0.58 |
| Leu | CTA | 166.00 | 3.44 | 0.03 |
| Leu | CTT | 238.00 | 4.93 | 0.05 |
| Leu | CTC | 1276.00 | 26.45 | 0.26 |
| | | | | |
| Pro | CCG | 482.00 | 9.99 | 0.17 |
| Pro | CCA | 456.00 | 9.45 | 0.16 |
| Pro | CCT | 568.00 | 11.77 | 0.19 |
| Pro | CCC | 1410.00 | 29.23 | 0.48 |

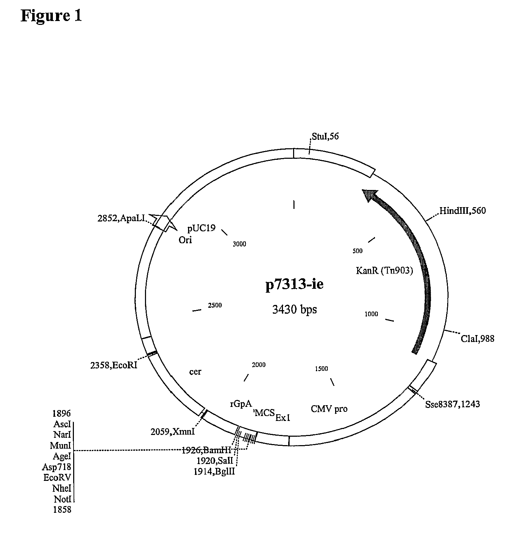

According to a further aspect of the invention, an expression vector is provided which comprises and is capable of directing the expression of a polynucleotide sequence according to the first aspect of the invention, in particular the codon usage pattern of the gag polynucleotide sequence is typical of highly expressed mammalian genes, preferably highly expressed human genes. The vector may be suitable for driving expression of heterologous DNA in bacterial insect or mammalian cells, particularly human cells. In one embodiment, the expression vector is p7313 (see figure 1).

In a third embodiment there is provided a gag gene under the control of a heterologous promoter fused to a DNA sequence encoding NEF, a fragment thereof, or HIV Reverse Transcriptase (RT) or fragment thereof. The gag portion of the gene may be either the N or C terminal portion of the fusion.

In a preferred embodiment, the gag gene does not encode the gag p6 peptide. Preferably the NEF gene is truncated to remove the sequence encoding the N terminal region i.e. removal of 30-85, preferably 60-85, typically about 81, preferably the N terminal 65 amino acids.

In a further embodiment the RT gene is also optimised to resemble a highly expressed human gene. The RT preferably encodes a mutation to substantially inactivate any reverse transcriptase activity. A preferred inactivation mutation involves the substitution of W tryptophan 229 for K (lysine).

According to a further aspect of the invention, a host cell comprising a polynucleotide sequence according to the invention, or an expression vector according to the invention is provided. The host cell may be bacterial, e.g. E.coli, mammalian, e.g. human, or may be an insect cell. Mammalian cells comprising a vector according to the present invention may be cultured cells transfected in vitro or may be transfected in vivo by administration of the vector to the mammal.

The present invention further provides a pharmaceutical composition comprising a polynucleotide sequence according to the invention. Preferably the composition comprises a DNA vector. In preferred embodiments the composition comprises a plurality of particles, preferably gold particles, coated with DNA comprising a vector encoding a polynucleotide sequence of the invention. Preferably the sequence encodes an HIV gag amino acid sequence, wherein the codon usage pattern of the polynucleotide sequence is typical of highly expressed mammalian genes, particularly human genes. In alternative embodiments, the composition comprises a pharmaceutically acceptable excipient and a DNA vector according to the second aspect of the present invention. The composition may also include an adjuvant.

Thus it is an embodiment of the invention that the vectors of the invention be utilised with immunostimulatory agents. Preferably the immunostimulatory agent are administered at the same time as the nucleic acid vector of the invention and in preferred embodiments are formulated together. Such immunostimulatory agents include, but this list is by no means exhaustive and does not preclude other agents: synthetic imidazoquinolines such as imiquimod [S-26308, R-837], (Harrison, et al. 'Reduction of recurrent HSV disease using imiquimod alone or combined with a glycoprotein vaccine', Vaccine 19: 1820-1826, (2001)); and resiquimod [S-28463, R-848] (Vasilakos, et al.' Adjuvant activites of immune response modifier R-848: Comparison with CpG ODN', Cellular immunology 204: 64-74 (2000).), Schiff bases of carbonyls and amines that are constitutively expressed on antigen presenting cell and T-cell surfaces, such as tucaresol (Rhodes, J. et al. ' Therapeutic potentiation of the immune system by costimulatory Schiff-base-forming drugs', Nature 377: 71-75 (1995)), cytokine, chemokine and co-stimulatory molecules as either protein or peptide, this would include pro-inflammatory cytokines such as GM-CSF, IL-1 alpha, IL-1 beta, TGF- alpha and TGF - beta, Th1 inducers such as interferon gamma, IL-2, IL-12, IL-15 and IL-18, Th2 inducers such as IL-4, IL-5, IL-6, IL-10 and IL-13 and other chemokine and co-stimulatory genes such as MCP-1, MIP-1 alpha, MIP-1 beta, RANTES, TCA-3, CD80, CD86 and CD40L, , other immunostimulatory targeting ligands such as CTLA-4 and L-selectin, apoptosis stimulating proteins and peptides such as Fas, (49), synthetic lipid based adjuvants, such as vaxfectin, (Reyes et al., 'Vaxfectin enhances antigen specific antibody titres and maintains Th1 type immune responses to plasmid DNA immunization', Vaccine 19: 3778-3786) squalene, alpha- tocopherol, polysorbate 80, DOPC and cholesterol, endotoxin, [LPS], Beutler, B., 'Endotoxin, 'Toll-like receptor 4, and the afferent limb of innate immunity', Current Opinion in Microbiology 3: 23-30 (2000)) ; CpG oligo- and di-nucleotides, Sato, Y. et al., 'Immunostimulatory DNA sequences necessary for effective intradermal gene immunization', Science 273 (5273): 352-354 (1996). Hemmi, H. et al., 'A Toll-like receptor recognizes bacterial DNA', Nature 408: 740-745, (2000) and other potential ligands that trigger Toll receptors to produce Th1 ― inducing cytokines, such as synthetic Mycobacterial lipoproteins, Mycobacterial protein p19, peptidoglycan, teichoic acid and lipid A.

Certain preferred adjuvants for eliciting a predominantly Th1-type response include, for example, a Lipid A derivative such as monophosphoryl lipid A, or preferably 3-de-O-acylated monophosphoryl lipid A. MPL^{®} adjuvants are available from Corixa Corporation (Seattle, WA; *see,* for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins.

Also provided are the use of a polynucleotide according to the invention, or of a vector according to the invention, in the treatment or prophylaxis of an HIV infection.

The present invention also provides methods of treating or preventing HIV infections, any symptoms or diseases associated therewith, comprising administering an effective amount of a polynucleotide, a vector or a pharmaceutical composition according to the invention. Administration of a pharmaceutical composition may take the form of one or more individual doses, for example as repeat doses of the same DNA plasmid, or in a "prime-boost" therapeutic vaccination regime. In certain cases the "prime" vaccination may be via particle mediated DNA delivery of a polynucleotide according to the present invention, preferably incorporated into a plasmid-derived vector and the "boost" by administration of a recombinant viral vector comprising the same polynucleotide sequence, or boosting with the protein in adjuvant. Conversly the priming may be with the viral vector or with a protein formulation typically a protein formulated in adjuvant and the boost a DNA vaccine of the present invention. Multiple doses of prime and/or boost may be employed.

In embodiments of the invention fragments of gag, nef or RT proteins are contemplated. For example, a polynucleotide of the invention may encode a fragment of an HIV gag, nef or RT protein. A polynucleotide which encodes a fragment of at least 8, for example 8-10 amino acids or up to 20, 50, 60, 70, 80, 100, 150 or 200 amino acids in length is considered to fall within the scope of the invention as long as the encoded oligo or polypeptide demonstrates HIV antigenicity. In particular, but not exclusively, this aspect of the invention encompasses the situation when the polynucleotide encodes a fragment of a complete HIV protein sequence and may represent one or more discrete epitopes of that protein. Such fragments may be codon optimised such that the fragment has a codon usage pattern which resembles that of a highly expressed mammalian gene.

Preferred constructs according to the present invention include:
1. p17, p24, fused to truncated NEF (devoid of nucleotides encoding terminal amino-acids 1-65)
2. p17, p24, RT, truncated NEF (devoid of nucleotides encoding terminal amino-acids 1-65)
3. p17, p24 (optimised gag) truncated NEF (devoid of nucleotides encoding terminal amino-acids 1-65)
4. p17, p24 (optimised gag) RT (optimised) truncated NEF (devoid of nucleotides encoding terminal amino-acids 1-85)
5. p17, p24, RT (optimised) truncated NEF (devoid of nucleotides encoding terminal amino-acids 1-65)
6. Truncated NEF - (devoid of nucleotide 1-65) fused to optimised p17, p24 gag.
7. Particularly preferred constructs of the invention include triple fusions RT-NEF-Gag, and RT-Gag-Nef particularly:
8. Optimised RT, truncated NEF and optimised P17, p24 (gag) (RNG)
and
9. Optimised RT, optimised p17, 24 (gag), Nef truncate (devoid of aa 1-65)RGN

It is preferred that the HIV constructs are derived from an HIV Clade B or Clade C, particularly clade B.

As discussed above, the present invention includes expression vectors that comprise the nucleotide sequences of the invention. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Other suitable vectors would be apparent to persons skilled in the art. By way of further example in this regard we refer to Sambrook et al. Molecular Cloning: a Laboratory Manual. 2nd Edition. CSH Laboratory Press. (1989) .

Preferably, a polynucleotide of the invention, or for use in the invention in a vector, is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

The vectors may be, for example, plasmids, artificial chromosomes (e.g. BAC, PAC, YAC), virus or phage vectors provided with a origin of replication, optionally a promoter for the expression of the polynucleotide and optionally a regulator of the promoter. The vectors may contain one or more selectable marker genes, for example an ampicillin or kanamycin resistance gene in the case of a bacterial plasmid or a resistance gene for a fungal vector. Vectors may be used *in vitro,* for example for the production of DNA or RNA or used to transfect or transform a host cell, for example, a mammalian host cell e.g. for the production of protein encoded by the vector. The vectors may also be adapted to be used *in vivo,* for example in a method of DNA vaccination or of gene therapy.

Promoters and other expression regulation signals may be selected to be compatible with the host cell for which expression is designed. For example, mammalian promoters include the metallothionein promoter, which can be induced in response to heavy metals such as cadmium, and the β-actin promoter. Viral promoters such as the SV40 large T antigen promoter, human cytomegalovirus (CMV) immediate early (IE) promoter, rous sarcoma virus LTR promoter, adenovirus promoter, or a HPV promoter, particularly the HPV upstream regulatory region (URR) may also be used. All these promoters are well described and readily available in the art.

A preferred promoter element is the CMV immediate early promoter, devoid of intron A but including exon 1. The promoter element may be the minimal promoter element or the enhanced promoter, the enhanced promoter being preferred. Accordingly there is provided a vector comprising a polynucleotide of the invention under the control of HCMV IE early promoter.

Examples of suitable viral vectors include herpes simplex viral vectors, vaccinia or alpha-virus vectors and retroviruses, including lentiviruses, adenoviruses and adeno-associated viruses. Gene transfer techniques using these viruses are known to those skilled in the art. Retrovirus vectors for example may be used to stably integrate the polynucleotide of the invention into the host genome, although such recombination is not preferred. Replication-defective adenovirus vectors by contrast remain episomal and therefore allow transient expression. Vectors capable of driving expression in insect cells (for example baculovirus vectors), in human cells, in yeast or in bacteria may be employed in order to produce quantities of the HIV protein encoded by the polynucleotides of the present invention, for example for use as subunit vaccines or in immunoassays.

The polynucleotides according to the invention have utility in the production by expression of the encoded proteins, which expression may take place *in vitro, in* vivo or ex *vivo.* The nucleotides may therefore be involved in recombinant protein synthesis, for example to increase yields, or indeed may find use as therapeutic agents in their own right, utilised in DNA vaccination techniques. Where the polynucleotides of the present invention are used in the production of the encoded proteins *in vitro* or ex *vivo,* cells, for example in cell culture, will be modified to include the polynucleotide to be expressed. Such cells include transient, or preferably stable mammalian cell lines. Particular examples of cells which may be modified by insertion of vectors encoding for a polypeptide according to the invention include mammalian HEK293T, CHO, HeLa, 293 and COS cells. Preferably the cell line selected will be one which is not only stable, but also allows for mature glycosylation and cell surface expression of a polypeptide. Expression may be achieved in transformed oocytes. A polypeptide may be expressed from a polynucleotide of the present invention, in cells of a transgenic non-human animal, preferably a mouse. A transgenic non-human animal expressing a polypeptide from a polynucleotide of the invention is included within the scope of the invention.

The invention further provides a method of vaccinating a mammalian subject which comprises administering thereto an effective amount of such a vaccine or vaccine composition. Most preferably, expression vectors for use in DNA vaccines, vaccine compositions and immunotherapeutics will be plasmid vectors.

DNA vaccines may be administered in the form of "naked DNA", for example in a liquid formulation administered using a syringe or high pressure jet, or DNA formulated with liposomes or an irritant transfection enhancer, or by particle mediated DNA delivery (PMDD). All of these delivery systems are well known in the art. The vector may be introduced to a mammal for example by means of a viral vector delivery system.

The compositions of the present invention can be delivered by a number of routes such as intramuscularly, subcutaneously, intraperitonally, intravenously or mucosally.

In a preferred embodiment, the composition is delivered intradermally. In particular, the composition is delivered by means of a gene gun particularly particle bombardment administration techniques which involve coating the vector on to a bead (eg gold) which are then administered under high pressure into the epidermis; such as, for example, as described in Haynes et al, J Biotechnology 44: 37-42 (1996).

In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide, are accelerated to high speed within a helium gas jet generated by a hand held device, propelling the particles into a target tissue of interest, typically the skin.

The particles are preferably gold beads of a 0.4 - 4.0 **µ**m, more preferably 0.6 - 2.0 **µ**m diameter and the DNA conjugate coated onto these and then encased in a cartridge or cassette for placing into the "gene gun".

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

The vectors which comprise the nucleotide sequences encoding antigenic peptides are administered in such amount as will be prophylactically or therapeutically effective. The quantity to be administered, is generally in the range of one picogram to 1 milligram, preferably 1 picogram to 10 micrograms for particle-mediated delivery, and 100 nanograms to 1 milligram, preferably 10 micrograms to 1 milligram, for other routes, of nucleotide per dose. The exact quantity may vary considerably depending on the weight of the patient being immunised and the route of administration,

It is possible for the immunogen component comprising the nucleotide sequence encoding the antigenic peptide, to be administered on a once off basis or to be administered repeatedly, for example, between 1 and 7 times, preferably between 1 and 4 times, at intervals between about 1 day and about 18 months. However, this treatment regime will be significantly varied depending upon the size the patient concerned, the amount of nucleotide sequence administered, the route of administration, and other factors which would be apparent to a skilled veterinary or medical practitioner. The patient may receive one or more other anti HIV retroviral drugs as part of their overall treatment regime. Additionally the nucleic acid immunogen may be administered with an adjuvant.

The adjuvant component specified herein can similarly be administered via a variety of different administration routes, such as for example, via the oral, nasal, pulmonary, intramuscular, subcutaneous, intradermal or topical routes. Preferably, the adjuvant component is administered via the intradermal or topical routes. Most preferably by the topical route. This administration may take place between about 14 days prior to and about 14 days post administration of the nucleotide sequence, preferably between about 1day prior to and about 3 days post administration of the nucleotide sequence. The adjuvant component is, in an embodiment, administered substantially simultaneously with the administration of the nucleotide sequence. By "substantially simultaneous" what is meant is that administration of the adjuvant component is preferably at the same time as administration of the nucleotide sequence, or if not, at least within a few hours either side of nucleotide sequence administration. In the most preferred treatment protocol, the adjuvant component will be administered substantially simultaneously to administration of the nucleotide sequence. Obviously, this protocol can be varied as necessary, in accordance with the type of variables referred to above. It is preferred that the adjuvant is a 1H - imidazo [4,5c] quinoline - 4 - amine derivative such as imiquimod. Typically imiquimod will be presented as a topical cream formulation and will be administered according to the above protocol.

Once again, depending upon such variables, the dose of administration of the derivative will also vary, but may, for example, range between about 0.1mg per kg to about 100mg per kg, where "per kg" refers to the body weight of the mammal concerned. This administration of the 1H-imidazo [4,5-c]quinolin-4-amine derivative would preferably be repeated with each subsequent or booster administration of the nucleotide sequence. Most preferably, the administration dose will be between about 1mg per kg to about 50mg per kg. In the case of a "prim-boost" scheme as described herein, the imiquimod or other 1H-imidazo[4,5-c]quinolin-4-amine derivative may be administered with either the prime or the boost or with both the prime and the boost.

While it is possible for the adjuvant component to comprise only 1H-imidazo[4,5-c]quinolin-4-amine derivatives to be administered in the raw chemical state, it is preferable for administration to be in the form of a pharmaceutical formulation. That is, the adjuvant component will preferably comprise the 1H-imidazo[4,5-c]quinolin-4-amine combined with one or more pharmaceutically acceptable carriers, and optionally other therapeutic ingredients. The carrier(s) must be "acceptable" in the sense of being compatible with other ingredients within the formulation, and not deleterious to the recipient thereof. The nature of the formulations will naturally vary according to the intended administration route, and may be prepared by methods well known in the pharmaceutical art. All methods include the step of bringing into association a 1H-imidazo[4,5-c]quinolin-4-amine derivative with an appropriate carrier or carriers. In general, the formulations are prepared by uniformly and intimately bringing into association the derivative with liquid carriers or finely divided solid carriers, or both, and then, if necessary, shaping the product into the desired formulation. Formulations of the present invention suitable for oral administration may be presented as discrete units such as capsules, cachets or tablets each containing a pre-determined amount of the active ingredient; as a powder or granules; as a solution or a suspension in an aqueous liquid or a non-aqueous liquid; or as an oil-in-water liquid emulsion or a water-in-oil emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent.

The tablets may optionally be coated or scored and may be formulated so as to provide slow or controlled release of the active ingredient.

Formulations for injection via, for example, the intramuscular, intraperitoneal, or subcutaneous administration routes include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilised) condition requiring only the addition of the sterile liquid carrier, for example, water for injections, immediately prior to use. Extemporaneous injection solutions and suspensions may be prepared from sterile powders, granules and tablets of the kind previously described. Formulations suitable for pulmonary administration via the buccal or nasal cavity are presented such that particles containing the active ingredient, desirably having a diameter in the range of 0.5 to 7 microns, are delivered into the bronchial tree of the recipient. Possibilities for such formulations are that they are in the form of finely comminuted powders which may conveniently be presented either in a piercable capsule, suitably of, for example, gelatine, for use in an inhalation device, or alternatively, as a self-propelling formulation comprising active ingredient, a suitable liquid propellant and optionally, other ingredients such as surfactant and/or a solid diluent. Self-propelling formulations may also be employed wherein the active ingredient is dispensed in the form of droplets of a solution or suspension. Such self-propelling formulations are analogous to those known in the art and may be prepared by established procedures. They are suitably provided with either a manually-operable or automatically functioning valve having the desired spray characteristics; advantageously the valve is of a metered type delivering a fixed volume, for example, 50 to 100 µL, upon each operation thereof.

In a further possibility, the adjuvant component may be in the form of a solution for use in an atomiser or nebuliser whereby an accelerated airstream or ultrasonic agitation is employed to produce a find droplet mist for inhalation.

Formulations suitable for intranasal administration generally include presentations similar to those described above for pulmonary administration, although it is preferred for such formulations to have a particle diameter in the range of about 10 to about 200 microns, to enable retention within the nasal cavity. This may be achieved by, as appropriate, use of a powder of a suitable particle size, or choice of an appropriate valve. Other suitable formulations include coarse powders having a particle diameter in the range of about 20 to about 500 microns, for administration by rapid inhalation through the nasal passage from a container held close up to the nose, and nasal drops comprising about 0.2 to 5% w/w of the active ingredient in aqueous or oily solutions. In one embodiment of the invention, it is possible for the vector which comprises the nucleotide sequence encoding the antigenic peptide to be administered within the same formulation as the 1H-imidazo[4,5-c]quinolin-4-amine derivative. Hence in this embodiment, the immunogenic and the adjuvant component are found within the same formulation.

In an embodiment the adjuvant component is prepared in a form suitable for gene-gun administration, and is administered via that route substantially simultaneous to administration of the nucleotide sequence. For preparation of formulations suitable for use in this manner, it may be necessary for the 1H-imidazo[4,5-c]quinolin-4-amine derivative to be lyophilised and adhered onto, for example, gold beads which are suited for gene-gun administration.

In an alternative embodiment, the adjuvant component may be administered as a dry powder, via high pressure gas propulsion.

Even if not formulated together, it may be appropriate for the adjuvant component to be administered at or about the same administration site as the nucleotide sequence.

Other details of pharmaceutical preparations can be found in Remington's Pharmaceutical Sciences, Mack Publishing Company, Easton, Pennysylvania (1985), the disclosure of which is included herein in its entirety, by way of reference.

Suitable techniques for introducing the naked polynucleotide or vector into a patient also include topical application with an appropriate vehicle. The nucleic acid may be administered topically to the skin, or to mucosal surfaces for example by intranasal, oral, intravaginal or intrarectal administration. The naked polynucleotide or vector may be present together with a pharmaceutically acceptable excipient, such as phosphate buffered saline (PBS). DNA uptake may be further facilitated by use of facilitating agents such as bupivacaine, either separately or included in the DNA formulation. Other methods of administering the nucleic acid directly to a recipient include ultrasound, electrical stimulation, electroporation and microseeding which is described in US-5,697,901.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectam and transfectam. The dosage of the nucleic acid to be administered can be altered.

A nucleic acid sequence of the present invention may also be administered by means of specialised delivery vectors useful in gene therapy. Gene therapy approaches are discussed for example by Verme et al, Nature 1997, 389:239-242. Both viral and non-viral vector systems can be used. Viral based systems include retroviral, lentiviral, adenoviral, adeno-associated viral, herpes viral, Canarypox and vaccinia-viral based systems. Non-viral based systems include direct administration of nucleic acids, microsphere encapsulation technology (poly(lactide-co-glycolide) and, liposome-based systems. Viral and non-viral delivery systems may be combined where it is desirable to provide booster injections after an initial vaccination, for example an initial "prime" DNA vaccination using a non-viral vector such as a plasmid followed by one or more "boost" vaccinations using a viral vector or non-viral based system. Similarly the invention contemplates prime boot systems with the polynucleotide of the invention, followed by boosting with protein in adjuvant or vice versa.

A nucleic acid sequence of the present invention may also be administered by means of transformed cells. Such cells include cells harvested from a subject. The naked polynucleotide or vector of the present invention can be introduced into such cells *in vitro* and the transformed cells can later be returned to the subject. The polynucleotide of the invention may integrate into nucleic acid already present in a cell by homologous recombination events. A transformed cell may, if desired, be grown up *in vitro* and one or more of the resultant cells may be used in the present invention.

Cells can be provided at an appropriate site in a patient by known surgical or microsurgical techniques (e.g. grafting, micro-injection, etc.)

The pharmaceutical compositions of the present invention may include adjuvant compounds, as detailed above, or other substances which may serve to increase the immune response induced by the protein which is encoded by the DNA. These may be encoded by the DNA, either separately from or as a fusion with the antigen, or may be included as non-DNA elements of the formulation. Examples of adjuvant-type substances which may be included in the formulations of the present invention include ubiquitin, lysosomal associated membrane protein (LAMP), hepatitis B virus core antigen, FLT3-ligand (a cytokine important in the generation of professional antigen presenting cells, particularly dentritic cells) and other cytokines such as IFN-γ and GMCSF. Other preferred adjuvants include Imiquimod and Resimquimod and Tucarasol. Imiquimod being particularly preferred.

The present invention in a preferred embodiments of the invention provides the use of a nucleic acid molecule as herein described for the treatment or prophylaxis of HIV infection. The nucleic acid molecule is preferably administered with Imiquimod. The Imiquimod is preferably administered topically, whereas the nucleic acid molecule is preferably administered by means of the particle mediated delivery.

Accordingly the present invention provides a method of treating a subject suffering from or susceptible to HIV infection, comprising administering a nucleic acid molecule as herein described and Imiquimod.

The present invention will now be described by reference to the following examples:

### EXAMPLES

### Example 1: Optimisation of p55 gag (p17, p24, p13) to resemble codon usage of highly expressed human genes.

### Gene of interest

A synthetic gene coding for the p55gag antigen of the HIV-1 clade B strain HXB2 (GenBank entry K03455), optimised for expression in mammalian cells was assembled from overlapping oligonucleotides by PCR.

Optimisation involved changing the codon usage pattern of the viral gene to give a codon frequency closer to that found in highly expressed human genes. Codons were assigned using a statistical Visual Basic program called Syngene (an updated version of Calcgene, written by R.S. Hale and G. Thompson, Protein Expression and Purification Vol. 12 pp 185-188, 1998)

### Cloning:

The 1528bp gag PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

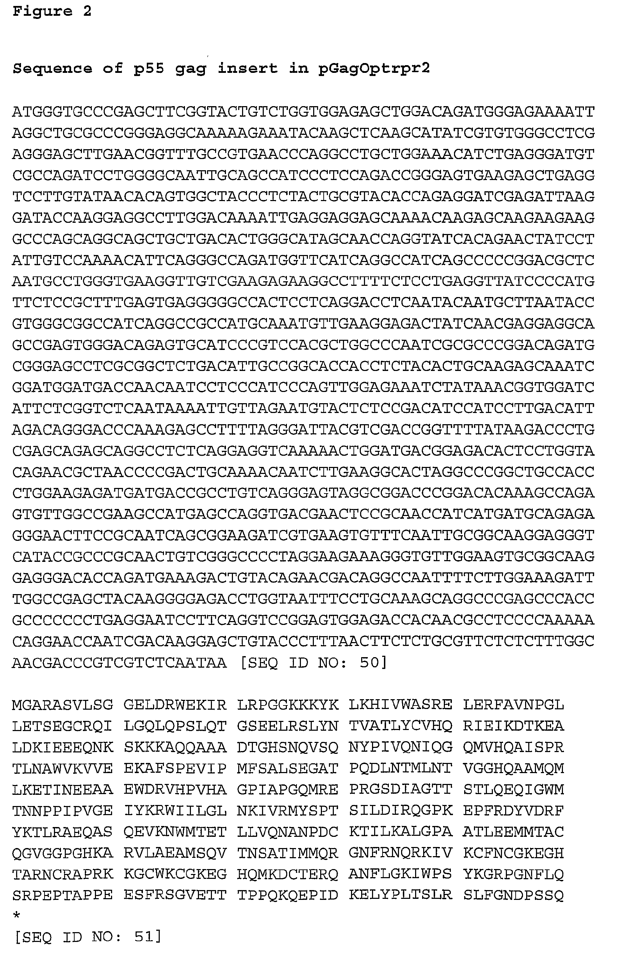

Clones were sequenced and checked for errors. No single clone was 100% correct. Regions of correct sequence from two clones were therefore combined by overlapping PCR using appropriate combinations of the optimisation oligo set to give a full length codon optimised gag gene. This final clone was subsequently found to contain a single nucleotide deletion which resulted in a frame shift and premature termination of translation. The deletion was repaired by cutting out the region of the gene containing the incorrect sequence and cloning in the correct sequence from the equivalent region of another clone. This gave the final codon optimised p55 gag clone: Gagoptrpr2. (See figure 2)

### Example 2: Production of a p17/p24 truncated Nef fusion gene

### Gene of interest

The p17 and p24 portions of the p55gag gene derived from the HIV-1 clade B strain HXB2 was PCR amplified from the plasmid pHXB?Pr (B.Maschera, E Furfine and E.D. Blair 1995 J.Virol 69 5431-5436). pHXB?Pr. 426bp from the 3' end of the HXB2 nef gene were amplified from the same plasmid. Since the HXB2 nef gene contains a premature termination codon two overlapping PCRs were used to repair the codon (TGA [stop] to TGG [Trp])

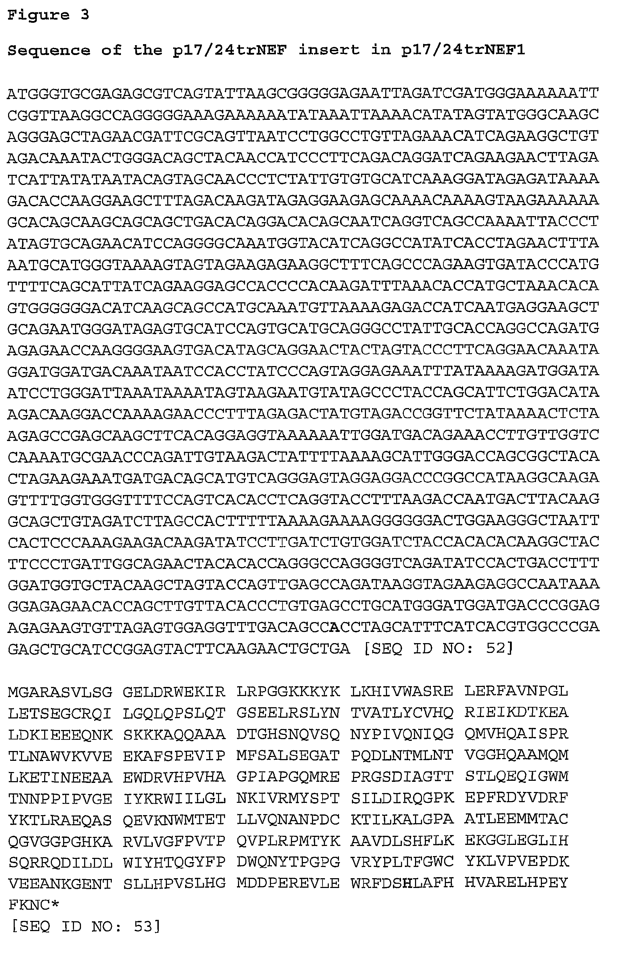

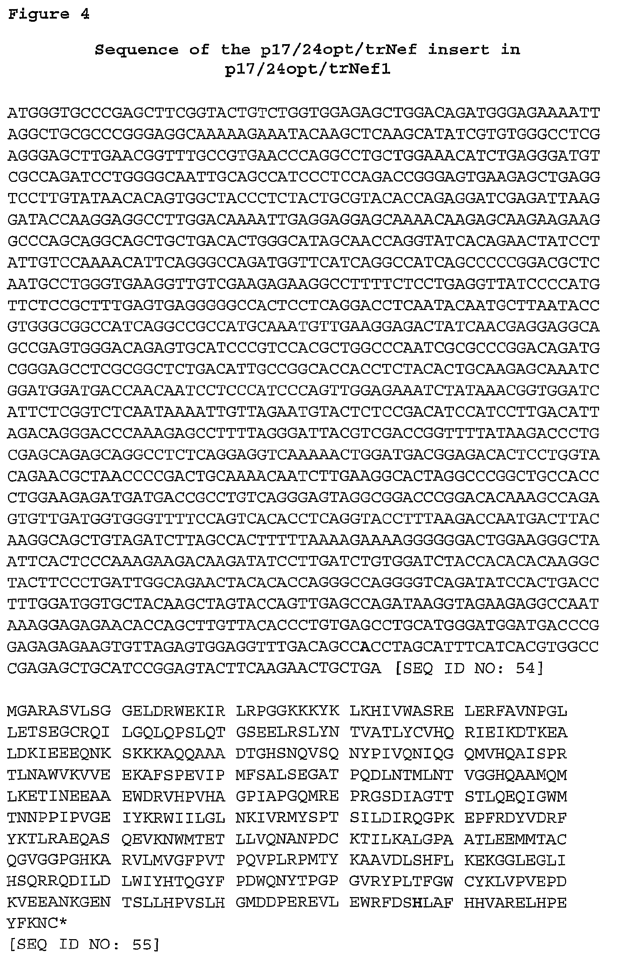

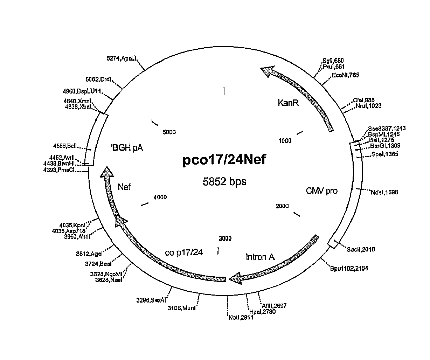

The p17/p241inker and trNEFlinker PCR products were joined to form the p17p24trNEF fusion gene (figure 3) in a PCR reaction (**antisense**)

The 1542bp product was gel purified, cut with restriction endonucleases NotI and BamHI and cloned into the NotI BamHI sites of vector WRG7077. This places the gene between the CMV promoter/intron A and the Bovine growth hormone polyadenylation signal.

### Example 3: Production of an Gag p17/24opt/trNef1 ('Gagopt/Nef') fusion gene.

### Gene of interest

The p17/p24 portion of the codon optimised p55gag gene derived from the HIV-1 clade B strain HXB2 was PCR amplified from the plasmid pGagOPTrpr2. The truncated HXB2 Nef gene with the premature termination codon repaired (TGA [stop] to TGG [Trp]) was amplified by PCR from the plasmid 7077trNef20. The two PCR products were designed to have overlapping ends so that the two genes could be joined in a second PCR.

The 1544bp product was gel purified, cut with restriction endonucleases NotI and BamHI and cloned (see figures) into the NotI BamHI sites of vector WRG7077. This places the gene between the CMV promoter/intron A and the Bovine growth hormone polyadenylation signal.

### Example 4: Plasmid: p7077-RT3 Clone #A

### Gene of interest:

A synthetic gene coding for the RT portion of the pol gene of HIV-1 clade B strain HXB2, optimised for expression in mammalian cells assembled from overlapping oligonucleotides by PCR. The sequence cloned is equivalent to positions 2550-4222 of the HXB2 reference sequence (GenBank entry K03455). To ensure expression the cloned sequence has two additional codons at the 5' end not present in the original gene - AUG GGC (Met Gly).

Optimisation involved changing the codon usage pattern of the viral gene to give a codon frequency closer to that found in highly expressed human genes, but excluding rarely used codons. Codons were assigned using a statistical Visual Basic program called Syngene (an updated version of Calcgene, written by R.S. Hale and G. Thompson, Protein Expression and Purification Vol. 12 pp 185-188, 1998)

The final clone was constructed from two intermediate clones, # 16 and #21.

### Cloning:

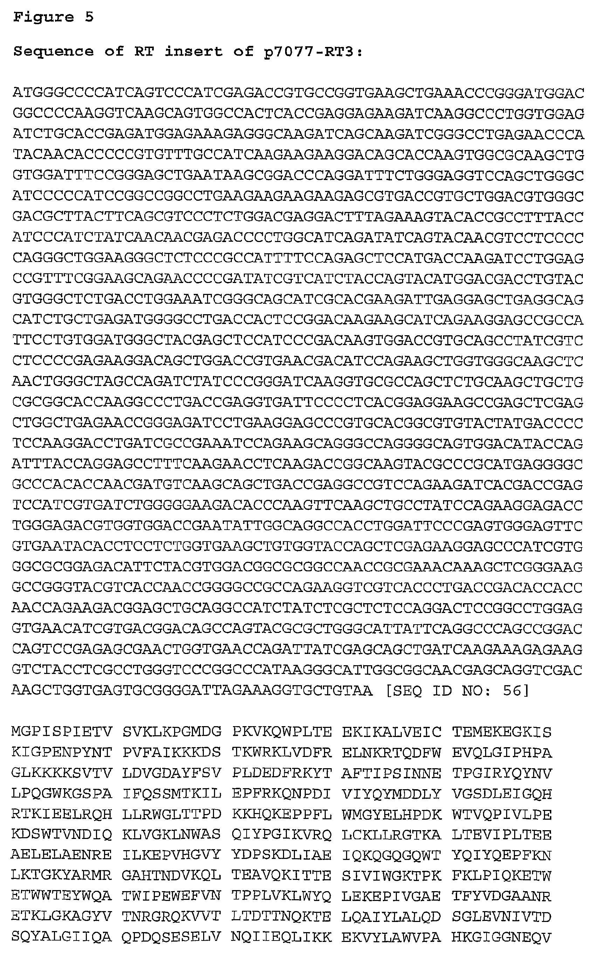

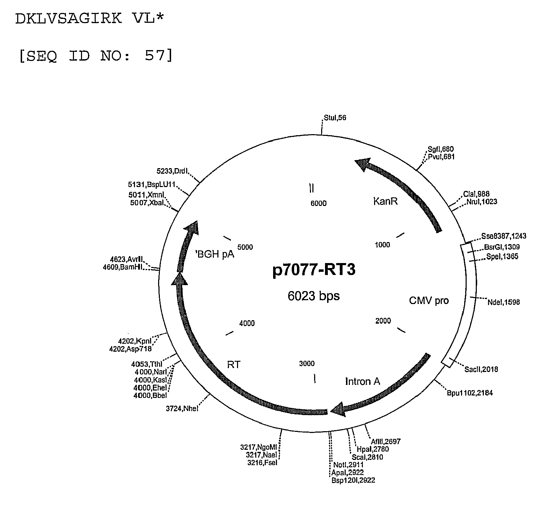

The 1.7kb PCR products were gel purified, cut with NotI and BamHI and PCR cleaned, before being ligated with NotI / BamHI cut pWRG7077. This places the gene between the CMV promoter and bovine growth hormone polyadenylation signal. Clones were sequenced. No clone was 100% correct, but clone #16 was corrected by replacing the 403bp KpnI-BamHI fragment containing 3 errors with a correct Kpn1-BamHI fragment from clone#21. The final clone was verified by sequencing. (see figure 5)

### Example 5: Optimised RT

### Gene of interest

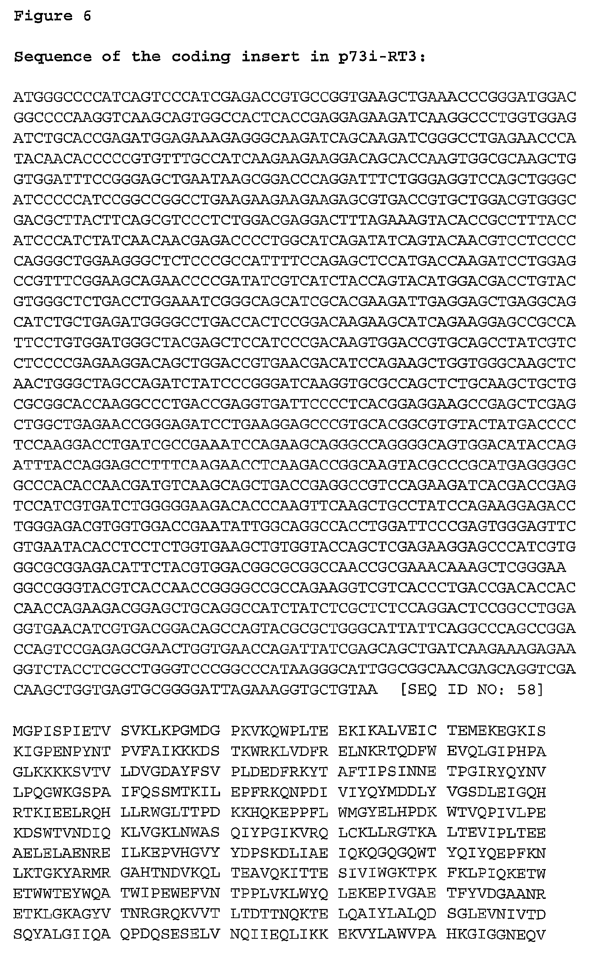

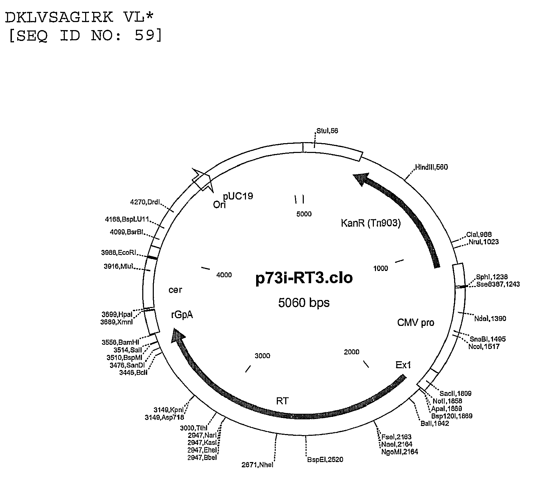

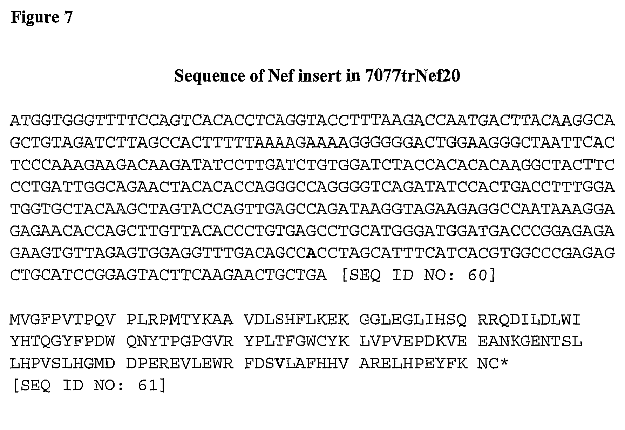

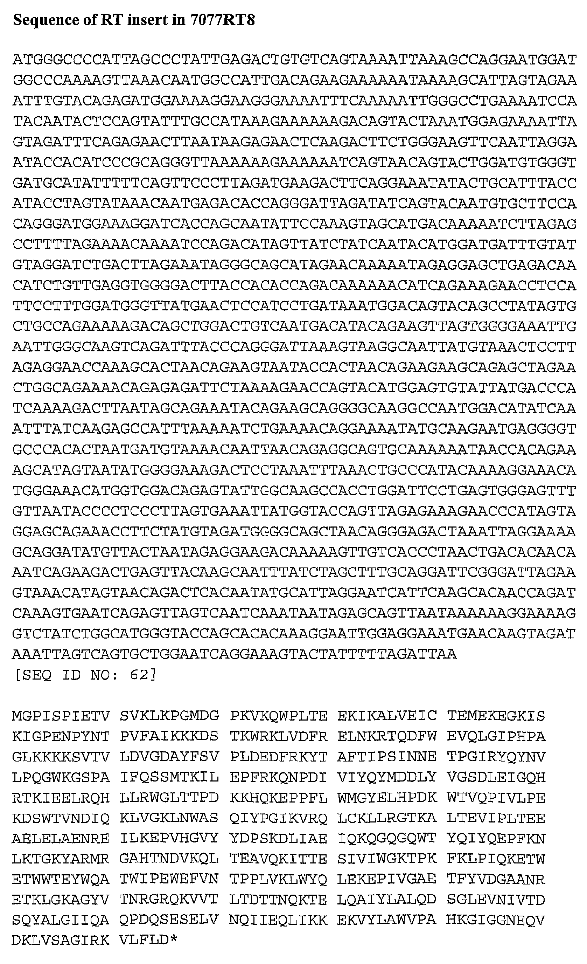

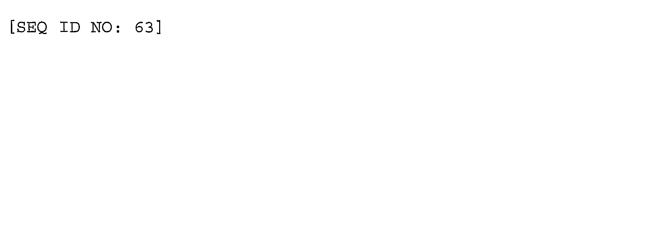

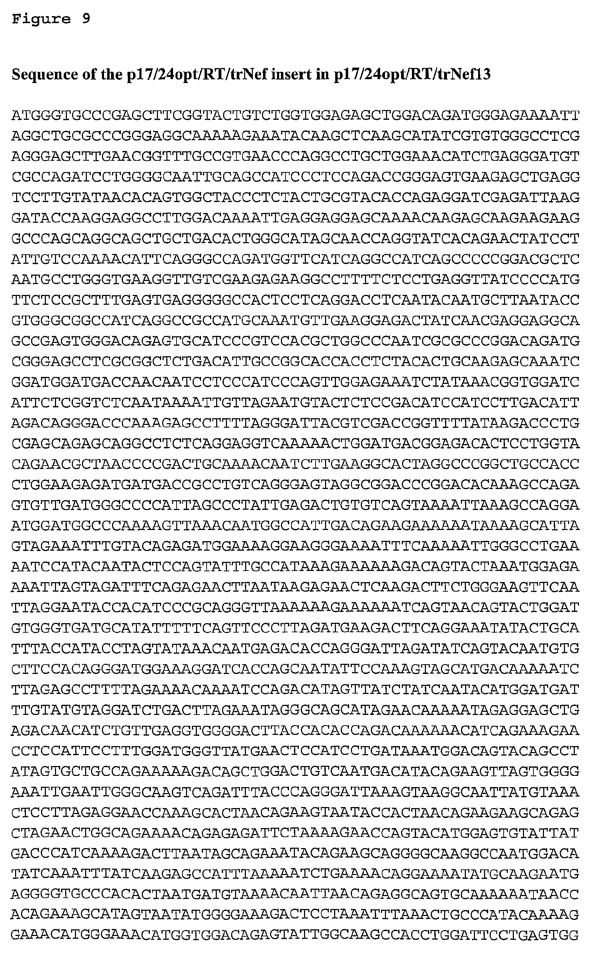

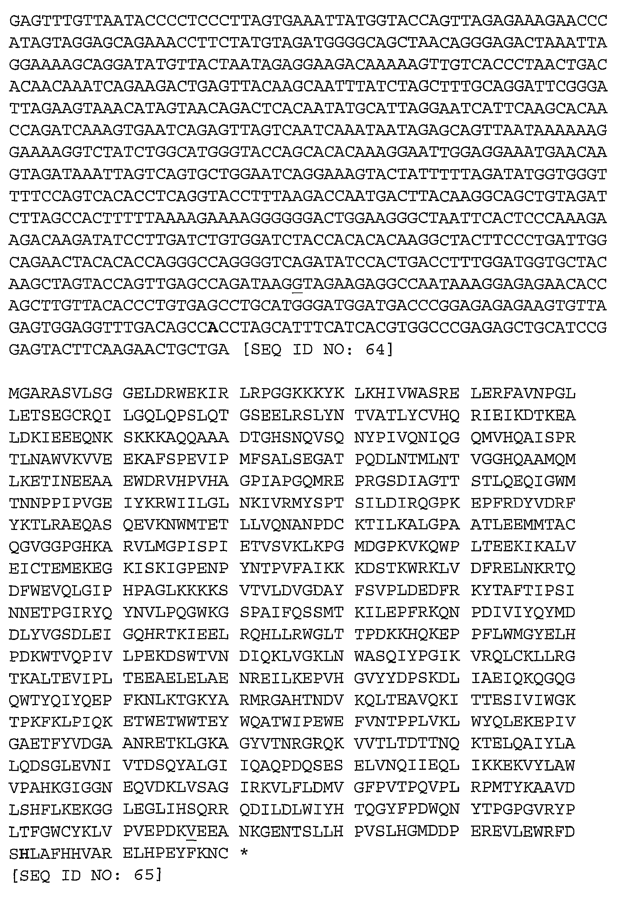

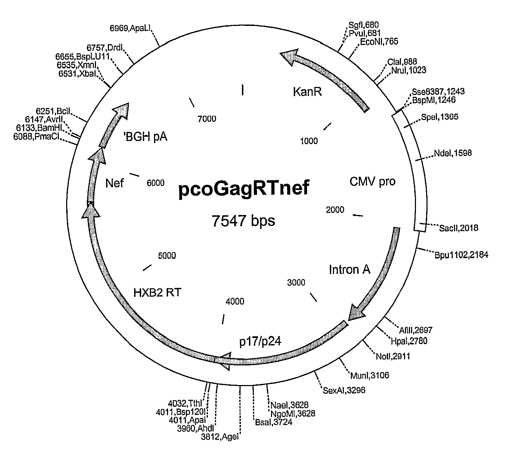

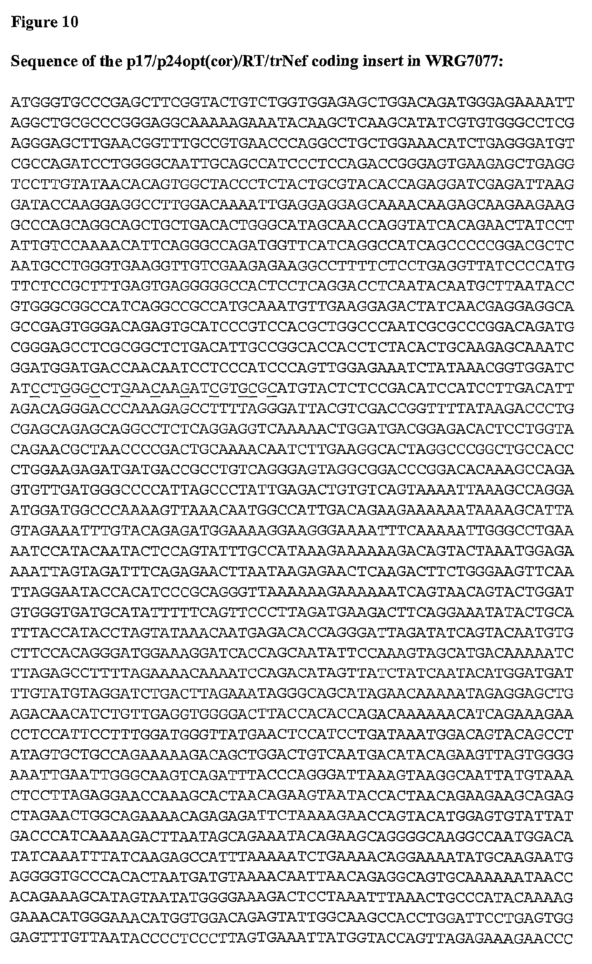

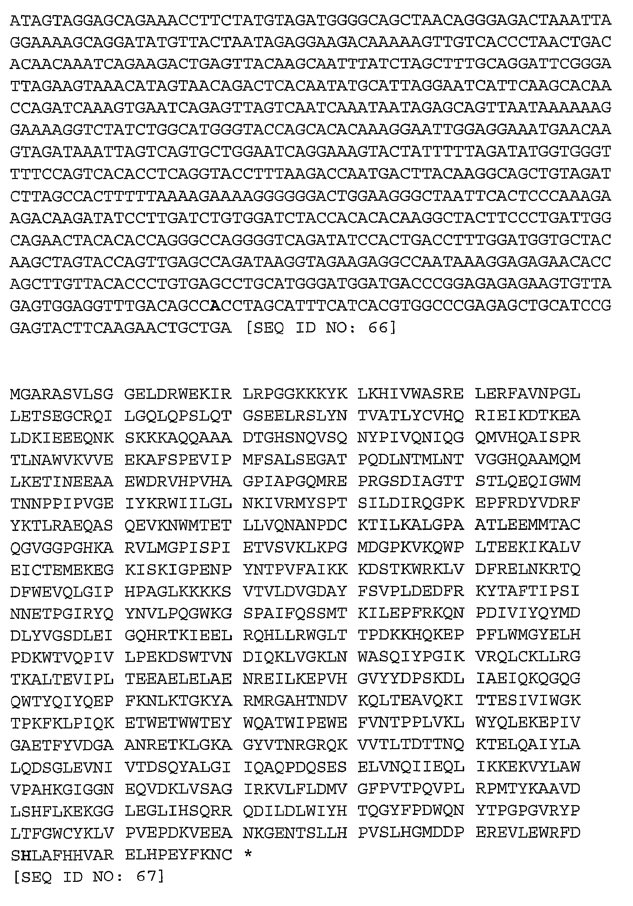

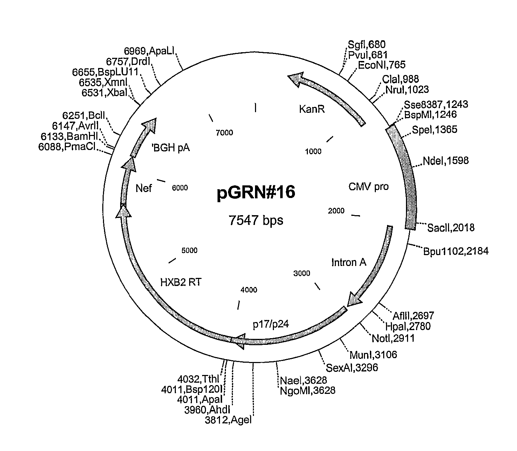

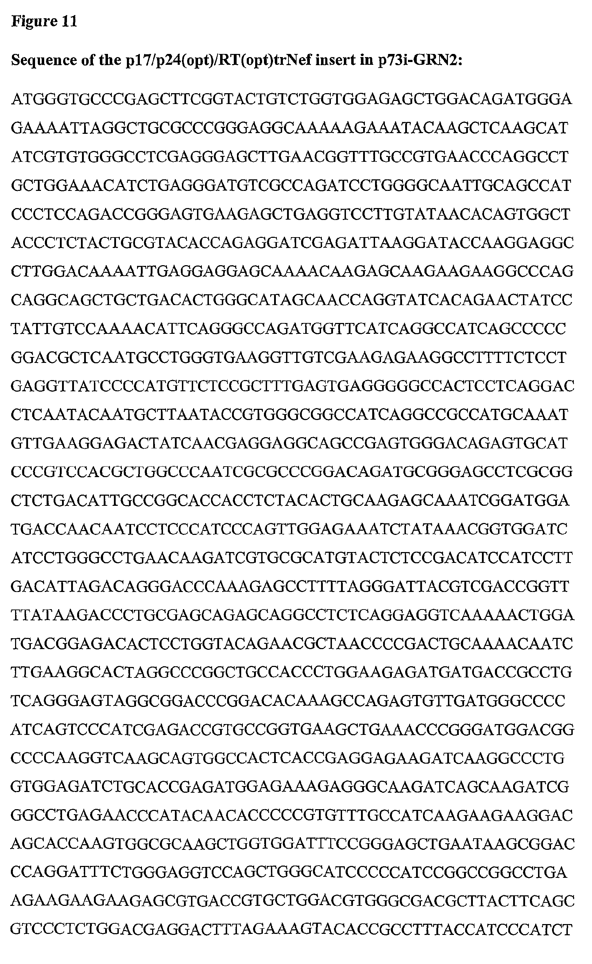

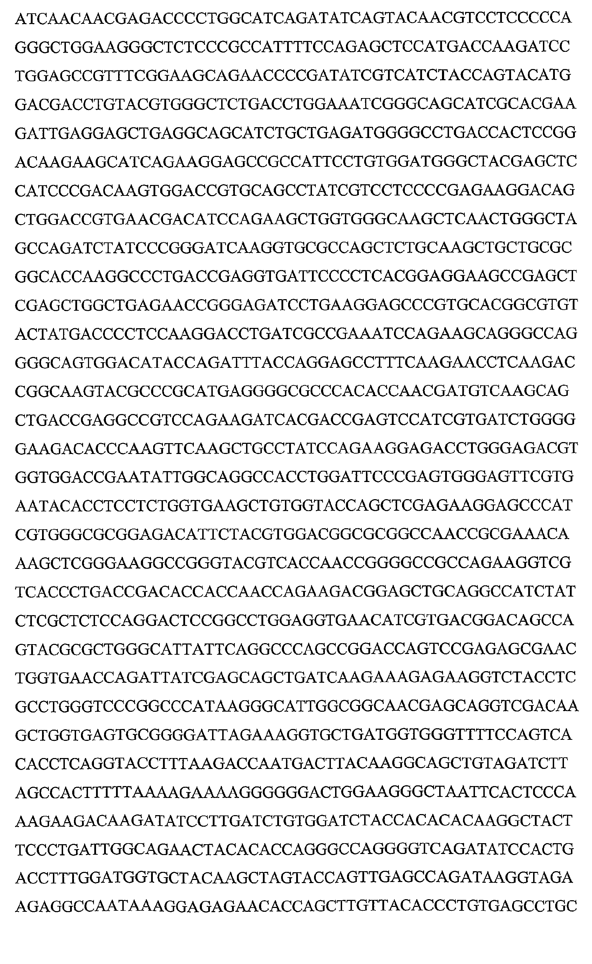

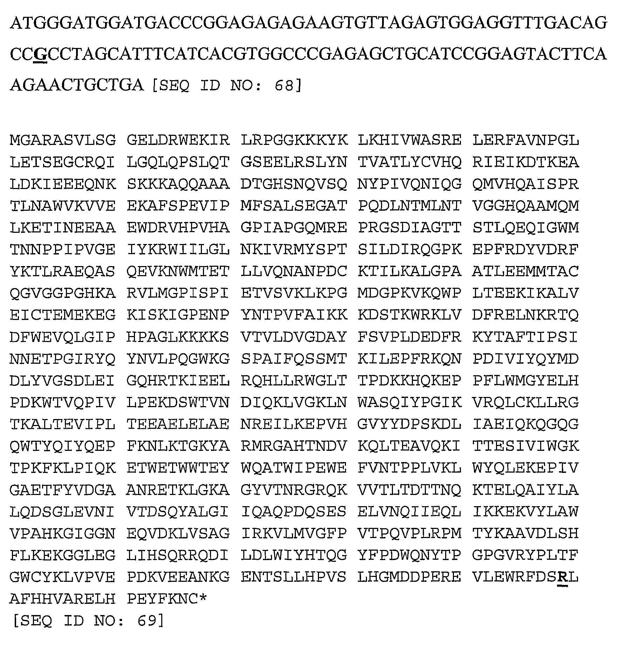

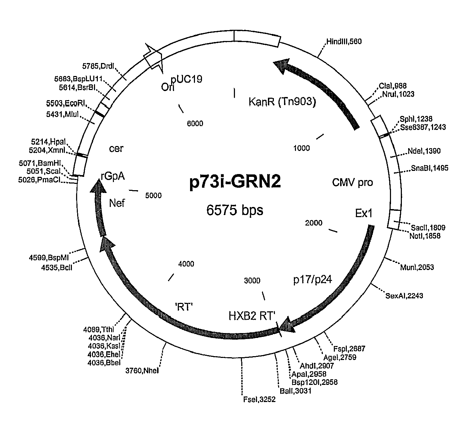

The synthetic gene coding for the RT portion of the pol gene of HIV-1 clade B strain HXB2, optimised for expression in mammalian cells was excised from plasmid p7077-RT3 as a 1697bp NotI / BamHI fragment, gel purified, and cloned into the NotI & BamHI sites of p7313-ie (derived from pspC31) to place the gene downstream of an Iowa length HCMV promoter + exon1, and upstream of a rabbit globin poly-adenylation signal. (R7004 p27) (figure 6)

### Example 6

### Plasmid: 7077trNef20

### Gene of interest

The insert comprises part of the Nef gene from the HIV-1 clade B strain HXB2. 195bp are deleted from the 5' end of the gene removing the codons for the first 65 amino acids of Nef. In addition the premature termination codon in the published HXB2 nef sequence has been repaired (TAG to TGG [Trp]) as has been described for plasmid p17/24trNEF1. The truncated nef sequence was PCR amplified from the plasmid p17/24trNef1. The sequence cloned is equivalent to positions 8992-9417 of the HXB2 reference sequence (GenBank entry K03455). To ensure expression the cloned sequence has an additional codon at the 5' end not present in the original gene - AUG (Met).

### Primers:

StrNef (sense) ATAAGAATGCGGCCGCCATGGTGGGTTTTCCAGTCACACCTT [SEQ ID NO: 1]
AStrNef (antisense)
   CGCGGATCCTCAGCAGTTCTTGAAGTACTCC [SEQ ID NO: 2]
PCR: 94° C 2min, then 25 cycles: 94° C 30sec, 50° C 30sec, 72° C 2min, ending 72° C 5min

### Cloning:

The 455bp RT PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

### Example 7

### Plasmid: 7077RT 8

### Gene of interest

The RT portion of the pol gene was derived from the HIV-1 clade B strain HXB2. It was PCR amplified from the plasmid p7077Po114.

The sequence cloned is equivalent to positions 2550-4234 of the HXB2 reference sequence (GenBank entry K03455). To ensure expression the cloned sequence has two additional codons at the 5' end not present in the original gene - AUG GGC (Met Gly) .

### Primers:

SRT (sense) ATAAGAATGCGGCCGCCATGGGCCCCATTAGCCCTATTGAGACT [SEQ ID NO: 3]
ASRT (antisense)
   CGCGGATCCTTAATCTAAAAATAGTACTTTCCTGATT [SEQ ID NO: 4]
PCR: 94° C 2min, then 25 cycles: 94° C 30sec, 50° C 30sec, 72° C 4min, ending 72° C 5min

### Cloning:

The 1720bp RT PCR product was gel purified, cut with restriction endonucleases NotI and Bam HI and ligated into NotI/BamHI cut vector WRG7077. This places the gene between the CMV promoter/intron A and the Bovine growth hormone polyadenylation signal.

### Example 8

### p17/24opt/RT/trNef13 ('Gagopt/RT/Nef')

This construct contains a PCR that causes an R to H amino acid change.

### Gene of interest:

The p17/p24 portion of the codon optimised p55gag gene derived from the HIV-1 clade B strain HXB2 was PCR amplified from the plasmid pGagOPTrpr2. The RT coding sequence was PCR amplified from the plasmid 7077RT 8. The truncated HXB2 Nef gene with the premature termination codon repaired (TGA [stop] to TGG [Trp]) was amplified by PCR from the plasmid 7077trNef20. The three PCR products were designed to have overlapping ends so that the three genes could be joined in a second PCR.

### Primers:

### (P17/24)

Sp17p24opt (sense)
   ATAAGAATGCGGCCGCCATGGGTGCCCGAGCTTCGGT [SEQ ID NO: 5]
ASp17p24optRTlinker (antisense)
   TGGGGCCCATCAACACTCTGGCTTTGTGTC [SEQ ID NO: 6]
PCR: 94° C 1min, then 20 cycles: 94° C 30sec, 50° C 30sec, 72° C 2min, ending 72° C 4min

The 1114bp p17/24opt product was gel purified.

### (RT)

Sp17p24optRTlinker (sense)
   CAGAGTGTTGATGGGCCCCATTAGCCCTAT [SEQ ID NO: 7]
ASRTtrNeflinker (antisense)
   AACCCACCATATCTAAAAATAGTACTTTCC [SEQ ID NO: 8]
PCR: as above

The 1711bp RT PCR product was gel purified

### (5' truncated nef)

SRTtrNef linker (sense)
   CTATTTTTAGATATGGTGGGTTTTCCAGTCAC [SEQ ID NO: 9]
AStrNef (antisense)
   CGCGGATCCTCAGCAGTTCTTGAAGTACTCC [SEQ ID NO: 10]
PCR as above.

The 448bp product was gel purified.

The three PCR products were then stitched together in a second PCR with primers Sp17/24opt and AstrNef.

PCR: 94° C 1min, then 30 cycles: 94° C 30sec, 50° C 30sec, 72° C 4min, ending 72° C 4min

The 3253bp product was gel purified, cut with restriction endonucleases NotI and BamHI and cloned into the NotI BamHI sites of vector WRG7077. This places the gene between the CMV promoter/intron A and the Bovine growth hormone polyadenylation signal.

### Example 9

### Plasmid: pGRN#16 (p17/p24opt corr /RT/trNef.)

### Gene of interest:

The polyprotein generated by p17/24opt/RT/trNef13 ('Gagopt/RT/Nef') was observed to express a truncated product of ∼30kDa due to a cluster of unfavourable codons within p24 around aminoacid 270. These were replaced with optimal codons by PCR stitching mutagenesis.
p17/24opt/RT/trNef13 was used as a template to amplify the portion of Gag 5' to the mutation with primers Sp17/p24opt and GTR-A, and the portion of Gag 3' to the mutation with primers GTR-S and Asp17/p24optRTlinker. The overlap of the products contained the codon changes, and the gel purified products were stitched together using the Sp17/p24opt and Asp17/p24optRTlinker primers. The product was cut with NotI and AgeI and inserted into similarly cut p17/24opt/RT/trNef13, to generate pGRN. Clone #16 was verified and progressed.

### Primers:

### 5' PCR:

Sp17p24opt (sense)
   ATAAGAATGCGGCCGCCATGGGTGCCCGAGCTTCGGT [SEQ ID NO: 11]
GTR-A (Antisense)
   GCGCACGATCTTGTTCAGGCCCAGGATGATCCACCGTTTATAGATTTCTCC [SEQ ID NO: 12]

### 3' PCR

Sense: GTR-S(Sense)
   ATCCTGGGCCTGAACAAGATCGTGCGCATGTACTCTCCGACATCCATCC [SEQ ID NO: 13]
ASp17p24optRTlinker (antisense)
   TGGGGCCCATCAACACTCTGGCTTTGTGTC [SEQ ID NO: 14]

PCR conditions for individual products and stitch, using PWO DNA polymerase (Roche): 95°C 1min, then 20 cycles 95°C 30s, 55°C 30s, 72°C 180s, ending 72°C 120s and 4°C hold.

The 1114bp product was gel purified and cut with NotI and AgeI to release a 6647bp fragment which was gel purified and ligated into NotI / AgeI cut gel purified p17/24opt/RT/trNef13 to generate pGRN#16.

### Example 10:

### Plasmid: p73i-GRN2 Clone #19

### (p17/p24 (opt) /RT (opt) trNef) - repaired

### Gene of interest:

The p17/p24 portion of the codon optimised gag, codon optimised RT and truncated Nef gene from the HIV-1 clade B strain HXB2 downstream of an Iowa length HCMV promoter + exon1, and upstream of a rabbit β-globin poly-adenylation signal.

Plasmids containing the trNef gene derived from plasmid p17/24trNef1 contain a PCR error that gives an R to H amino acid change 19 amino acids from the end of nef. This was corrected by PCR mutagenesis, the corrected nef PCR stitched to codon optimised RT from p7077-RT3, and the stitched fragment cut with ApaI and BamHI, and cloned into ApaI/BamHI cut p73i-GRN.

### Primers:

PCR coRT from p7077-RT3 using primers:
(Polymerase = PWO (Roche) throughout.

### Sense: U1

GAATTCGCGGCCGCGATGGGCCCCATCAGTCCCATCGAGACCGTGCCGGTGAAGCTG
AAACCCGGGAT [SEQ ID NO: 15]

AScoRT-Nef
GGTGTGACTGGAAAACCCACCATCAGCACCTTTCTAATCCCCGC [SEQ ID NO: 16]
Cycle: 95°C (30s) then 20 cycles 95°C (30s), 55°C (30s) , 72°C (180s), then 72°C (120s) and hold at 4°C
The 1.7kb PCR product was gel purified.

PCR 5' Nef from p17/24trNef1 using primers:
Sense: S-Nef
   ATGGTGGGTTTTCCAGTCACACC [SEQ ID NO: 17]
Antisense: ASNef-G:
   GATGAAATGCTAGGCGGCTGTCAAACCTC [SEQ ID NO: 18]
Cycle: 95°C (30s) then 15 cycles 95°C (30s) , 55°C (30s), 72°C(60s), then 72°C(120s) and hold at 4°C

PCR 3' Nef from p17/24trNef1 using primers:
Sense: SNEF-G
   GAGGTTTGACAGCCGCCTAGCATTTCATC [SEQ ID NO: 19]
Antisense:
   AStrNef (antisense)
   CGCGGATCCTCAGCAGTTCTTGAAGTACTCC [SEQ ID NO: 20]
Cycle: 95°C (30s) then 15 cycles 95°C (30s), 55°C (30s) , 72°C(60s), then 72°C(120s) and hold at 4°C

The PCR products were gel purified. Initially the two Nef products were stitched using the 5' (S-Nef) and 3'
(AstrNef) primers.
Cycle: 95°C (30s) then 15 cycles 95°C (30s), 55°C (30s), 72°C(60s), then 72°C(180s) and hold at 4°C .

The PCR product was PCR cleaned, and stitched to the RT product using the U1 and AstrNef primers:
Cycle: 95°C (30s) then 20 cycles 95°C (30s), 55°C (30s), 72°C (180s), then 72°C (180s) and hold at 4°C

The 2.1kb product was gel purified, and cut with ApaI and BamHI. The plasmid p73I-GRN was also cut with Apa1 and BamHI gel purified and ligated with the ApaI-Bam RT3trNef to regenerate the p17/p24(opt)/RT(opt)trNef gene.

### Example 11

### p73i-GN2 Clone #2 (p17/p24opt/trNef) - repaired

### Gene of interest:

The p17/p24 portion of the codon optimised gag and truncated Nef genes from the HIV-1 clade B strain HXB2 downstream of an Iowa length HCMV promoter + exon1, and upstream of a rabbit β-globin poly-adenylation signal.

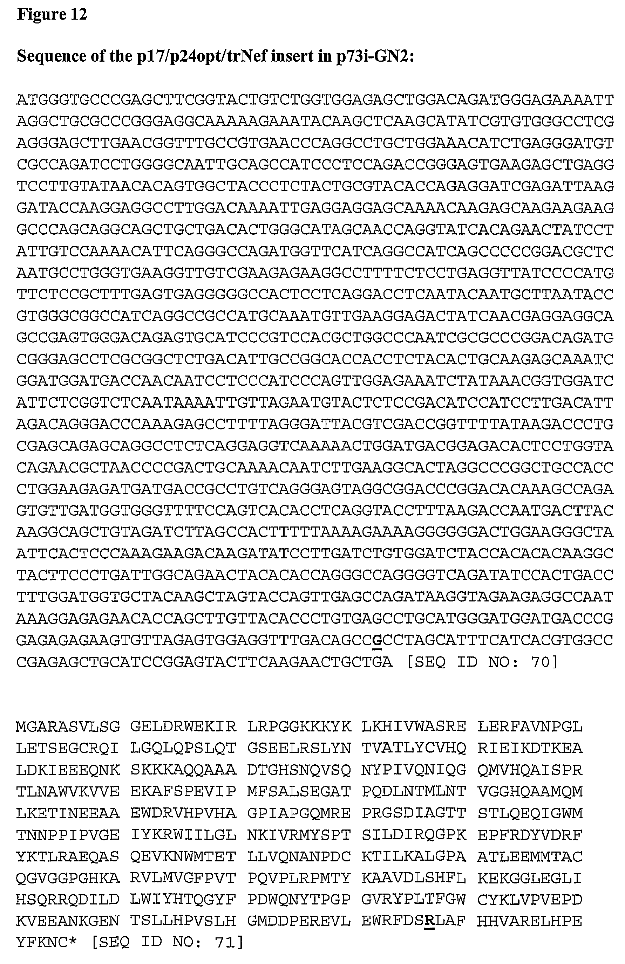

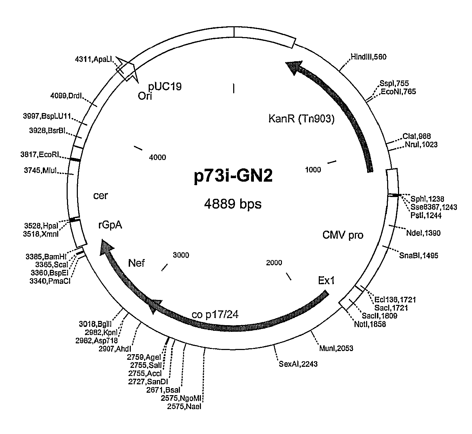

Plasmids containing the trNef gene derived from plasmid p17/24trNef1 contain a PCR error that gives an R to H amino acid change 19 amino acids from the end of Nef. This was corrected by PCR mutagenesis and the corrected fragment cut with BglII and BamHI, and cloned into BglII/BamHI cut p73I-GN. (Figure 12) regenerate the corrected p17/p24opt/trNef fusion gene downstream of the Iowa length HCMV promoter + exon1, and upstream of the rabbit β-globin polyadenylation signal.

### PCR 5' Nef from p17/24trNef1 using primers:

Polymerase = PWO (Roche) throughout.
Sense: S-Nef
   ATGGTGGGTTTTCCAGTCACACC [SEQ ID NO: 21]
Antisense: ASNef-G:
   GATGAAATGCTAGGCGGCTGTCAAACCTC [SEQ ID NO: 22]
Cycle: 95°C (30s) then 15 cycles 95°C (30s), 55°C (30s) , 72°C(60s), then 72°C(120s) and hold at 4°C

### PCR 3' Nef from p17/24trNef1 using primers:

Sense: SNEF-G
   GAGGTTTGACAGCCGCCTAGCATTTCATC [SEQ ID NO: 23]
Antisense: AStrNef
   CGCGGATCCTCAGCAGTTCTTGAAGTACTCC [SEQ ID NO: 24]
Cycle: 95°C (30s) then 15 cycles 95°C (30s) , 55°C (30s) , 72°C(60s), then 72°C(120s) and hold at 4°C

The PCR products were gel purified, and stitched using the 5' (S-Nef) and 3' (AstrNef) primers.

Cycle: 95°C (30s) then 15 cycles 95°C (30s), 55°C (30s), 72°C(60s), then 72°C(180s) and hold at 4°C .

The PCR product was PCR cleaned, cut with BglII / BamHI , and the 367bp fragment gel purified and cloned into BglII/BamHI cut gel purified p73i-GN.

### Example 12

### Plasmid: p73I-RT w229k (Inactivated RT)

### Gene of Interest:

Generation of an inactivated RT gene downstream of an Iowa length HCMV promoter + exon 1, and upstream of a rabbit β-globin poly-adenylation signal.

Due to concerns over the use of an active HIV RT species in a therapeutic vaccine inactivation of the gene was desirable. This was achieved by PCR mutagenesis of the RT (derived from P73I-GRN2) amino acid position 229 from Trp to Lys (R7271 p1-28) .

### Primers:

PCR 5' RT + mutation using primers:
(polymerase = PWO (Roche) throughout)
Sense : RT3-u:1 
GAATTCGCGGCCGCGATGGGCCCCATCAGTCCCATCGAGACCGTGCCGGTGAAGCTG
AAACCCGGGAT [SEQ ID NO: 25]
Antisense: AScoRT-Trp229Lys
   GGAGCTCGTAGCCCATCTTCAGGAATGGCGGCTCCTTCT [SEQ ID NO: 26]
Cycle:
   1 x [94°C (30s)]
   15 x [94°C (30s) /55°C (30s) /72°C (60s)
   1 x [72°C (180s)]
   PCR gel purify

PCR 3' RT + mutation using primers:
Antiense: RT3- 1:1 
GAATTCGGATCCTTACAGCACCTTTCTAATCCCCGCACTCACCAGCTTGTCGACCTG
CTCGTTGCCGC [SEQ ID NO: 27]
Sense: ScoRT-Trp229Lys
   CCTGAAGATGGGCTACGAGCTCCATG [SEQ ID NO: 28]
Cycle:
   1 x [94°C (30s)]
   15 x [94°C (30s) /55°C (30s) /72°C (60s)]
   1 x [72°C (180s)]
   PCR gel purify

The PCR products were gel purified and the 5' and 3' ends of RT were stitched using the 5' (RT3-U1) and 3' (RT3-L1) primers.

Cycle:
1 x [94°C (30s)]
15 x [94°C (30s) /55°C (30s) /72°C (120s)]
1 x [72°C (180s)]

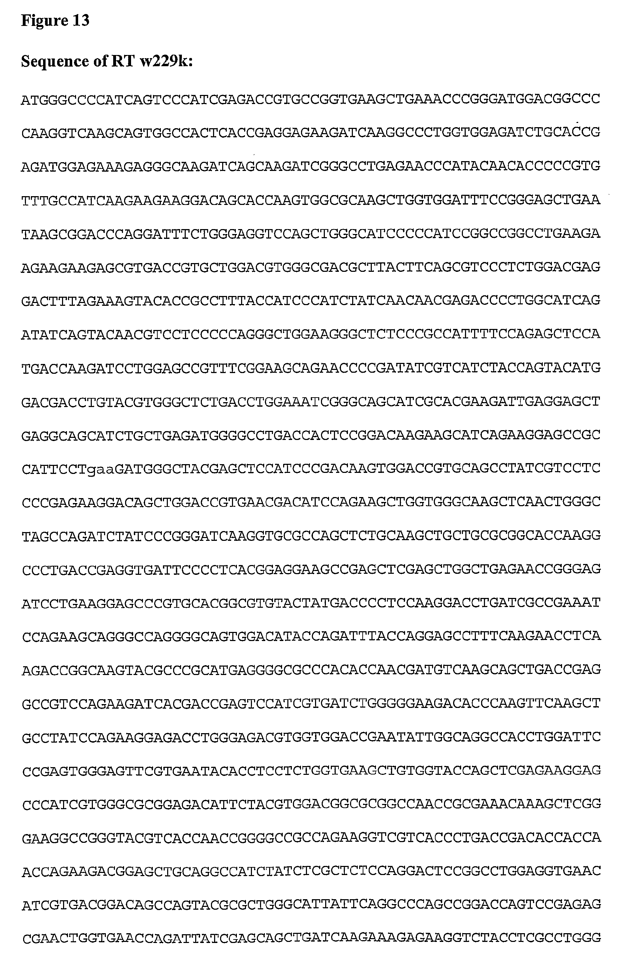

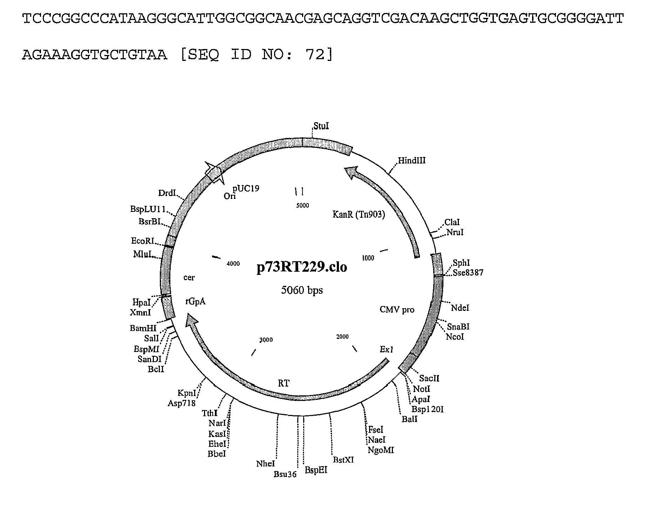

The PCR product was gel purified, and cloned into p7313ie, utilising NotI and BamHI restriction sites, to generate p731-RT w229k. (See figure 13)

### Example 13:

### Plasmid: p73i-Tgrn (#3)

### Gene of interest:

The p17/p24 portion of the codon optimised gag, codon optimised RT and truncated Nef gene from the HIV-1 clade B strain HXB2 downstream of an Iowa length HCMV promoter + exon1, and upstream of a rabbit β-globin poly-adenylation signal.

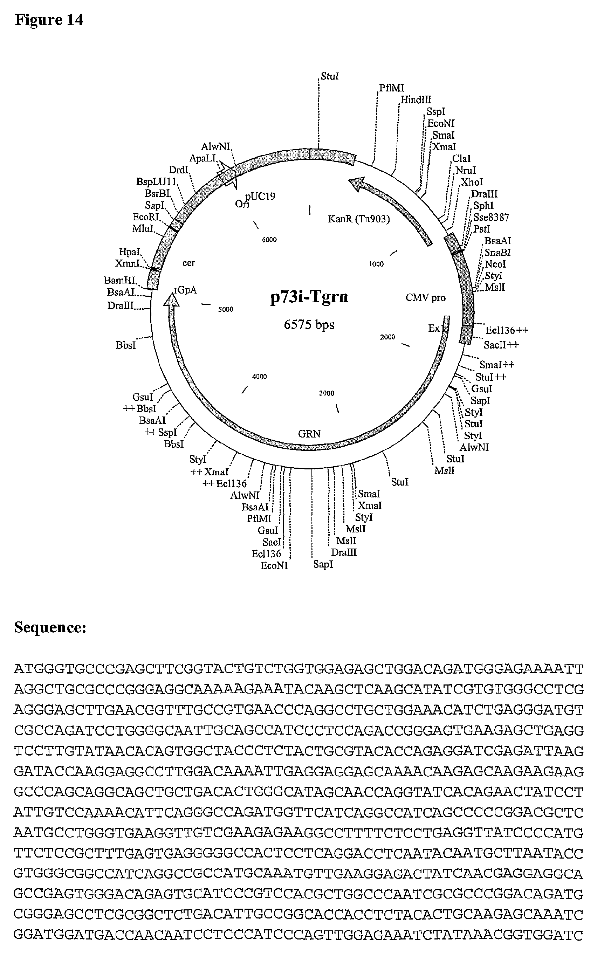

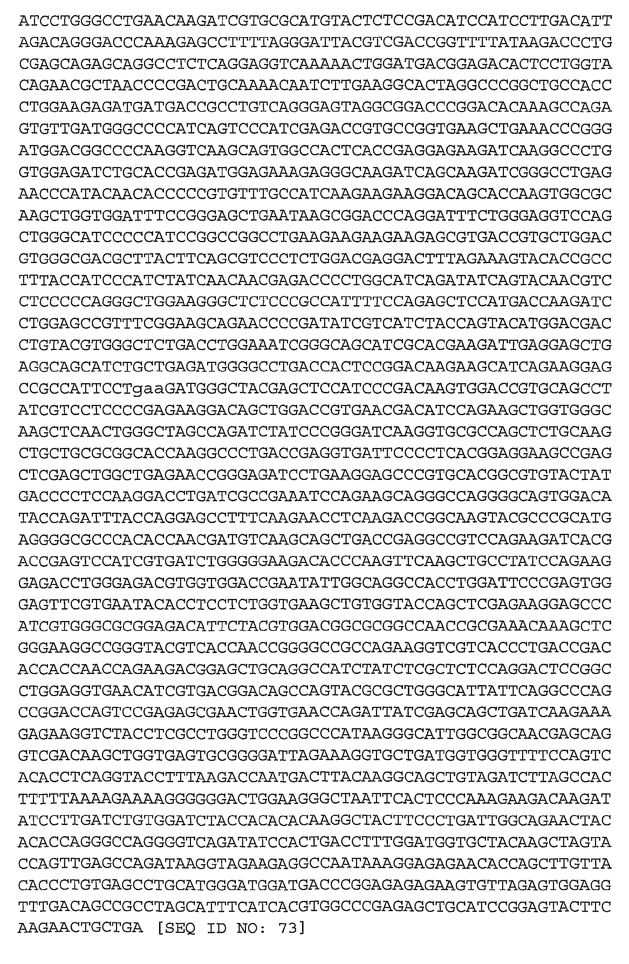

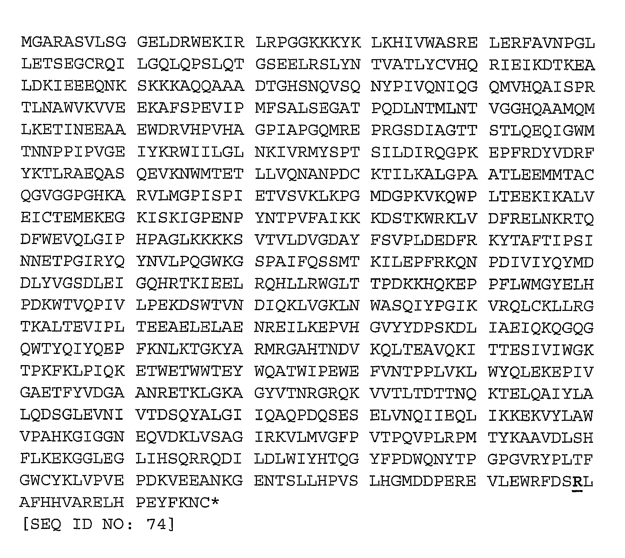

Triple fusion constructs which contain an active form of RT, may not be acceptable to regulatory authorities for human use thus inactivation of RT was achieved by Insertion of a NheI and ApaI cut fragment from p73i-RT w229k, into NheI/ApaI cut p73i-GRN2#19 (Figure 14). This results in a W → K change at position 229 in RT.

### Example 14

### p73I-Tnrg (#16)

### Gene of interest:

The truncated Nef , inactivated codon optimised RT and p17/p24 portion of the codon optimised gag gene from the HIV-1 clade B strain HXB2 downstream of an Iowa length HCMV promoter + exon1, and upstream of a rabbit β-globin poly-adenylation signal.

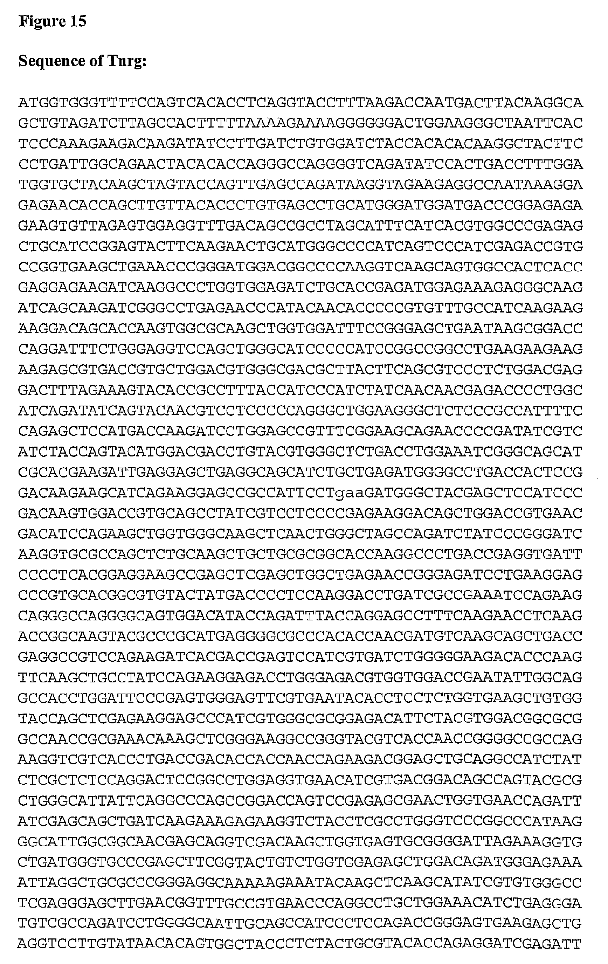

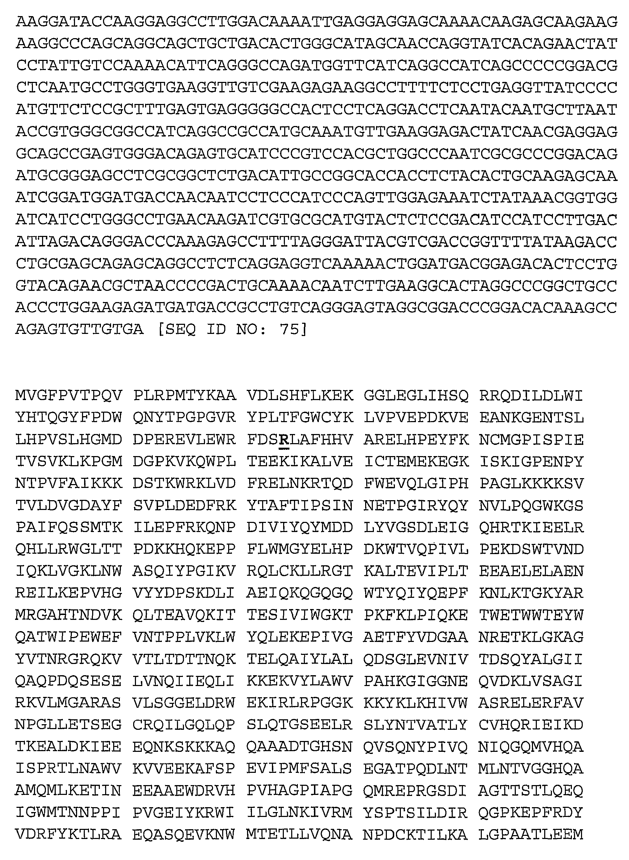

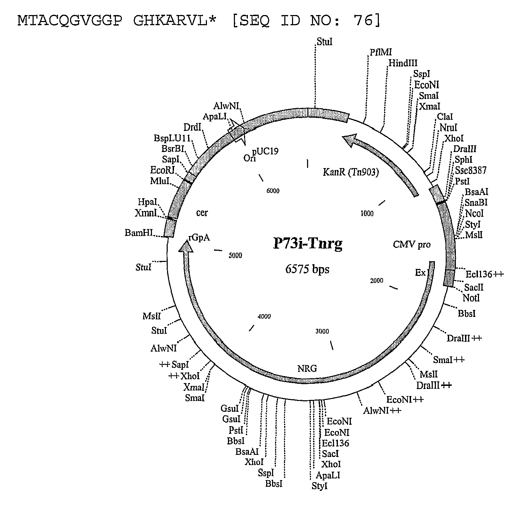

The order of the genes in the polyprotein encoded by p73i-Tgrn were rearranged by PCR and PCR stitching to generate p73I-Tnrg (Figure 15). Each gene was PCR amplified and gel purified prior to PCR stitching of the genes to form a single polyprotein. The product was gel purified, NotI/BamHI digested and ligated into NotI/BamHI cut p7313ie.

### Primers:

### trNef PCR

S-Nef (Not I)
   CATTAGAGCGGCCGCGATGGTGGGTTTTCCAC [SEQ ID NO: 29]
AS-Nef-coRT linker
   GATGGGACTGATGGGGCCCATGCAGTTCTTGAACTACTCCGG [SEQ ID NO: 30]

### RTw229k PCR

S-coRT
   ATGGGCCCCATCAGTCCCATCGAG [SEQ ID NO: 31]
AS-coRT-p17p24 linker
   CAGTACCGAAGCTCGGGCACCCATCAGCACCTTTCTAATCCCCGC [SEQ ID NO: 32]

### p17p24opt PCR

S-p17p24opt
   ATGGGTGCCCGAGCTTCGGTACTG [SEQ ID NO: 33]
AS-p17p24opt (BamHI)
   GATGGGGGATCCTCACAACACTCTGGCTTTGTGTCC [SEQ ID NO: 34]

PCR conditions for individual products and stitching using VENT DNA polymerase (NEB):
1 x [94°C (30s)]
25 x [94°C (30s)/55°C (30s)/72°C (120s [p17p24 or RT] or 60s [trNef])]
1 x [72°C (240s)]

The PCR products were gel purified and used in a PCR stitching utilising the primers S-trNef (NotI) and ASp17p24opt (*Bam*HI) :
1 x [94°C (30s)]
25 x [94°C (30s) /55°C (30s) /72°C (210s)]
1 x [72°C (240s)]

The 3000bp product was gel purified and cut with NotI and BamHI which was PCR cleaned and ligated into NotI/BamHI digested gel purified p7313ie to generate p73i-Tnrg.

### Example 15:

### 1. Plasmid: P73i-Tngr (#3)

### Gene of Interest:

The truncated Nef , p17/p24 portion of the codon optimised gag and inactivated codon optimised RT gene from the HIV-1 clade B strain HXB2 downstream of an Iowa length HCMV promoter + exon1, and upstream of a rabbit β-globin poly-adenylation signal.

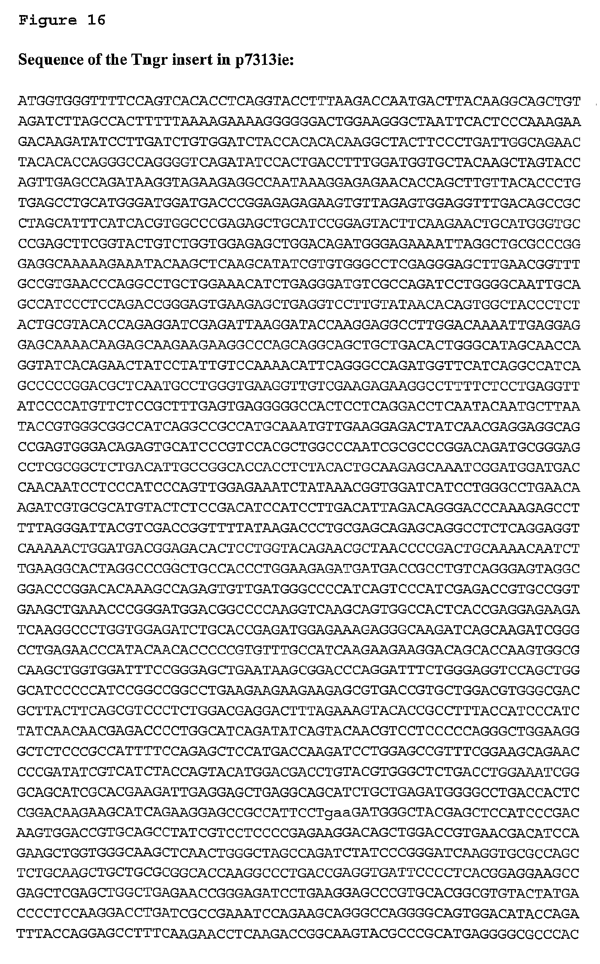

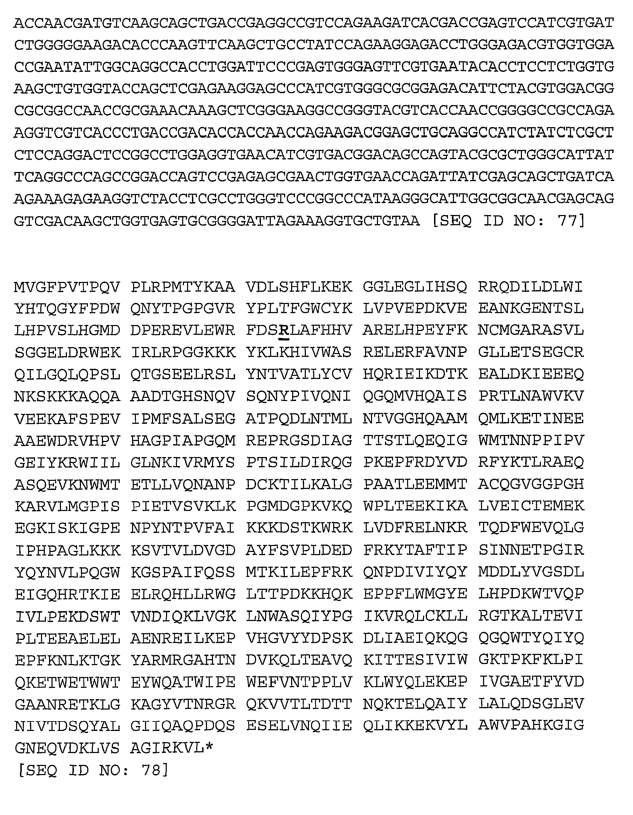

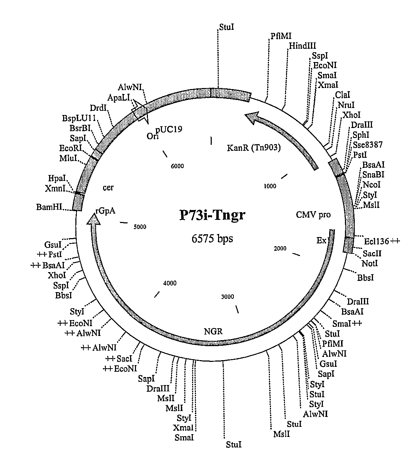

The order of the genes in the polyprotein encoded by p73i-Tgrn were rearranged by PCR to generate p73I-Tngr (Figure 16). Codon optimised p17/p24 and RT were generated as a single product, and PCR stitched to amplified trNef. The product was gel purified, NotI/BamHI digested and ligated into NotI/BamHI cut p7313ie.

### Primers:

P17/p24 - RT 3'PCR:
Sp17p24opt (sense)
   ATGGGTGCCCGAGCTTCGGTACTG [SEQ ID NO: 35]
RT3 1:1 (antisense)

GAATTCGGATCCTTACAGCACCTTTCTAATCCCCGCACTCACCAGCTTGTCGACCTG
CTCGTTGCCGC [SEQ ID NO: 36]

### TrNef 5' PCR

S-Nef (NotI)
   CATTAGAGCGGCCGCGATGGTGGGTTTTCCAC [SEQ ID NO: 37]
AS-Nef-p17p24
   CAGTACCGAAGCTCGGGCACCCATGCAGTTCTTGAACTACTCCGG [SEQ ID NO: 38]

PCR conditions for individual products and stitching using VENT DNA polymerase (NEB):
1 x [94°C (30s)]
25 x [94°C (30s)/55°C (30s)/72°C (180s [p17p24+RT] or 60s [trNef] or 210s [stitching])]
1 x [72°C (240s)]

The 3000bp product was gel purified and cut with NotI and BamHI which was PCR cleaned and ligated into NotI/BamHI digested gel purified p7313ie to generate p73i-Tngr.

### Example 16:

### Plasmid: p73I-Trgn (#6)

### Gene of interest:

The inactivated codon optimised RT, p17/p24 portion of the codon optimised gag and truncated Nef gene from the HIV-1 clade B strain HXB2 downstream of an Iowa length HCMV promoter + exon1, and upstream of a rabbit β-globin poly-adenylation signal.

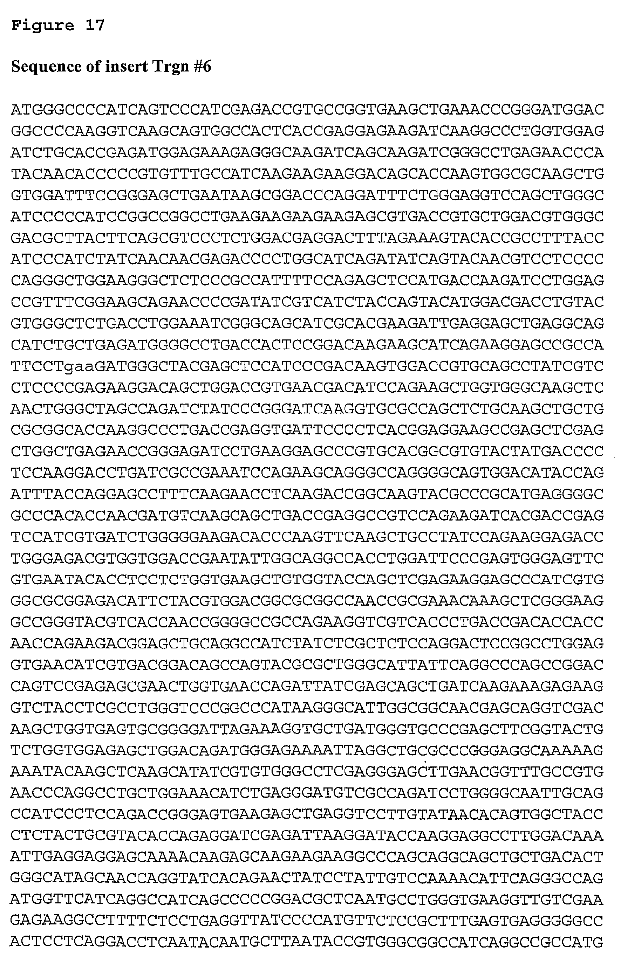

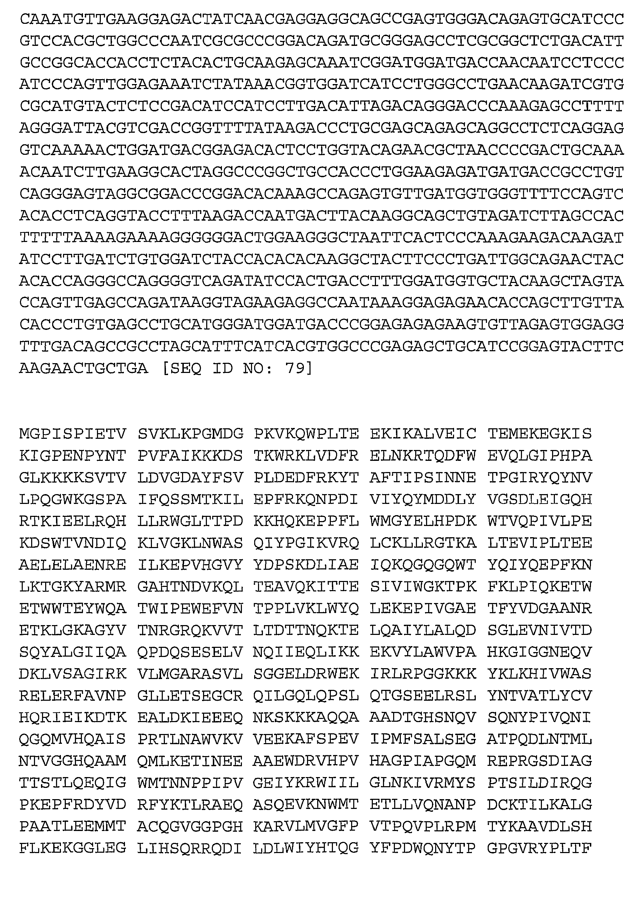

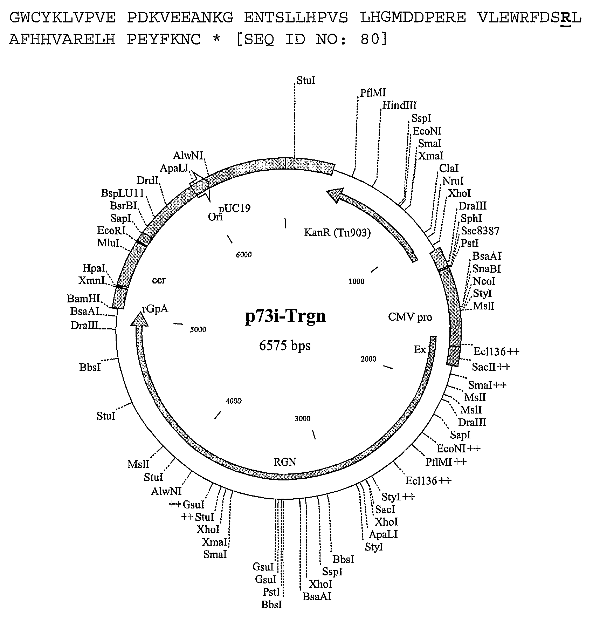

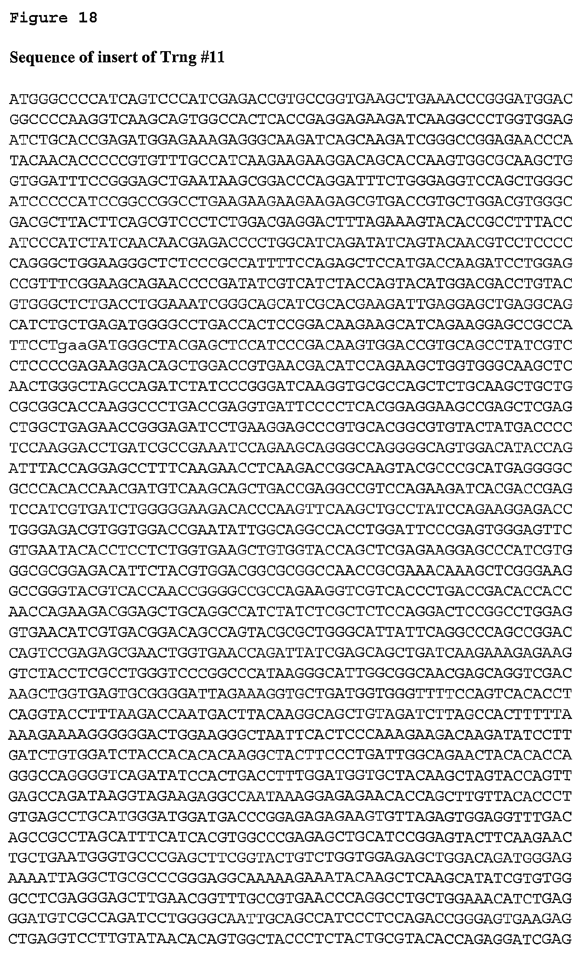

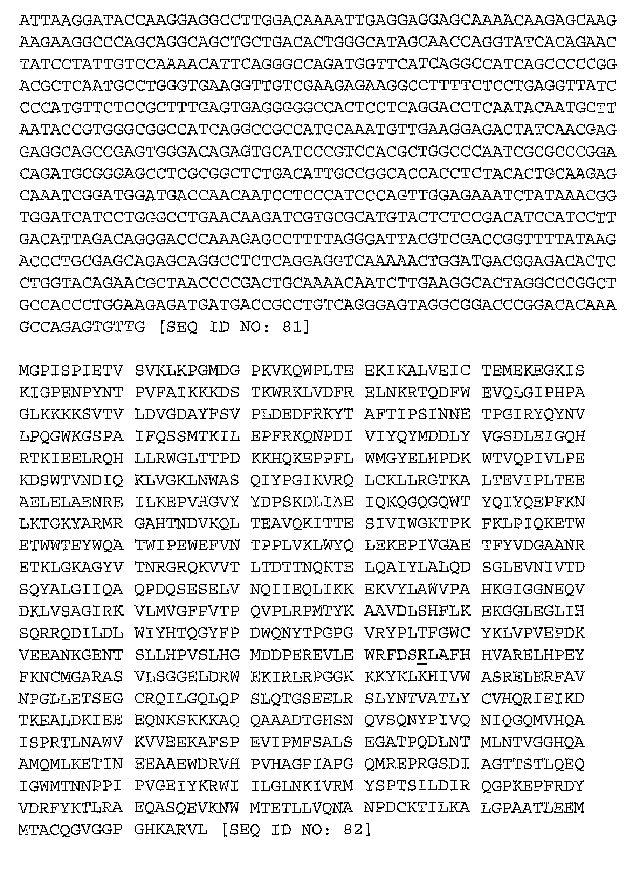

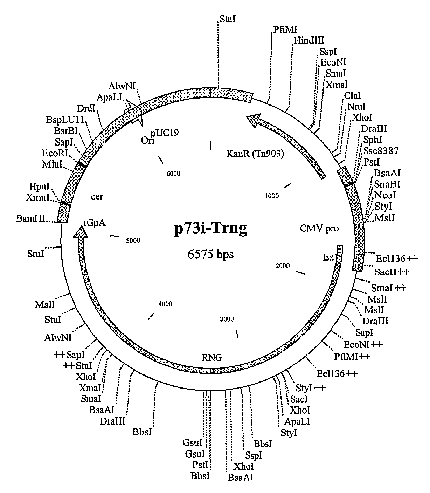

The order of the genes within the construct was achieved by PCR amplification of p17p24-trNef and RTw229k from the plasmids p73I-GN2 and p73I-RTw229k respectively. PCR stitching was performed and the product gel purified and NotI/BamHI cut prior to ligation with NotI/BamHI digested p7313ie. Sequencing revealed that p17p24 was not fully optimised a 700bp fragment was then AgeI/MunI cut from the coding region and replaced with MunI/Age fragment from p73i-Tgrn#3 containing the correct coding sequence. (See figure 17).

### Primers:

### p17p24-trNef PCR

S-p17p24opt
   ATGGGTGCCCGAGCTTCGGTACTG [SEQ ID NO: 39]

### AstrNef (BamHI)

### RTw229k

RT3-U:1 
GAATTCGCGGCCGCGATGGGCCCCATCAGTCCCATCGAGACCGTGCCGGTGAAGCTG
AAACCCGGGAT [SEQ ID NO: 40]
AS-coRT-p17p24opt linker
   CAGTACCGAAGCTCGGGCACCCATCAGCACCTTTCTAATCCCCGC [SEQ ID NO: 41]

PCR conditions for individual products and stitching using VENT DNA polymerase (NEB):
1 x [94°C (30s)]
25 x [94°C (30s) /55°C (30s) /72°C (120s (PCR) or 180s (stitching)
1 x [72°C (240s)]

The 3000bp product from the PCR stitch was gel purified and cut with NotI and BamHI which was PCR cleaned and ligated into NotI/BamHI digested gel purified p7313ie to generate p73i-Tngr. Sequence analysis showed that p17p24 sequence obtained from p73I-GN2 was not fully codon optimised and that this had been carried over into the new plasmid. This was rectified by cutting a 700bp fragment from p73i-Tngr cut with MunI and AgeI, and replacing it by ligation with a 700bp MunI/AgeI digested product from p73i-Tgrn to generate the construct p73I-Tngr#6.

### Example 17:

### Plasmid: p73i-Trng (#11)

### Gene of Interest:

The inactivated codon optimised RT, truncated Nef and p17/p24 portion of the codon optimised gag gene from the HIV-1 clade B strain HXB2 downstream of an Iowa length HCMV promoter + exon1, and upstream of a rabbit β-globin poly-adenylation signal.

The order of the genes within the construct was achieved by PCR amplification of the RT-trNef and p17p24 genes from p73i-Tgrn. PCR stitching of the two DNA fragments was performed and the 3kb product gel purified and NotI/BamHI cut prior to ligation with NotI/BamHI digested p7313ie, and yielded p73I Trng (#11).

### Primers:

### RTw229k-trNef

RT3-u:1 
GAATTCGCGGCCGCGATGGGCCCCATCAGTCCCATCGAGACCGTGCCGGTGAAGCTG
AAACCCGGGAT [SEQ ID NO: 42]
AS-Nef-p17p24opt linker
   CAGTACCGAAGCTCGGGCACCCATGCAGTTCTTGAACTACTCCGG [SEQ ID NO: 43]

### P17p24

S-p17p24opt
   ATGGGTGCCCGAGCTTCGGTACTG [SEQ ID NO: 44]
AS-p17p24opt (BamHI)
   GATGGGGGATCCTCACAACACTCTGGCTTTGTGTCC [SEQ ID NO: 45]

PCR conditions for individual products and stitching using VENT DNA polymerase (NEB):
1 x [94°C (30s)]
25 x [94°C (30s) /55°C (30s) /72°C (120s (PCR of genes) or 180s (stitching)
1 x [72°C (240s)]

The 3000bp product from the PCR stitch was gel purified and cut with NotI and BamHI which was PCR cleaned and ligated into NotI/BamHI digested gel purified p7313ie to generate p73i-Tngr.

### Example 18:

### p73i-Tgnr (#f1)

### Gene of interest:

The p17/p24 portion of the codon optimised gag, truncated Nef and codon optimised inactivated RT gene from the HIV-1 clade B strain HXB2 downstream of an Iowa length HCMV promoter + exon1, and upstream of a rabbit β-globin poly-adenylation signal.

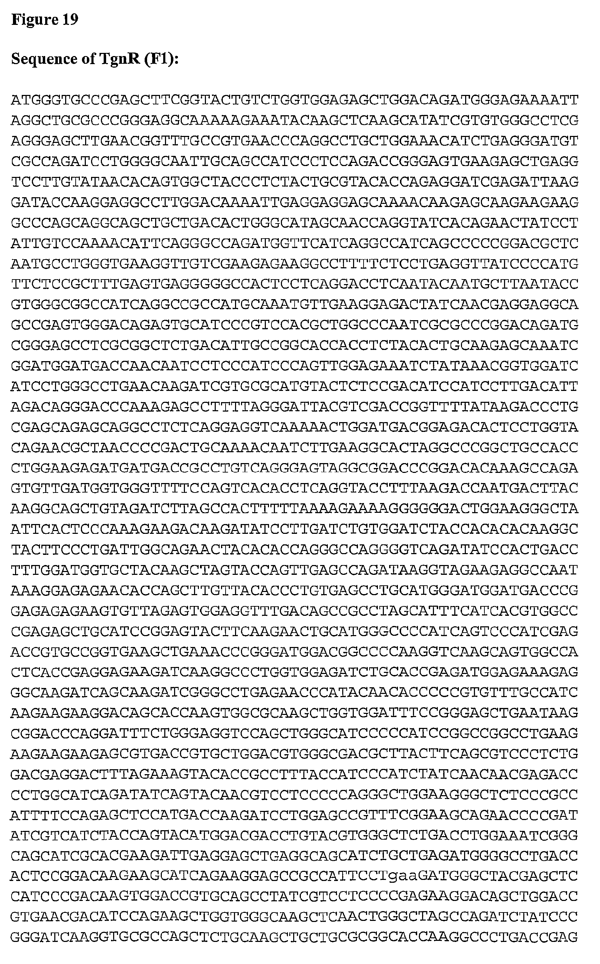

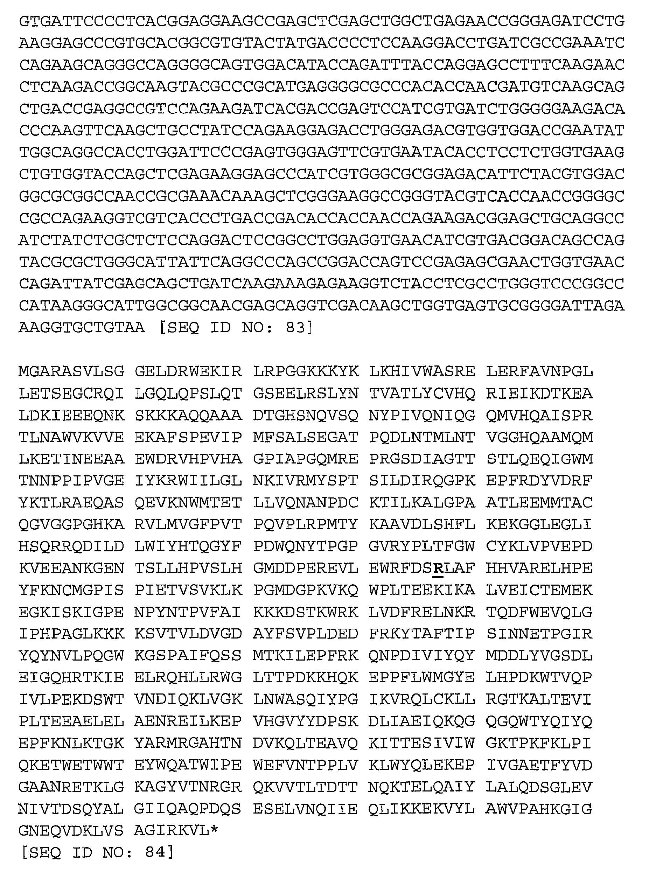

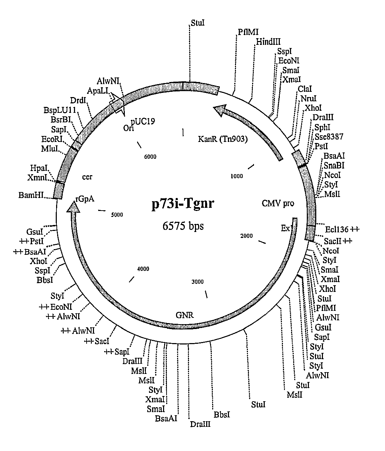

The order of the genes within the construct was achieved by PCR amplification of p17p24-trNef and RTw229k from the plasmids p73I-GN2 and p73I-RTw229k respectively. PCR stitching was performed and the product gel purified and NotI/BamHI cut prior to ligation with NotI/BamHI digested p7313ie. Two sequence errors were spotted in the sequence (p17p24 and RT) which were subsequently repaired by replacement with correct portions of the genes utilising restriction sites within the polyprotein. (See figure 19) .

### Primers:

### p17p24-trNef PCR

S-p17p24opt
   ATGGGTGCCCGAGCTTCGGTACTG [SEQ ID NO: 46]
AS-Nef-coRTlinker
   GATGGGACTGATGGGGCCCATGCAGTTCTTGAACTACTCCGG [SEQ ID NO: 47]

### RTw229k

S-coRT
   ATGGGCCCCATCAGTCCCATCGAG [SEQ ID NO: 48]
RT3-1:1

GAATTCGGATCCTTACAGCACCTTTCTAATCCCCGCACTCACCAGCTTGTCGACCTG
CTCGTTGCCGC [SEQ ID NO: 49]

PCR conditions for individual products and stitching using VENT DNA polymerase (NEB):
1 x [94°C (30s)]
25 x [94°C (30s) /55°C (30s) /72°C (120s (PCR) or 180s (stitching)
1 x [72°C (240s)]

The 3000bp product was gel purified and cut with NotI and BamHI which was PCR cleaned and ligated into NotI/BamHI digested gel purified p7313ie to generate p73i-Tngr. Sequencing revealed that p17p24 was not fully optimised a 700bp fragment was subsequently AgeI/MunI cut from the coding region and replaced with MunI/Age fragment from p73i-Tgrn#3 containing the correct coding sequence. The polyprotein also contained a single point mutation (G2609A) resulting in an amino acid substitution of Thr to Ala in the RT portion of the polyprotein. The mutation was corrected by ApaI/BamHI digestion of the construct and PCR clean up to remove the mutated sequence, which was replaced by ligation with an ApaI/BamHI digested portion of RT from p73i-Tgnr.

### Example 19:

### Preparation of plasmid-coated 'gold slurry' for 'gene gun' DNA cartridges

Plasmid DNA (approximately 1µg/µ1), eg. 100 ug, and 2µm gold particles, eg. 50 mg, (PowderJect), were suspended in 0.05M spermidine, eg. 100 ul, (Sigma). The DNA was precipitated on to the gold particles by addition of 1M CaC1₂, eg. 100ul (American Pharmaceutical Partners, Inc., USA). The DNA/gold complex was incubated for 10 minutes at room temperature, washed 3 times in absolute ethanol, eg. 3 x 1 ml, (previously dried on molecular sieve 3A (BDH)). Samples were resuspended in absolute ethanol containing 0.05mg/ml of polyvinylpyrrolidone ( PVP, Sigma), and split into three equal aliquots in 1.5 ml microfuge tubes, (Eppendorf). The aliquots were for analysis of (a) 'gold slurry', (b) eluate- plasmid eluted from (a) and (c) for preparation of gold/ plasmid coated Tefzel cartridges for the 'gene gun', (see Example 3 below). For preparation of samples (a) and (b), the tubes containing plasmid DNA / 'gold slurry' in ethanol / PVP were spun for 2 minutes at top speed in an Eppendorf 5418 microfuge, the supernatant was removed and the 'gold slurry' dried for 10 minutes at room temperature. Sample (a) was resuspended to 0.5 - 1.0 ug / ul of plasmid DNA in TE pH 8.0, assuming approx. 50 % coating. For elution, sample (b) was resuspended to 0.5 - 1.0 ug / ul of plasmid DNA in TE pH 8.0 and incubated at 37°C for 30 minutes, shaking vigorously, and then spun for 2 minutes at top speed in an Eppendorf 5418 microfuge and the supernatant, eluate, was removed and stored at -20°C. The exact DNA concentration eluted was determined by spectrophotometric quantitation using a Genequant II (Pharmacia Biotech).

### Example 20:

### Preparations of Cartridges for DNA immunisation

Preparation of cartridges for the Accell gene transfer device was as previously described (Eisenbraun et al DNA and Cell Biology, 1993 Vol 12 No 9 pp 791-797; Pertner et al). Briefly, plasmid DNA was coated onto 2 **µ**m gold particles (DeGussa Corp., South Plainfield, N.J., USA) and loaded into Tefzel tubing, which was subsequently cut into 1.27 cm lengths to serve as cartridges and stored desiccated at 4°C until use. In a typical vaccination, each cartridge contained 0.5 mg gold coated with a total of 0.5 **µ**g DNA/cartridge.

### Example 21:

### Immune Response to HIV antigens following DNA vaccination utilising the gene gun.

Mice (n=3/group) were vaccinated with antigens encoded by nucleic acid and located in two vectors. P7077 utilises the HCMV IE promoter including Intron A and exon 1 (fcmv promoter). P73I delivers the same antigen, but contains the HCMV IE promoter (icmv promoter) that is devoid of Intron A, but includes exon 1.

Plasmid was delivered to the shaved target site of abdominal skin of F1 (C3H x Balb/c) mice. Mice were given a primary immunisation of 2 x 0.5 µg DNA on day 0, boosted with 2 x 0.5 µg DNA on day 35 and cellular response were detected on day 40 using IFN - gamma Elispot.
- P73I - empty vector
- P7077 - empty vector
- P7077 GRN - (f CMV promoter) Gag, RT, Nef
- P73I GRN - (i CMV promoter) Gag, RT, Nef
- P73I GR3N - (CMV promoter) Optimised Gag, Optimised RT, Nef
- P7077 GN - (f CMV promoter)Gag, Nef
- P73I GN - (i CMV promoter) Gag, Nef
Cytotoxic T Cell Responses

The cytotoxic T cell response was assessed by CD8+ T cell-restricted IFN-γ ELISPOT assay of splenocytes collected 5 days later. Mice were killed by cervical dislocation and spleens were collected into ice-cold PBS. Splenocytes were teased out into phosphate buffered saline (PBS) followed by lysis of red blood cells (1 minute in buffer consisting of 155mM NH₄C1, 10 mM KHCO₃, 0.1mM EDTA). After two washes in PBS to remove particulate matter the single cell suspension was aliquoted into ELISPOT plates previously coated with capture IFN-γ antibody and stimulated with CD8-restricted cognate peptide (Gag, Nef or RT). After overnight culture, IFN-γ producing cells were visualised by application of anti-murine IFN-γ-biotin labelled antibody (Pharmingen) followed by streptavidin -conjugated alkaline phosphatase and quantitated using image analysis.

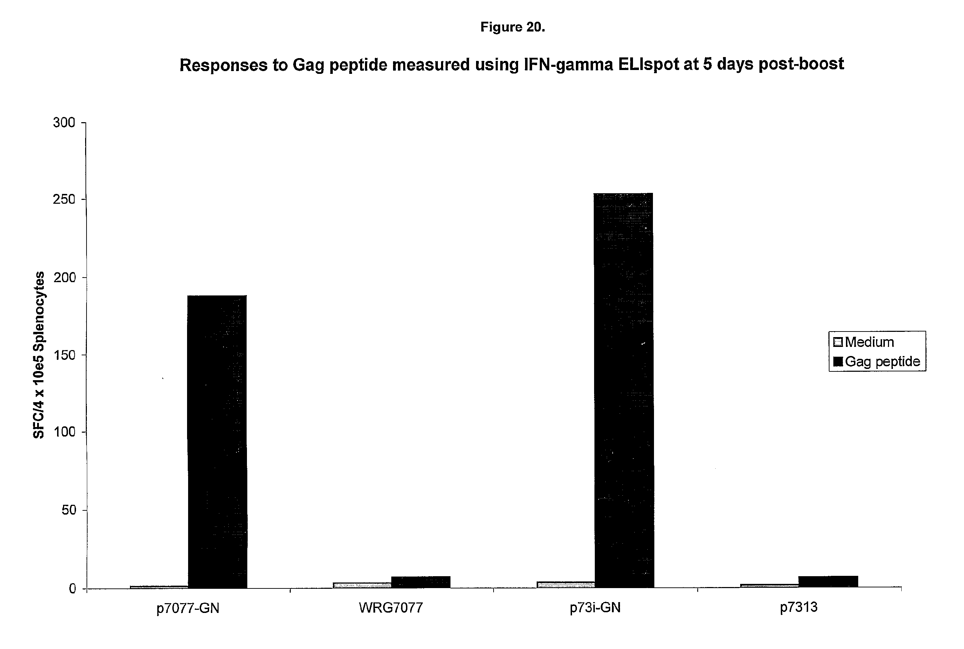

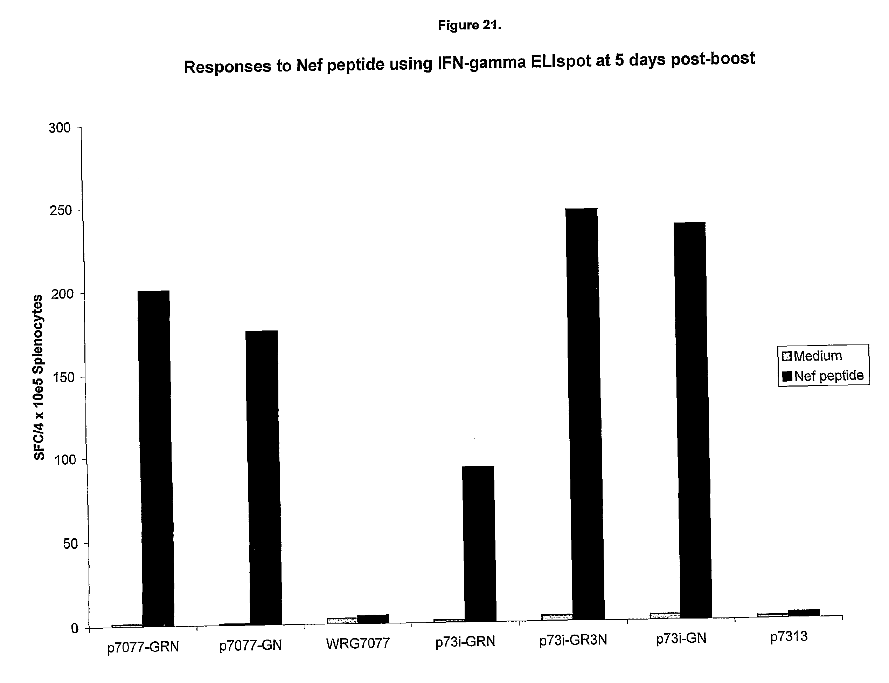

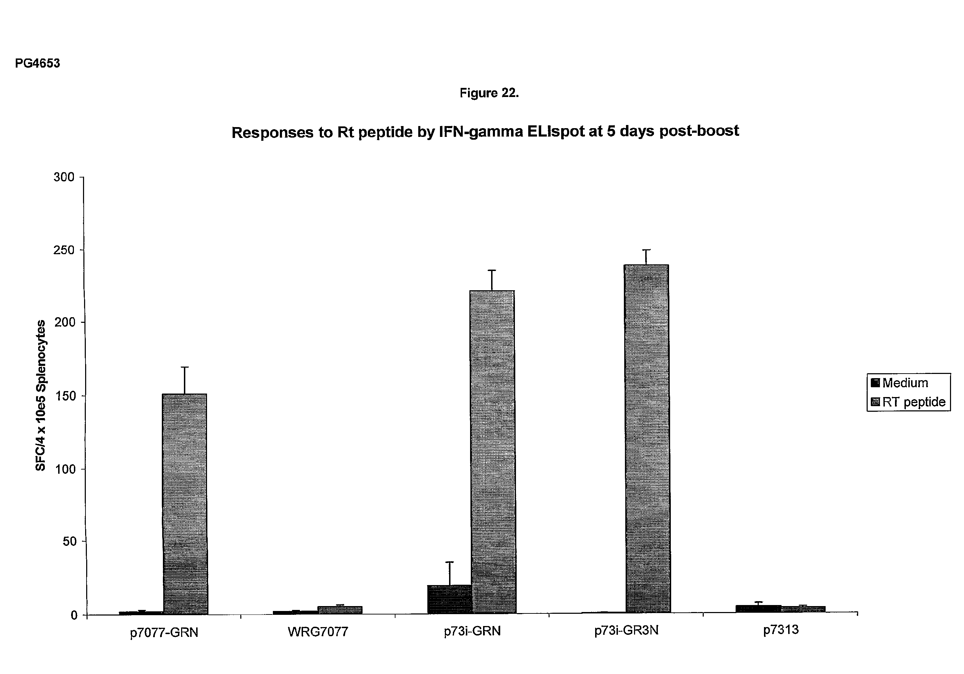

The result of this experiment are shown in figures 20, 21, and 22.

### Example 22:

### Immunogenicity of Vaccine Constructs

### 1. Cellular Assays

The cellular immune response comprises cytotoxic CD8 cells and helper CD4 cells. A sensitive method to detect specific CD8 and CD4 cells is the ELIspot assay which can be used to quantify the number of cells capable of secreting interferon-y or IL-2. The ELIspot assay relies on the capture of cytokines secreted from individual cells. Briefly, specialised microtitre plates are coated with anti-cytokine antibodies. Splenocytes isolated from immunised animals are incubated overnight in the presence of specific peptides representing known epitopes (CD8) or proteins (CD4). If cells are stimulated to release cytokines they will bind to the antibodies on the surface of the plate surrounding the locality of the individual producing cells. Cytokines remain attached to the coating antibody after the cells have been lysed and plates washed. The assay is developed in a similar way to an ELISA assay using a biotin/avidin amplification system. The number of spots equates to the number of cytokine producing cells.

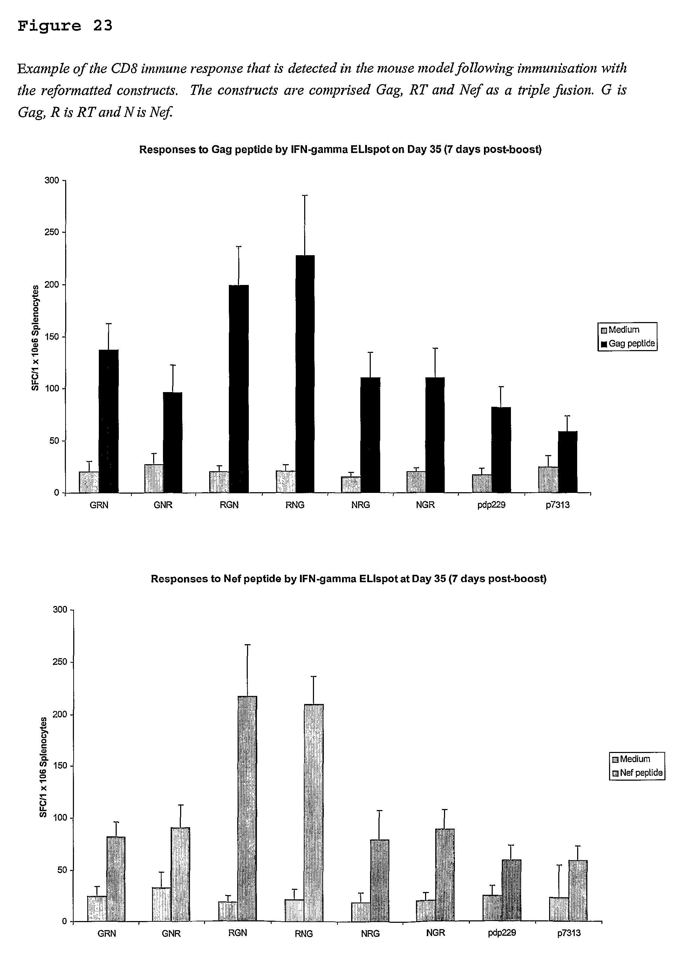

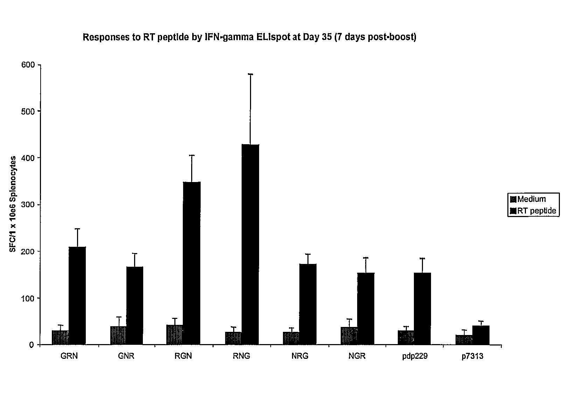

CD8 responses to the following K2^{d}-restricted murine epitopes: Gag (AMQMLKETI), Nef (MTYKAAVDL) and RT (YYDPSKDLI) and CD4 responses to Gag and RT proteins were recorded for all 6 constructs. The results of these assays were analysed statistically and constructs were ranked according to their immunogenicity. The result is shown in figure 23 of the figures.

### 2. Humoral Assays

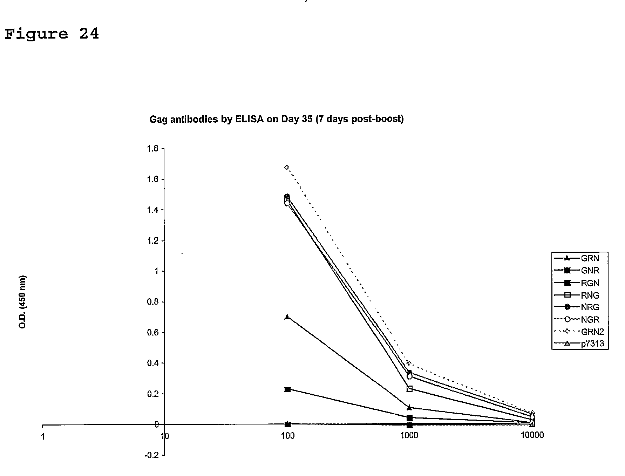

Blood samples were collected for antibody analysis at 7 and 14 days post-boost from two experiments. Serum was separated and stored frozen until antibody titres could be measured using specific ELISA assays. All samples were tested for antibodies to Gag, Nef and RT. Briefly, ELISA plates were coated with the relevant protein. Excess protein was washed off before diluted serum samples were incubated in the wells. The serum samples were washed off and anti-mouse antiserum conjugated to an appropriate tag was added. The plate was developed and read on a plate reader. The results are shown in figure 24.

### 3. Antibody Data

Antibody titres were measured for all six constructs in four experiments. Construct p73i-GNR consistently generated no antibody responses to Gag and limited antibody responses to Nef. The reason for this is unclear, as T-cell responses were observed from splenocytes isolated from the same mice, indicating that the Gag protein was being expressed *in vivo.*

The ranking for the generation of Gag specific antibodies was:
RNG>GRN>NRG>RGN>NGR>GNR

### Analysis cellular immunology data

The objective was to rank the 6 constructs on the basis of spot count data from 3 immunology experiments. Three sets of responses were assessed:
CD8 responses to Gag, Nef and RT at Day 7 (7 days post primary),
CD4 responses to Gag and RT at Day 35 (7 days post boost),
CD8 responses to Gag, Nef and RT at Day 35 (7 days post boost) .

Each response (e.g. CD8 response to Gag) was modeled using a linear mixed effect model in SAS version 8. The model included fixed effects of construct, whether the particular antigen (Gag, Nef or RT) was present or absent, and whether IL-2 was present or absent. In addition, for CD8 responses, where data were available from each individual mouse, subject was included as a random effect in the model. The model included interaction terms to allow for a different effect of construct for each combination of the antigen (present/absent) and IL-2 (present/absent).

From the model, the difference in adjusted mean response between each construct and p7313 (the control group) was estimated separately for each combination of antigen (present/absent) and IL-2 (present/absent), together with a p-value indicating whether the difference was statistically significant. Based on the differences and p-values in the presence of the antigen and the absence of IL-2, constructs were ranked, by assigning a score of 6 to the construct with the largest difference, 5 to the next largest, etc, but 0 to any constructs where the difference was not statistically significant at the 5% level.

The assumptions of the model - that the residuals were normally distributed with constant variance, were assessed using graphical methods and sensitivity analyses, where first a log and second a square root transformation of the response was modeled. The ranking of the constructs was not sensitive to departures from the assumptions of the model.

Having calculated the ranks for each response in each experiment separately, total ranks for the 3 sets of responses were calculated across all 3 experiments. The following table shows the total rankings across the 3 experiments.

**Total rankings of constructs for each of 3 sets of responses, combined across 3 immunology experiments.**

| **Construct** | **Day 7 (7 days post primary)** | **Day 35 (7 days post boost)** | |
|---|---|---|---|
| | CD8 | CD4 | CD8 |
| GRN | 5 | 18 | 3 |
| GNR | 17 | 24 | 28 |
| RGN | **28** | 23 | 33 |
| RNG | 25 | **27** | **37** |
| NRG | 25 | 19 | 0 |
| NGR | 4 | 14 | 10 |

RNG has the highest ranking for both sets of responses at Day 35, and the second highest ranking behind RGN at Day 7. RGN also receives high rankings for both sets of responses at Day 35.

The invention is further described by the following numbered statements:
1. A nucleotide sequence that encodes an HIV-1 gag protein or fragment containing a gag epitope thereof and a second HIV antigen or a fragment encoding an epitope of said second HIV antigen, operably linked to a heterologous promoter.
2. A nucleotide sequence according to statement 1 wherein the second antigen is selected from the group consisting of: Nef or a fragment thereof containing a nef epitope, or reverse transcriptase (RT) or a fragment thereof containing an RT epitope.
3. A nucleotide sequence according to statement 1 or 2 wherein the gag protein comprises p17.
4. A nucleotide sequence according to statement 3 wherein the gag protein additionally comprises p24.
5. A nucleotide sequence according to any one of statements 1 to 4 wherein the gag sequence is codon optimised to resemble the codon usage in a highly expressed human gene.
6. A nucleotide sequence according to any one of statements 1-5 wherein the sequence encodes an HIV-1 gag protein or fragment containing a gag epitope, a nef protein or a fragment containing a nef epitope and an RT protein or a fragment containing an RT epitope, preferably in the order RT, Gag, Nef or RT, Nef, Gag.
7. A nucleotide sequence according to any one of statements 2 to 6 wherein the RT sequence or fragment thereof is codon optimised to resemble a highly expressed human gene.
8. A nucleotide sequence according to any one of statements 1 to 7 wherein the nucleotide sequence encodes a nef protein or epitope thereof.
9. A nucleotide sequence selected from the group consisting of:
   - Gag (p17,p24), Nef truncate
   - Gag (p17,p24) (codon optimised), Nef (truncate)
   - Gag (p17,p24), RT, Nef (truncate)
   - Gag (p17,p24) codon optimised, RT, Nef (truncate)
   - Gag (p17,p24) codon optimised, RT codon optimised, Nef truncate
   - RT (codon optimised), Gag (p17, p24) codon optimised, Nef truncate
   - RT (codon optimised), Nef truncate, gag p17, p24 codon optimised
10. A nucleotide sequence according to any one of statements 1 to 9 wherein the heterologous promoter is the promoter from HCMV IE gene.
11. A nucleotide sequence according to statement 10 wherein the 5' of the promoter comprises exon 1.
12. A vector comprising a nucleotide sequence according to any one of statements 1 to 11.
13. A vector according to statement 12 which is a double stranded DNA plasmid.
14. A pharmaceutical composition comprising a nucleotide sequence according to any one of statements 1 to 11 or a vector according to statement 12 or 13 and a pharmaceutically acceptable excipient, diluent, carrier or adjuvant.
15. A pharmaceutical composition according to statement 14 adapted for intra-muscular or intra-dermal delivery.
16. A pharmaceutical composition according to statement 14 or 15 wherein the carrier is a gold bead.
17. An intra-dermal delivery device comprising a pharmaceutical composition according to any one of statements 14, 15 or 16.
18. A method of treating a patient suffering from or susceptible to a disease comprising administration of a safe and effective amount of a pharmaceutical composition according to any one of statements 14 to 16.
19. A nucleotide sequence according to any one of statements 1 to 11, a vector according to statement 12 or 13, or a composition according to any one of statements 14 to 16, for use in medicine.
20. Use of a nucleotide sequence according to any one of statements 1 to 11 in the manufacture of a medicament for the treatment of disease.
21. A process for the production of a nucleotide according to any one of statements 1 to 11 comprising operably linking a nucleotide sequence encoding an HIV-1 gag protein or fragment thereof and a second HIV protein or fragement thereof to a heterologous promoter sequence.

## Claims

1. A nucleotide sequence that encodes an HIV-1 gag protein or fragment containing a gag epitope thereof, an HIV-1 Nef protein or a fragment containing a Nef epitope thereof, and an RT protein or a fragment containing an RT epitope thereof, operably linked to a heterologous promoter, wherein the order of the sequence is Gag, RT, Nef.

2. A nucleotide sequence as claimed in claim 1 wherein the gag protein comprises p17.

3. A nucleotide sequence as claimed in claim 2 wherein the gag protein additionally comprises p24.

4. A nucleotide sequence as claimed in any one of claims 1 to 3 wherein the gag sequence or fragment thereof is codon optimised to resemble the codon usage in a highly expressed human gene.

5. A nucleotide sequence as claimed in any of claims 1 to 4 wherein the RT sequence or fragment thereof is codon optimised to resemble the codon usage in a highly expressed human gene.

6. A nucleotide sequence selected from the group consisting of:
a. Gag (p17,p24), RT, Nef (truncate);
b. Gag (p17,p24) codon optimised, RT, Nef (truncate);
c. Gag (p17,p24) codon optimised, RT codon optimised, Nef truncate.

7. A nucleotide sequence as claimed in any of claims 1 to 6 wherein the heterologous promoter is the promoter from HCMV IE gene.

8. A nucleotide sequence as claimed in claim 7 wherein the 5' of the promoter comprises exon 1.

9. A vector comprising a nucleotide sequence as claimed in any one of claims 1 to 6.

10. A vector as claimed in claim 9 which is an adenovirus.

11. A vector as claimed in claim 9 which is a double stranded DNA plasmid.

12. A pharmaceutical composition comprising a nucleotide sequence as claimed in any one of claims 1 to 6 or a vector as claimed in any one of claims 9 to 11 and a pharmaceutically acceptable excipient, diluent, carrier or adjuvant.

13. A pharmaceutical composition as claimed in claim 12 adapted for intra-muscular or intra-dermal delivery.

14. A pharmaceutical composition as claimed in either claim 13 or 14 wherein the carrier is a gold bead.

15. An intra-dermal delivery device comprising a pharmaceutical composition as claimed in any one of claims 12 to 14.

16. A nucleotide sequence as claimed in any one of claims 1 to 6, a vector as claimed in any one of claims 9 to 11, or a composition as claimed in any one of claims 12 to 14, for use in medicine.

17. A process for the production of a nucleotide as claimed in any one of claims 1 to 6 comprising operably linking a nucleotide sequence encoding an HIV-1 gag protein or fragment containing a gag epitope thereof, a nucleotide sequence encoding an HIV-1 Nef protein or a fragment containing a Nef epitope thereof, and a nucleotide sequence encoding an RT protein or a fragment containing an RT epitope thereof, to a heterologous promoter sequence.


SEQUENCE LISTING

<110> Glaxo group limited

<120> Vaccines

<130> PG4653

<140> PCT/epo210592
<141> 2002-09-18

<160> 84

<170> FastSEQ for Windows Version 4.0

<210> 1
<211> 42
<212> DNA
<213> Artificial Sequence

<220>
<223> Nef primer

<400> 1
ataagaatgc ggccgccatg gtgggttttc cagtcacacc tt                42

<210> 2
<211> 31
<212> DNA
<213> Artifical sequence

<400> 2
cgcggatcct cagcagttct tgaagtactc c                            31

<210> 3
<211> 44
<212> DNA
<213> Artificial Sequence

<220>
<223> srt primer

<400> 3
ataagaatgc ggccgccatg ggccccatta gccctattga gact              44

<210> 4
<211> 44
<212> DNA
<213> Artificial Sequence

<220>
<223> srt primer

<400> 4
ataagaatgc ggccgccatg ggccccatta gccctattga gact              44

<210> 5
<211> 37
<212> DNA
<213> Artificial Sequence

<220>
<223> sp17p24primer

<400> 5
ataagaatgc ggccgccatg ggtgcccgag cttcggt                      37

<210> 6
<211> 30
<212> DNA
<213> Artificial Sequence

<220>
<223> sp17p24primer

<400> 6
tggggcccat caacactctg gctttgtgtc                                        30

<210> 7
<211> 30
<212> DNA
<213> Artificial Sequence

<220>
<223> linker

<400> 7
cagagtgttg atgggcccca ttagccctat                                        30

<210> 8
<211> 30
<212> DNA
<213> Artificial Sequence

<220>
<223> linker

<400> 8
aacccaccat atctaaaaat agtactttcc                                        30

<210> 9
<211> 32
<212> DNA
<213> Artificial Sequence

<220>
<223> linker

<400> 9
ctatttttag atatggtggg ttttccagtc ac                                     32

<210> 10
<211> 31
<212> DNA
<213> Artificial Sequence

<220>
<223> linker

<400> 10
cgcggatcct cagcagttct tgaagtactc c                                      31

<210> 11
<211> 37
<212> DNA
<213> Artificial Sequence

<220>
<223> PCR primer

<400> 11
ataagaatgc ggccgccatg ggtgcccgag cttcggt                                37

<210> 12
<211> 51
<212> DNA
<213> Artificial Sequence

<220>
<223> PCR primer

<400> 12
gcgcacgatc ttgttcaggc ccaggatgat ccaccgttta tagatttctc c                51

<210> 13
<211> 49
<212> DNA
<213> Artificial Sequence

<220>

<223> PCR primer

<400> 13
atcctgggcc tgaacaagat cgtgcgcatg tactctccga catccatcc 49

<210> 14
<211> 30
<212> DNA
<213> Artificial Sequence

<220>
<223> PCR primer

<400> 14
tggggcccat caacactctg gctttgtgtc 30

<210> 15
<211> 68
<212> DNA
<213> Artificial Sequence

<220>
<223> PCR primer

<400> 15
gaattcgcgg ccgcgatggg ccccatcagt cccatcgaga ccgtgccggt gaagctgaaa 60
cccgggat 68

<210> 16
<211> 44
<212> DNA
<213> Artificial Sequence

<220>
<223> PCR primer

<400> 16
ggtgtgactg gaaaacccac catcagcacc tttctaatcc ccgc 44

<210> 17
<211> 23
<212> DNA
<213> Artificial Sequence

<220>
<223> PCR primer

<400> 17
atggtgggtt ttccagtcac acc 23

<210> 18
<211> 29
<212> DNA
<213> Artificial Sequence

<220>
<223> PCR primer

<400> 18
gatgaaatgc taggcggctg tcaaacctc 29

<210> 19
<211> 29
<212> DNA
<213> Artificial Sequence

<220>
<223> PCR primer

<400> 19
gaggtttgac agccgcctag catttcatc 29

<210> 20
<211> 31
<212> DNA
<213> Artificial Sequence

<220>
<223> PCR primer

<400> 20
cgcggatcct cagcagttct tgaagtactc c                                    31

<210> 21
<211> 23
<212> DNA
<213> Artificial Sequence

<220>
<223> PCR primer

<400> 21
atggtgggtt ttccagtcac acc                                             23

<210> 22
<211> 29
<212> DNA
<213> Artificial Sequence

<220>
<223> PCR primer

<400> 22
gatgaaatgc taggcggctg tcaaacctc                                       29

<210> 23
<211> 29
<212> DNA
<213> Artificial Sequence

<220>
<223> PCR primer

<400> 23
gaggtttgac agccgcctag catttcatc                                       29

<210> 24
<211> 31
<212> DNA
<213> Artificial Sequence

<220>
<223> PCR primer

<400> 24
cgcggatcct cagcagttct tgaagtactc c                                    31

<210> 25
<211> 68
<212> DNA
<213> Artificial Sequence

<220>
<223> PCR primer

<400> 25
gaattcgcgg ccgcgatggg ccccatcagt cccatcgaga ccgtgccggt gaagctgaaa 60
cccgggat                                                              68

<210> 26
<211> 39
<212> DNA
<213> Artificial Sequence

<220>
<223> PCR primer

<400> 26
ggagctcgta gcccatcttc aggaatggcg gctccttct                            39

<210> 27
<211> 68

<212> DNA
<213> Artificial Sequence

<220>
<223> PCR primer

<400> 27
gaattcggat ccttacagca cctttctaat ccccgcactc accagcttgt cgacctgctc 60
gttgccgc 68

<210> 28
<211> 26
<212> DNA
<213> Artificial Sequence

<220>
<223> PCR primer

<400> 28
cctgaagatg ggctacgagc tccatg 26

<210> 29
<211> 32
<212> DNA
<213> Artificial Sequence

<220>
<223> PCR primer

<400> 29
cattagagcg gccgcgatgg tgggttttcc ac 32

<210> 30
<211> 42
<212> DNA
<213> Artificial Sequence

<220>
<223> PCR primer

<400> 30
gatgggactg atggggccca tgcagttctt gaactactcc gg 42

<210> 31
<211> 24
<212> DNA
<213> Artificial Sequence

<220>
<223> PCR primer

<400> 31
atgggcccca tcagtcccat cgag 24

<210> 32
<211> 45
<212> DNA
<213> Artificial Sequence

<220>
<223> PCR primer

<400> 32
cagtaccgaa gctcgggcac ccatcagcac ctttctaatc cccgc 45

<210> 33
<211> 24
<212> DNA
<213> Artificial Sequence

<220>
<223> PCR primer

<400> 33
atgggtgccc gagcttcggt actg 24

<210> 34
<211> 36
<212> DNA
<213> Artificial Sequence

<220>
<223> PCR primer

<400> 34
gatgggggat cctcacaaca ctctggcttt gtgtcc                                36

<210> 35
<211> 24
<212> DNA
<213> Artificial Sequence

<220>
<223> PCR primer

<400> 35
atgggtgccc gagcttcggt actg                                             24

<210> 36
<211> 68
<212> DNA
<213> Artificial Sequence

<220>
<223> PCR primer

<400> 36
gaattcggat ccttacagca cctttctaat ccccgcactc accagcttgt cgacctgctc 60
gttgccgc                                                               68

<210> 37
<211> 32
<212> DNA
<213> Artificial Sequence

<220>
<223> PCR primer

<400> 37
cattagagcg gccgcgatgg tgggttttcc ac                                    32

<210> 38
<211> 45
<212> DNA
<213> Artificial Sequence

<220>
<223> PCR primer

<400> 38
cagtaccgaa gctcgggcac ccatgcagtt cttgaactac tccgg                      45

<210> 39
<211> 24
<212> DNA
<213> Artificial Sequence

<220>
<223> PCR primer

<400> 39
atgggtgccc gagcttcggt actg                                             24

<210> 40
<211> 68
<212> DNA
<213> Artificial Sequence

<220>
<223> PCR primer

<400> 40

gaattcgcgg ccgcgatggg ccccatcagt cccatcgaga ccgtgccggt gaagctgaaa 60
cccgggat 68

<210> 41
<211> 45
<212> DNA
<213> Artificial Sequence

<220>
<223> PCR primer

<400> 41
cagtaccgaa gctcgggcac ccatcagcac ctttctaatc cccgc 45

<210> 42
<211> 68
<212> DNA
<213> Artificial Sequence

<220>
<223> PCR primer

<400> 42
gaattcgcgg ccgcgatggg ccccatcagt cccatcgaga ccgtgccggt gaagctgaaa 60
cccgggat 68

<210> 43
<211> 45
<212> DNA
<213> Artificial Sequence

<220>
<223> PCR primer

<400> 43
cagtaccgaa gctcgggcac ccatgcagtt cttgaactac tccgg 45

<210> 44
<211> 24
<212> DNA
<213> Artificial Sequence

<220>
<223> PCR primer

<400> 44
atgggtgccc gagcttcggt actg 24

<210> 45
<211> 36
<212> DNA
<213> Artificial Sequence

<220>
<223> PCR primer

<400> 45
gatgggggat cctcacaaca ctctggcttt gtgtcc 36

<210> 46
<211> 24
<212> DNA
<213> Artificial Sequence

<220>
<223> PCR primer

<400> 46
atgggtgccc gagcttcggt actg 24

<210> 47
<211> 42
<212> DNA
<213> Artificial Sequence

<220>

<223> PCR primer

<400> 47
gatgggactg atggggccca tgcagttctt gaactactcc gg                    42

<210> 48
<211> 24
<212> DNA
<213> Artificial Sequence

<220>
<223> PCR primer

<400> 48
atgggcccca tcagtcccat cgag                                        24

<210> 49
<211> 68
<212> DNA
<213> Artificial Sequence

<220>
<223> PCR primer

<400> 49
gaattcggat ccttacagca cctttctaat ccccgcactc accagcttgt cgacctgctc 60
gttgccgc                                                          68

<210> 50
<211> 1503
<212> DNA
<213> HIV

<400> 50
   atgggtgccc gagcttcggt actgtctggt ggagagctgg acagatggga gaaaattagg 60
   ctgcgcccgg gaggcaaaaa gaaatacaag ctcaagcata tcgtgtgggc ctcgagggag 120
   cttgaacggt ttgccgtgaa cccaggcctg ctggaaacat ctgagggatg tcgccagatc 180
   ctggggcaat tgcagccatc cctccagacc gggagtgaag agctgaggtc cttgtataac 240
   acagtggcta ccctctactg cgtacaccag aggatcgaga ttaaggatac caaggaggcc 300
   ttggacaaaa ttgaggagga gcaaaacaag agcaagaaga aggcccagca ggcagctgct 360
   gacactgggc atagcaacca ggtatcacag aactatccta ttgtccaaaa cattcagggc 420
   cagatggttc atcaggccat cagcccccgg acgctcaatg cctgggtgaa ggttgtcgaa 480
   gagaaggcct tttctcctga ggttatcccc atgttctccg ctttgagtga gggggccact 540
   cctcaggacc tcaatacaat gcttaatacc gtgggcggcc atcaggccgc catgcaaatg 600
   ttgaaggaga ctatcaacga ggaggcagcc gagtgggaca gagtgcatcc cgtccacgct 660
   ggcccaatcg cgcccggaca gatgcgggag cctcgcggct ctgacattgc cggcaccacc 720
   tctacactgc aagagcaaat cggatggatg accaacaatc ctcccatccc agttggagaa 780
   atctataaac ggtggatcat tctcggtctc aataaaattg ttagaatgta ctctccgaca 840
   tccatccttg acattagaca gggacccaaa gagcctttta gggattacgt cgaccggttt 900
   tataagaccc tgcgagcaga gcaggcctct caggaggtca aaaactggat gacggagaca 960
   ctcctggtac agaacgctaa ccccgactgc aaaacaatct tgaaggcact aggcccggct 1020
   gccaccctgg aagagatgat gaccgcctgt cagggagtag gcggacccgg acacaaagcc 1080
   agagtgttgg ccgaagccat gagccaggtg acgaactccg caaccatcat gatgcagaga 1140
   gggaacttcc gcaatcagcg gaagatcgtg aagtgtttca attgcggcaa ggagggtcat 1200
   accgcccgca actgtcgggc ccctaggaag aaagggtgtt ggaagtgcgg caaggaggga 1260
   caccagatga aagactgtac agaacgacag gccaattttc ttggaaagat ttggccgagc 1320
   tacaagggga gacctggtaa tttcctgcaa agcaggcccg agcccaccgc cccccctgag 1380
   gaatccttca ggtccggagt ggagaccaca acgcctcccc aaaaacagga accaatcgac 1440
   aaggagctgt accctttaac ttctctgcgt tctctctttg gcaacgaccc gtcgtctcaa 1500
   taa                                                               1503

<210> 51
<211> 500
<212> PRT
<213> HIV

<400> 51
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn

65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Ala Glu Ala Met Ser
        355                 360                 365
Gln Val Thr Asn Ser Ala Thr Ile Met Met Gln Arg Gly Asn Phe Arg
    370                 375                 380
Asn Gln Arg Lys Ile Val Lys Cys Phe Asn Cys Gly Lys Glu Gly His
385                 390                 395                 400
Thr Ala Arg Asn Cys Arg Ala Pro Arg Lys Lys Gly Cys Trp Lys Cys
                405                 410                 415
Gly Lys Glu Gly His Gln Met Lys Asp Cys Thr Glu Arg Gln Ala Asn
            420                 425                 430
Phe Leu Gly Lys Ile Trp Pro Ser Tyr Lys Gly Arg Pro Gly Asn Phe
        435                 440                 445
Leu Gln Ser Arg Pro Glu Pro Thr Ala Pro Pro Glu Glu Ser Phe Arg
    450                 455                 460
Ser Gly Val Glu Thr Thr Thr Pro Pro Gln Lys Gln Glu Pro Ile Asp
465                 470                 475                 480
Lys Glu Leu Tyr Pro Leu Thr Ser Leu Arg Ser Leu Phe Gly Asn Asp
                485                 490                 495
Pro Ser Ser Gln
            500

<210> 52
<211> 1515
<212> DNA
<213> HIV

<400> 52
atgggtgcga gagcgtcagt attaagcggg ggagaattag atcgatggga aaaaattcgg 60
ttaaggccag ggggaaagaa aaaatataaa ttaaaacata tagtatgggc aagcagggag 120
ctagaacgat tcgcagttaa tcctggcctg ttagaaacat cagaaggctg tagacaaata 180
ctgggacagc tacaaccatc ccttcagaca ggatcagaag aacttagatc attatataat 240
acagtagcaa ccctctattg tgtgcatcaa aggatagaga taaaagacac caaggaagct 300
ttagacaaga tagaggaaga gcaaaacaaa agtaagaaaa aagcacagca agcagcagct 360
gacacaggac acagcaatca ggtcagccaa aattacccta tagtgcagaa catccagggg 420
caaatggtac atcaggccat atcacctaga actttaaatg catgggtaaa agtagtagaa 480
gagaaggctt tcagcccaga agtgataccc atgttttcag cattatcaga aggagccacc 540
ccacaagatt taaacaccat gctaaacaca gtggggggac atcaagcagc catgcaaatg 600
ttaaaagaga ccatcaatga ggaagctgca gaatgggata gagtgcatcc agtgcatgca 660
gggcctattg caccaggcca gatgagagaa ccaaggggaa gtgacatagc aggaactact 720
agtacccttc aggaacaaat aggatggatg acaaataatc cacctatccc agtaggagaa 780

atttataaaa gatggataat cctgggatta aataaaatag taagaatgta tagccctacc 840
agcattctgg acataagaca aggaccaaaa gaacccttta gagactatgt agaccggttc 900
tataaaactc taagagccga gcaagcttca caggaggtaa aaaattggat gacagaaacc 960
ttgttggtcc aaaatgcgaa cccagattgt aagactattt taaaagcatt gggaccagcg 1020
gctacactag aagaaatgat gacagcatgt cagggagtag gaggacccgg ccataaggca 1080
agagttttgg tgggttttcc agtcacacct caggtacctt taagaccaat gacttacaag 1140
gcagctgtag atcttagcca ctttttaaaa gaaaaggggg gactggaagg gctaattcac 1200
tcccaaagaa gacaagatat ccttgatctg tggatctacc acacacaagg ctacttccct 1260
gattggcaga actacacacc agggccaggg gtcagatatc cactgacctt tggatggtgc 1320
tacaagctag taccagttga gccagataag gtagaagagg ccaataaagg agagaacacc 1380
agcttgttac accctgtgag cctgcatggg atggatgacc cggagagaga agtgttagag 1440
tggaggtttg acagccacct agcatttcat cacgtggccc gagagctgca tccggagtac 1500
ttcaagaact gctga 1515

<210> 53
<211> 504
<212> PRT
<213> HIV

<400> 53
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Val Gly Phe Pro Val
        355                 360                 365
Thr Pro Gln Val Pro Leu Arg Pro Met Thr Tyr Lys Ala Ala Val Asp
    370                 375                 380
Leu Ser His Phe Leu Lys Glu Lys Gly Gly Leu Glu Gly Leu Ile His
385                 390                 395                 400
Ser Gln Arg Arg Gln Asp Ile Leu Asp Leu Trp Ile Tyr His Thr Gln
                405                 410                 415
Gly Tyr Phe Pro Asp Trp Gln Asn Tyr Thr Pro Gly Pro Gly Val Arg
            420                 425                 430
Tyr Pro Leu Thr Phe Gly Trp Cys Tyr Lys Leu Val Pro Val Glu Pro
        435                 440                 445

Asp Lys Val Glu Glu Ala Asn Lys Gly Glu Asn Thr Ser Leu Leu His
    450                 455                 460
Pro Val Ser Leu His Gly Met Asp Asp Pro Glu Arg Glu Val Leu Glu
465                 470                 475                 480
Trp Arg Phe Asp Ser His Leu Ala Phe His His Val Ala Arg Glu Leu
                485                 490                 495
His Pro Glu Tyr Phe Lys Asn Cys
            500

<210> 54
<211> 1518
<212> DNA
<213> HIV

<400> 54
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300
ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat tctcggtctc aataaaattg ttagaatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tggtgggttt tccagtcaca cctcaggtac ctttaagacc aatgacttac 1140
aaggcagctg tagatcttag ccacttttta aaagaaaagg ggggactgga agggctaatt 1200
cactcccaaa gaagacaaga tatccttgat ctgtggatct accacacaca aggctacttc 1260
cctgattggc agaactacac accagggcca ggggtcagat atccactgac ctttggatgg 1320
tgctacaagc tagtaccagt tgagccagat aaggtagaag aggccaataa aggagagaac 1380
accagcttgt tacaccctgt gagcctgcat gggatggatg acccggagag agaagtgtta 1440
gagtggaggt ttgacagcca cctagcattt catcacgtgg cccgagagct gcatccggag 1500
tacttcaaga actgctga 1518

<210> 55
<211> 505
<212> PRT
<213> HIV

<400> 55
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala

210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Met Val Gly Phe Pro
        355                 360                 365
Val Thr Pro Gln Val Pro Leu Arg Pro Met Thr Tyr Lys Ala Ala Val
    370                 375                 380
Asp Leu Ser His Phe Leu Lys Glu Lys Gly Gly Leu Glu Gly Leu Ile
385                 390                 395                 400
His Ser Gln Arg Arg Gln Asp Ile Leu Asp Leu Trp Ile Tyr His Thr
                405                 410                 415
Gln Gly Tyr Phe Pro Asp Trp Gln Asn Tyr Thr Pro Gly Pro Gly Val
            420                 425                 430
Arg Tyr Pro Leu Thr Phe Gly Trp Cys Tyr Lys Leu Val Pro Val Glu
        435                 440                 445
Pro Asp Lys Val Glu Glu Ala Asn Lys Gly Glu Asn Thr Ser Leu Leu
    450                 455                 460
His Pro Val Ser Leu His Gly Met Asp Asp Pro Glu Arg Glu Val Leu
465                 470                 475                 480
Glu Trp Arg Phe Asp Ser His Leu Ala Phe His His Val Ala Arg Glu
                485                 490                 495
Leu His Pro Glu Tyr Phe Lys Asn Cys
            500                 505

<210> 56
<211> 1689
<212> DNA
<213> HIV

<400> 56
atgggcccca tcagtcccat cgagaccgtg ccggtgaagc tgaaacccgg gatggacggc 60
cccaaggtca agcagtggcc actcaccgag gagaagatca aggccctggt ggagatctgc 120
accgagatgg agaaagaggg caagatcagc aagatcgggc ctgagaaccc atacaacacc 180
cccgtgtttg ccatcaagaa gaaggacagc accaagtggc gcaagctggt ggatttccgg 240
gagctgaata agcggaccca ggatttctgg gaggtccagc tgggcatccc ccatccggcc 300
ggcctgaaga agaagaagag cgtgaccgtg ctggacgtgg gcgacgctta cttcagcgtc 360
cctctggacg aggactttag aaagtacacc gcctttacca tcccatctat caacaacgag 420
acccctggca tcagatatca gtacaacgtc ctcccccagg gctggaaggg ctctcccgcc 480
attttccaga gctccatgac caagatcctg gagccgtttc ggaagcagaa ccccgatatc 540
gtcatctacc agtacatgga cgacctgtac gtgggctctg acctggaaat cgggcagcat 600
cgcacgaaga ttgaggagct gaggcagcat ctgctgagat ggggcctgac cactccggac 660
aagaagcatc agaaggagcc gccattcctg tggatgggct acgagctcca tcccgacaag 720
tggaccgtgc agcctatcgt cctccccgag aaggacagct ggaccgtgaa cgacatccag 780
aagctggtgg gcaagctcaa ctgggctagc cagatctatc ccgggatcaa ggtgcgccag 840
ctctgcaagc tgctgcgcgg caccaaggcc ctgaccgagg tgattcccct cacggaggaa 900
gccgagctcg agctggctga gaaccgggag atcctgaagg agcccgtgca cggcgtgtac 960
tatgacccct ccaaggacct gatcgccgaa atccagaagc agggccaggg gcagtggaca 1020
taccagattt accaggagcc tttcaagaac ctcaagaccg gcaagtacgc ccgcatgagg 1080
ggcgcccaca ccaacgatgt caagcagctg accgaggccg tccagaagat cacgaccgag 1140
tccatcgtga tctgggggaa gacacccaag ttcaagctgc ctatccagaa ggagacctgg 1200
gagacgtggt ggaccgaata ttggcaggcc acctggattc ccgagtggga gttcgtgaat 1260
acacctcctc tggtgaagct gtggtaccag ctcgagaagg agcccatcgt gggcgcggag 1320
acattctacg tggacggcgc ggccaaccgc gaaacaaagc tcgggaaggc cgggtacgtc 1380
accaaccggg gccgccagaa ggtcgtcacc ctgaccgaca ccaccaacca gaagacggag 1440
ctgcaggcca tctatctcgc tctccaggac tccggcctgg aggtgaacat cgtgacggac 1500
agccagtacg cgctgggcat tattcaggcc cagccggacc agtccgagag cgaactggtg 1560
aaccagatta tcgagcagct gatcaagaaa gagaaggtct acctcgcctg ggtcccggcc 1620
cataagggca ttggcggcaa cgagcaggtc gacaagctgg tgagtgcggg gattagaaag 1680
gtgctgtaa                                                         1689

<210> 57

<211> 562
<212> PRT
<213> HIV

<400> 57
Met Gly Pro Ile Ser Pro Ile Glu Thr Val Ser Val Lys Leu Lys Pro
1               5                   10                  15
Gly Met Asp Gly Pro Lys Val Lys Gln Trp Pro Leu Thr Glu Glu Lys
            20                  25                  30
Ile Lys Ala Leu Val Glu Ile Cys Thr Glu Met Glu Lys Glu Gly Lys
        35                  40                  45
Ile Ser Lys Ile Gly Pro Glu Asn Pro Tyr Asn Thr Pro Val Phe Ala
    50                  55                  60
Ile Lys Lys Lys Asp Ser Thr Lys Trp Arg Lys Leu Val Asp Phe Arg
65                  70                  75                  80
Glu Leu Asn Lys Arg Thr Gln Asp Phe Trp Glu Val Gln Leu Gly Ile
                85                  90                  95
Pro His Pro Ala Gly Leu Lys Lys Lys Lys Ser Val Thr Val Leu Asp
            100                 105                 110
Val Gly Asp Ala Tyr Phe Ser Val Pro Leu Asp Glu Asp Phe Arg Lys
        115                 120                 125
Tyr Thr Ala Phe Thr Ile Pro Ser Ile Asn Asn Glu Thr Pro Gly Ile
    130                 135                 140
Arg Tyr Gln Tyr Asn Val Leu Pro Gln Gly Trp Lys Gly Ser Pro Ala
145                 150                 155                 160
Ile Phe Gln Ser Ser Met Thr Lys Ile Leu Glu Pro Phe Arg Lys Gln
                165                 170                 175
Asn Pro Asp Ile Val Ile Tyr Gln Tyr Met Asp Asp Leu Tyr Val Gly
            180                 185                 190
Ser Asp Leu Glu Ile Gly Gln His Arg Thr Lys Ile Glu Glu Leu Arg
        195                 200                 205
Gln His Leu Leu Arg Trp Gly Leu Thr Thr Pro Asp Lys Lys His Gln
    210                 215                 220
Lys Glu Pro Pro Phe Leu Trp Met Gly Tyr Glu Leu His Pro Asp Lys
225                 230                 235                 240
Trp Thr Val Gln Pro Ile Val Leu Pro Glu Lys Asp Ser Trp Thr Val
                245                 250                 255
Asn Asp Ile Gln Lys Leu Val Gly Lys Leu Asn Trp Ala Ser Gln Ile
            260                 265                 270
Tyr Pro Gly Ile Lys Val Arg Gln Leu Cys Lys Leu Leu Arg Gly Thr
        275                 280                 285
Lys Ala Leu Thr Glu Val Ile Pro Leu Thr Glu Glu Ala Glu Leu Glu
    290                 295                 300
Leu Ala Glu Asn Arg Glu Ile Leu Lys Glu Pro Val His Gly Val Tyr
305                 310                 315                 320
Tyr Asp Pro Ser Lys Asp Leu Ile Ala Glu Ile Gln Lys Gln Gly Gln
                325                 330                 335
Gly Gln Trp Thr Tyr Gln Ile Tyr Gln Glu Pro Phe Lys Asn Leu Lys
            340                 345                 350
Thr Gly Lys Tyr Ala Arg Met Arg Gly Ala His Thr Asn Asp Val Lys
        355                 360                 365
Gln Leu Thr Glu Ala Val Gln Lys Ile Thr Thr Glu Ser Ile Val Ile
    370                 375                 380
Trp Gly Lys Thr Pro Lys Phe Lys Leu Pro Ile Gln Lys Glu Thr Trp
385                 390                 395                 400
Glu Thr Trp Trp Thr Glu Tyr Trp Gln Ala Thr Trp Ile Pro Glu Trp
                405                 410                 415
Glu Phe Val Asn Thr Pro Pro Leu Val Lys Leu Trp Tyr Gln Leu Glu
            420                 425                 430
Lys Glu Pro Ile Val Gly Ala Glu Thr Phe Tyr Val Asp Gly Ala Ala
        435                 440                 445
Asn Arg Glu Thr Lys Leu Gly Lys Ala Gly Tyr Val Thr Asn Arg Gly
    450                 455                 460
Arg Gln Lys Val Val Thr Leu Thr Asp Thr Thr Asn Gln Lys Thr Glu
465                 470                 475                 480
Leu Gln Ala Ile Tyr Leu Ala Leu Gln Asp Ser Gly Leu Glu Val Asn
                485                 490                 495
Ile Val Thr Asp Ser Gln Tyr Ala Leu Gly Ile Ile Gln Ala Gln Pro
            500                 505                 510
Asp Gln Ser Glu Ser Glu Leu Val Asn Gln Ile Ile Glu Gln Leu Ile
        515                 520                 525
Lys Lys Glu Lys Val Tyr Leu Ala Trp Val Pro Ala His Lys Gly Ile
    530                 535                 540
Gly Gly Asn Glu Gln Val Asp Lys Leu Val Ser Ala Gly Ile Arg Lys
545                 550                 555                 560
Val Leu

<210> 58
<211> 1689
<212> DNA
<213> HIV

<400> 58
atgggcccca tcagtcccat cgagaccgtg ccggtgaagc tgaaacccgg gatggacggc 60
cccaaggtca agcagtggcc actcaccgag gagaagatca aggccctggt ggagatctgc 120
accgagatgg agaaagaggg caagatcagc aagatcgggc ctgagaaccc atacaacacc 180
cccgtgtttg ccatcaagaa gaaggacagc accaagtggc gcaagctggt ggatttccgg 240
gagctgaata agcggaccca ggatttctgg gaggtccagc tgggcatccc ccatccggcc 300
ggcctgaaga agaagaagag cgtgaccgtg ctggacgtgg gcgacgctta cttcagcgtc 360
cctctggacg aggactttag aaagtacacc gcctttacca tcccatctat caacaacgag 420
acccctggca tcagatatca gtacaacgtc ctcccccagg gctggaaggg ctctcccgcc 480
attttccaga gctccatgac caagatcctg gagccgtttc ggaagcagaa ccccgatatc 540
gtcatctacc agtacatgga cgacctgtac gtgggctctg acctggaaat cgggcagcat 600
cgcacgaaga ttgaggagct gaggcagcat ctgctgagat ggggcctgac cactccggac 660
aagaagcatc agaaggagcc gccattcctg tggatgggct acgagctcca tcccgacaag 720
tggaccgtgc agcctatcgt cctccccgag aaggacagct ggaccgtgaa cgacatccag 780
aagctggtgg gcaagctcaa ctgggctagc cagatctatc ccgggatcaa ggtgcgccag 840
ctctgcaagc tgctgcgcgg caccaaggcc ctgaccgagg tgattcccct cacggaggaa 900
gccgagctcg agctggctga gaaccgggag atcctgaagg agcccgtgca cggcgtgtac 960
tatgacccct ccaaggacct gatcgccgaa atccagaagc agggccaggg gcagtggaca 1020
taccagattt accaggagcc tttcaagaac ctcaagaccg gcaagtacgc ccgcatgagg 1080
ggcgcccaca ccaacgatgt caagcagctg accgaggccg tccagaagat cacgaccgag 1140
tccatcgtga tctgggggaa gacacccaag ttcaagctgc ctatccagaa ggagacctgg 1200
gagacgtggt ggaccgaata ttggcaggcc acctggattc ccgagtggga gttcgtgaat 1260
acacctcctc tggtgaagct gtggtaccag ctcgagaagg agcccatcgt gggcgcggag 1320
acattctacg tggacggcgc ggccaaccgc gaaacaaagc tcgggaaggc cgggtacgtc 1380
accaaccggg gccgccagaa ggtcgtcacc ctgaccgaca ccaccaacca gaagacggag 1440
ctgcaggcca tctatctcgc tctccaggac tccggcctgg aggtgaacat cgtgacggac 1500
agccagtacg cgctgggcat tattcaggcc cagccggacc agtccgagag cgaactggtg 1560
aaccagatta tcgagcagct gatcaagaaa gagaaggtct acctcgcctg ggtcccggcc 1620
cataagggca ttggcggcaa cgagcaggtc gacaagctgg tgagtgcggg gattagaaag 1680
gtgctgtaa 1689

<210> 59
<211> 562
<212> PRT
<213> HIV

<400> 59
Met Gly Pro Ile Ser Pro Ile Glu Thr Val Ser Val Lys Leu Lys Pro
1               5                   10                  15
Gly Met Asp Gly Pro Lys Val Lys Gln Trp Pro Leu Thr Glu Glu Lys
            20                  25                  30
Ile Lys Ala Leu Val Glu Ile Cys Thr Glu Met Glu Lys Glu Gly Lys
        35                  40                  45
Ile Ser Lys Ile Gly Pro Glu Asn Pro Tyr Asn Thr Pro Val Phe Ala
    50                  55                  60
Ile Lys Lys Lys Asp Ser Thr Lys Trp Arg Lys Leu Val Asp Phe Arg
65                  70                  75                  80
Glu Leu Asn Lys Arg Thr Gln Asp Phe Trp Glu Val Gln Leu Gly Ile
                85                  90                  95
Pro His Pro Ala Gly Leu Lys Lys Lys Lys Ser Val Thr Val Leu Asp
            100                 105                 110
Val Gly Asp Ala Tyr Phe Ser Val Pro Leu Asp Glu Asp Phe Arg Lys
        115                 120                 125
Tyr Thr Ala Phe Thr Ile Pro Ser Ile Asn Asn Glu Thr Pro Gly Ile
    130                 135                 140
Arg Tyr Gln Tyr Asn Val Leu Pro Gln Gly Trp Lys Gly Ser Pro Ala
145                 150                 155                 160
Ile Phe Gln Ser Ser Met Thr Lys Ile Leu Glu Pro Phe Arg Lys Gln
                165                 170                 175
Asn Pro Asp Ile Val Ile Tyr Gln Tyr Met Asp Asp Leu Tyr Val Gly
            180                 185                 190
Ser Asp Leu Glu Ile Gly Gln His Arg Thr Lys Ile Glu Glu Leu Arg
        195                 200                 205
Gln His Leu Leu Arg Trp Gly Leu Thr Thr Pro Asp Lys Lys His Gln
    210                 215                 220
Lys Glu Pro Pro Phe Leu Trp Met Gly Tyr Glu Leu His Pro Asp Lys
225                 230                 235                 240
Trp Thr Val Gln Pro Ile Val Leu Pro Glu Lys Asp Ser Trp Thr Val

                245                 250                 255
Asn Asp Ile Gln Lys Leu Val Gly Lys Leu Asn Trp Ala Ser Gln Ile
            260                 265                 270
Tyr Pro Gly Ile Lys Val Arg Gln Leu Cys Lys Leu Leu Arg Gly Thr
        275                 280                 285
Lys Ala Leu Thr Glu Val Ile Pro Leu Thr Glu Glu Ala Glu Leu Glu
    290                 295                 300
Leu Ala Glu Asn Arg Glu Ile Leu Lys Glu Pro Val His Gly Val Tyr
305                 310                 315                 320
Tyr Asp Pro Ser Lys Asp Leu Ile Ala Glu Ile Gln Lys Gln Gly Gln
                325                 330                 335
Gly Gln Trp Thr Tyr Gln Ile Tyr Gln Glu Pro Phe Lys Asn Leu Lys
            340                 345                 350
Thr Gly Lys Tyr Ala Arg Met Arg Gly Ala His Thr Asn Asp Val Lys
        355                 360                 365
Gln Leu Thr Glu Ala Val Gln Lys Ile Thr Thr Glu Ser Ile Val Ile
    370                 375                 380
Trp Gly Lys Thr Pro Lys Phe Lys Leu Pro Ile Gln Lys Glu Thr Trp
385                 390                 395                 400
Glu Thr Trp Trp Thr Glu Tyr Trp Gln Ala Thr Trp Ile Pro Glu Trp
                405                 410                 415
Glu Phe Val Asn Thr Pro Pro Leu Val Lys Leu Trp Tyr Gln Leu Glu
            420                 425                 430
Lys Glu Pro Ile Val Gly Ala Glu Thr Phe Tyr Val Asp Gly Ala Ala
        435                 440                 445
Asn Arg Glu Thr Lys Leu Gly Lys Ala Gly Tyr Val Thr Asn Arg Gly
    450                 455                 460
Arg Gln Lys Val Val Thr Leu Thr Asp Thr Thr Asn Gln Lys Thr Glu
465                 470                 475                 480
Leu Gln Ala Ile Tyr Leu Ala Leu Gln Asp Ser Gly Leu Glu Val Asn
                485                 490                 495
Ile Val Thr Asp Ser Gln Tyr Ala Leu Gly Ile Ile Gln Ala Gln Pro
            500                 505                 510
Asp Gln Ser Glu Ser Glu Leu Val Asn Gln Ile Ile Glu Gln Leu Ile
        515                 520                 525
Lys Lys Glu Lys Val Tyr Leu Ala Trp Val Pro Ala His Lys Gly Ile
    530                 535                 540
Gly Gly Asn Glu Gln Val Asp Lys Leu Val Ser Ala Gly Ile Arg Lys
545                 550                 555                 560
Val Leu

<210> 60
<211> 429
<212> DNA
<213> HIV

<400> 60
atggtgggtt ttccagtcac acctcaggta cctttaagac caatgactta caaggcagct 60
gtagatctta gccacttttt aaaagaaaag gggggactgg aagggctaat tcactcccaa 120
agaagacaag atatccttga tctgtggatc taccacacac aaggctactt ccctgattgg 180
cagaactaca caccagggcc aggggtcaga tatccactga cctttggatg gtgctacaag 240
ctagtaccag ttgagccaga taaggtagaa gaggccaata aaggagagaa caccagcttg 300
ttacaccctg tgagcctgca tgggatggat gacccggaga gagaagtgtt agagtggagg 360
tttgacagcc acctagcatt tcatcacgtg gcccgagagc tgcatccgga gtacttcaag 420
aactgctga                                                         429

<210> 61
<211> 142
<212> PRT
<213> HIV

<400> 61
Met Val Gly Phe Pro Val Thr Pro Gln Val Pro Leu Arg Pro Met Thr
1               5                   10                  15
Tyr Lys Ala Ala Val Asp Leu Ser His Phe Leu Lys Glu Lys Gly Gly
            20                  25                  30
Leu Glu Gly Leu Ile His Ser Gln Arg Arg Gln Asp Ile Leu Asp Leu
        35                  40                  45
Trp Ile Tyr His Thr Gln Gly Tyr Phe Pro Asp Trp Gln Asn Tyr Thr
    50                  55                  60
Pro Gly Pro Gly Val Arg Tyr Pro Leu Thr Phe Gly Trp Cys Tyr Lys
65                  70                  75                  80
Leu Val Pro Val Glu Pro Asp Lys Val Glu Glu Ala Asn Lys Gly Glu
                85                  90                  95

Asn Thr Ser Leu Leu His Pro Val Ser Leu His Gly Met Asp Asp Pro
            100                 105                 110
Glu Arg Glu Val Leu Glu Trp Arg Phe Asp Ser Val Leu Ala Phe His
        115                 120                 125
His Val Ala Arg Glu Leu His Pro Glu Tyr Phe Lys Asn Cys
    130                 135                 140

<210> 62
<211> 1698
<212> DNA
<213> hiv

<400> 62
atgggcccca ttagccctat tgagactgtg tcagtaaaat taaagccagg aatggatggc 60
ccaaaagtta aacaatggcc attgacagaa gaaaaaataa aagcattagt agaaatttgt 120
acagagatgg aaaaggaagg gaaaatttca aaaattgggc ctgaaaatcc atacaatact 180
ccagtatttg ccataaagaa aaaagacagt actaaatgga gaaaattagt agatttcaga 240
gaacttaata agagaactca agacttctgg gaagttcaat taggaatacc acatcccgca 300
gggttaaaaa agaaaaaatc agtaacagta ctggatgtgg gtgatgcata tttttcagtt 360
cccttagatg aagacttcag gaaatatact gcatttacca tacctagtat aaacaatgag 420
acaccaggga ttagatatca gtacaatgtg cttccacagg gatggaaagg atcaccagca 480
atattccaaa gtagcatgac aaaaatctta gagcctttta gaaaacaaaa tccagacata 540
gttatctatc aatacatgga tgatttgtat gtaggatctg acttagaaat agggcagcat 600
agaacaaaaa tagaggagct gagacaacat ctgttgaggt ggggacttac cacaccagac 660
aaaaaacatc agaaagaacc tccattcctt tggatgggtt atgaactcca tcctgataaa 720
tggacagtac agcctatagt gctgccagaa aaagacagct ggactgtcaa tgacatacag 780
aagttagtgg ggaaattgaa ttgggcaagt cagatttacc cagggattaa agtaaggcaa 840
ttatgtaaac tccttagagg aaccaaagca ctaacagaag taataccact aacagaagaa 900
gcagagctag aactggcaga aaacagagag attctaaaag aaccagtaca tggagtgtat 960
tatgacccat caaaagactt aatagcagaa atacagaagc aggggcaagg ccaatggaca 1020
tatcaaattt atcaagagcc atttaaaaat ctgaaaacag gaaaatatgc aagaatgagg 1080
ggtgcccaca ctaatgatgt aaaacaatta acagaggcag tgcaaaaaat aaccacagaa 1140
agcatagtaa tatggggaaa gactcctaaa tttaaactgc ccatacaaaa ggaaacatgg 1200
gaaacatggt ggacagagta ttggcaagcc acctggattc ctgagtggga gtttgttaat 1260
acccctccct tagtgaaatt atggtaccag ttagagaaag aacccatagt aggagcagaa 1320
accttctatg tagatggggc agctaacagg gagactaaat taggaaaagc aggatatgtt 1380
actaatagag gaagacaaaa agttgtcacc ctaactgaca caacaaatca gaagactgag 1440
ttacaagcaa tttatctagc tttgcaggat tcgggattag aagtaaacat agtaacagac 1500
tcacaatatg cattaggaat cattcaagca caaccagatc aaagtgaatc agagttagtc 1560
aatcaaataa tagagcagtt aataaaaaag gaaaaggtct atctggcatg ggtaccagca 1620
cacaaaggaa ttggaggaaa tgaacaagta gataaattag tcagtgctgg aatcaggaaa 1680
gtactatttt tagattaa                                                1698

<210> 63
<211> 565
<212> PRT
<213> HIV

<400> 63
Met Gly Pro Ile Ser Pro Ile Glu Thr Val Ser Val Lys Leu Lys Pro
1               5                   10                  15
Gly Met Asp Gly Pro Lys Val Lys Gln Trp Pro Leu Thr Glu Glu Lys
            20                  25                  30
Ile Lys Ala Leu Val Glu Ile Cys Thr Glu Met Glu Lys Glu Gly Lys
        35                  40                  45
Ile Ser Lys Ile Gly Pro Glu Asn Pro Tyr Asn Thr Pro Val Phe Ala
    50                  55                  60
Ile Lys Lys Lys Asp Ser Thr Lys Trp Arg Lys Leu Val Asp Phe Arg
65                  70                  75                  80
Glu Leu Asn Lys Arg Thr Gln Asp Phe Trp Glu Val Gln Leu Gly Ile
                85                  90                  95
Pro His Pro Ala Gly Leu Lys Lys Lys Lys Ser Val Thr Val Leu Asp
            100                 105                 110
Val Gly Asp Ala Tyr Phe Ser Val Pro Leu Asp Glu Asp Phe Arg Lys
        115                 120                 125
Tyr Thr Ala Phe Thr Ile Pro Ser Ile Asn Asn Glu Thr Pro Gly Ile
    130                 135                 140
Arg Tyr Gln Tyr Asn Val Leu Pro Gln Gly Trp Lys Gly Ser Pro Ala
145                 150                 155                 160
Ile Phe Gln Ser Ser Met Thr Lys Ile Leu Glu Pro Phe Arg Lys Gln
                165                 170                 175
Asn Pro Asp Ile Val Ile Tyr Gln Tyr Met Asp Asp Leu Tyr Val Gly
            180                 185                 190
Ser Asp Leu Glu Ile Gly Gln His Arg Thr Lys Ile Glu Glu Leu Arg
        195                 200                 205

Gln His Leu Leu Arg Trp Gly Leu Thr Thr Pro Asp Lys Lys His Gln
    210                 215                 220
Lys Glu Pro Pro Phe Leu Trp Met Gly Tyr Glu Leu His Pro Asp Lys
225                 230                 235                 240
Trp Thr Val Gln Pro Ile Val Leu Pro Glu Lys Asp Ser Trp Thr Val
                245                 250                 255
Asn Asp Ile Gln Lys Leu Val Gly Lys Leu Asn Trp Ala Ser Gln Ile
            260                 265                 270
Tyr Pro Gly Ile Lys Val Arg Gln Leu Cys Lys Leu Leu Arg Gly Thr
        275                 280                 285
Lys Ala Leu Thr Glu Val Ile Pro Leu Thr Glu Glu Ala Glu Leu Glu
    290                 295                 300
Leu Ala Glu Asn Arg Glu Ile Leu Lys Glu Pro Val His Gly Val Tyr
305                 310                 315                 320
Tyr Asp Pro Ser Lys Asp Leu Ile Ala Glu Ile Gln Lys Gln Gly Gln
                325                 330                 335
Gly Gln Trp Thr Tyr Gln Ile Tyr Gln Glu Pro Phe Lys Asn Leu Lys
            340                 345                 350
Thr Gly Lys Tyr Ala Arg Met Arg Gly Ala His Thr Asn Asp Val Lys
        355                 360                 365
Gln Leu Thr Glu Ala Val Gln Lys Ile Thr Thr Glu Ser Ile Val Ile
    370                 375                 380
Trp Gly Lys Thr Pro Lys Phe Lys Leu Pro Ile Gln Lys Glu Thr Trp
385                 390                 395                 400
Glu Thr Trp Trp Thr Glu Tyr Trp Gln Ala Thr Trp Ile Pro Glu Trp
                405                 410                 415
Glu Phe Val Asn Thr Pro Pro Leu Val Lys Leu Trp Tyr Gln Leu Glu
            420                 425                 430
Lys Glu Pro Ile Val Gly Ala Glu Thr Phe Tyr Val Asp Gly Ala Ala
        435                 440                 445
Asn Arg Glu Thr Lys Leu Gly Lys Ala Gly Tyr Val Thr Asn Arg Gly
    450                 455                 460
Arg Gln Lys Val Val Thr Leu Thr Asp Thr Thr Asn Gln Lys Thr Glu
465                 470                 475                 480
Leu Gln Ala Ile Tyr Leu Ala Leu Gln Asp Ser Gly Leu Glu Val Asn
                485                 490                 495
Ile Val Thr Asp Ser Gln Tyr Ala Leu Gly Ile Ile Gln Ala Gln Pro
            500                 505                 510
Asp Gln Ser Glu Ser Glu Leu Val Asn Gln Ile Ile Glu Gln Leu Ile
        515                 520                 525
Lys Lys Glu Lys Val Tyr Leu Ala Trp Val Pro Ala His Lys Gly Ile
    530                 535                 540
Gly Gly Asn Glu Gln Val Asp Lys Leu Val Ser Ala Gly Ile Arg Lys
545                 550                 555                 560
Val Leu Phe Leu Asp
                565

<210> 64
<211> 3213
<212> DNA
<213> HIV

<400> 64
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300
ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat tctcggtctc aataaaattg ttagaatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tgggccccat tagccctatt gagactgtgt cagtaaaatt aaagccagga 1140
atggatggcc caaaagttaa acaatggcca ttgacagaag aaaaaataaa agcattagta 1200
gaaatttgta cagagatgga aaaggaaggg aaaatttcaa aaattgggcc tgaaaatcca 1260
tacaatactc cagtatttgc cataaagaaa aaagacagta ctaaatggag aaaattagta 1320

gatttcagag aacttaataa gagaactcaa gacttctggg aagttcaatt aggaatacca 1380
catcccgcag ggttaaaaaa gaaaaaatca gtaacagtac tggatgtggg tgatgcatat 1440
ttttcagttc ccttagatga agacttcagg aaatatactg catttaccat acctagtata 1500
aacaatgaga caccagggat tagatatcag tacaatgtgc ttccacaggg atggaaagga 1560
tcaccagcaa tattccaaag tagcatgaca aaaatcttag agccttttag aaaacaaaat 1620
ccagacatag ttatctatca atacatggat gatttgtatg taggatctga cttagaaata 1680
gggcagcata gaacaaaaat agaggagctg agacaacatc tgttgaggtg gggacttacc 1740
acaccagaca aaaaacatca gaaagaacct ccattccttt ggatgggtta tgaactccat 1800
cctgataaat ggacagtaca gcctatagtg ctgccagaaa aagacagctg gactgtcaat 1860
gacatacaga agttagtggg gaaattgaat tgggcaagtc agatttaccc agggattaaa 1920
gtaaggcaat tatgtaaact ccttagagga accaaagcac taacagaagt aataccacta 1980
acagaagaag cagagctaga actggcagaa aacagagaga ttctaaaaga accagtacat 2040
ggagtgtatt atgacccatc aaaagactta atagcagaaa tacagaagca ggggcaaggc 2100
caatggacat atcaaattta tcaagagcca tttaaaaatc tgaaaacagg aaaatatgca 2160
agaatgaggg gtgcccacac taatgatgta aaacaattaa cagaggcagt gcaaaaaata 2220
accacagaaa gcatagtaat atggggaaag actcctaaat ttaaactgcc catacaaaag 2280
gaaacatggg aaacatggtg gacagagtat tggcaagcca cctggattcc tgagtgggag 2340
tttgttaata cccctccctt agtgaaatta tggtaccagt tagagaaaga acccatagta 2400
ggagcagaaa ccttctatgt agatggggca gctaacaggg agactaaatt aggaaaagca 2460
ggatatgtta ctaatagagg aagacaaaaa gttgtcaccc taactgacac aacaaatcag 2520
aagactgagt tacaagcaat ttatctagct ttgcaggatt cgggattaga agtaaacata 2580
gtaacagact cacaatatgc attaggaatc attcaagcac aaccagatca aagtgaatca 2640
gagttagtca atcaaataat agagcagtta ataaaaaagg aaaaggtcta tctggcatgg 2700
gtaccagcac acaaaggaat tggaggaaat gaacaagtag ataaattagt cagtgctgga 2760
atcaggaaag tactattttt agatatggtg ggttttccag tcacacctca ggtaccttta 2820
agaccaatga cttacaaggc agctgtagat cttagccact ttttaaaaga aaagggggga 2880
ctggaagggc taattcactc ccaaagaaga caagatatcc ttgatctgtg gatctaccac 2940
acacaaggct acttccctga ttggcagaac tacacaccag ggccaggggt cagatatcca 3000
ctgacctttg gatggtgcta caagctagta ccagttgagc cagataaggt agaagaggcc 3060
aataaaggag agaacaccag cttgttacac cctgtgagcc tgcatggaat ggatgacccg 3120
gagagagaag tgttagagtg gaggtttgac agccacctag catttcatca cgtggcccga 3180
gagctgcatc cggagtactt caagaactgc tga 3213

<210> 65
<211> 1070
<212> PRT
<213> HIV

<400> 65
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu

290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Met Gly Pro Ile Ser
        355                 360                 365
Pro Ile Glu Thr Val Ser Val Lys Leu Lys Pro Gly Met Asp Gly Pro
    370                 375                 380
Lys Val Lys Gln Trp Pro Leu Thr Glu Glu Lys Ile Lys Ala Leu Val
385                 390                 395                 400
Glu Ile Cys Thr Glu Met Glu Lys Glu Gly Lys Ile Ser Lys Ile Gly
                405                 410                 415
Pro Glu Asn Pro Tyr Asn Thr Pro Val Phe Ala Ile Lys Lys Lys Asp
            420                 425                 430
Ser Thr Lys Trp Arg Lys Leu Val Asp Phe Arg Glu Leu Asn Lys Arg
        435                 440                 445
Thr Gln Asp Phe Trp Glu Val Gln Leu Gly Ile Pro His Pro Ala Gly
    450                 455                 460
Leu Lys Lys Lys Lys Ser Val Thr Val Leu Asp Val Gly Asp Ala Tyr
465                 470                 475                 480
Phe Ser Val Pro Leu Asp Glu Asp Phe Arg Lys Tyr Thr Ala Phe Thr
                485                 490                 495
Ile Pro Ser Ile Asn Asn Glu Thr Pro Gly Ile Arg Tyr Gln Tyr Asn
            500                 505                 510
Val Leu Pro Gln Gly Trp Lys Gly Ser Pro Ala Ile Phe Gln Ser Ser
        515                 520                 525
Met Thr Lys Ile Leu Glu Pro Phe Arg Lys Gln Asn Pro Asp Ile Val
    530                 535                 540
Ile Tyr Gln Tyr Met Asp Asp Leu Tyr Val Gly Ser Asp Leu Glu Ile
545                 550                 555                 560
Gly Gln His Arg Thr Lys Ile Glu Glu Leu Arg Gln His Leu Leu Arg
                565                 570                 575
Trp Gly Leu Thr Thr Pro Asp Lys Lys His Gln Lys Glu Pro Pro Phe
            580                 585                 590
Leu Trp Met Gly Tyr Glu Leu His Pro Asp Lys Trp Thr Val Gln Pro
        595                 600                 605
Ile Val Leu Pro Glu Lys Asp Ser Trp Thr Val Asn Asp Ile Gln Lys
    610                 615                 620
Leu Val Gly Lys Leu Asn Trp Ala Ser Gln Ile Tyr Pro Gly Ile Lys
625                 630                 635                 640
Val Arg Gln Leu Cys Lys Leu Leu Arg Gly Thr Lys Ala Leu Thr Glu
                645                 650                 655
Val Ile Pro Leu Thr Glu Glu Ala Glu Leu Glu Leu Ala Glu Asn Arg
            660                 665                 670
Glu Ile Leu Lys Glu Pro Val His Gly Val Tyr Tyr Asp Pro Ser Lys
        675                 680                 685
Asp Leu Ile Ala Glu Ile Gln Lys Gln Gly Gln Gly Gln Trp Thr Tyr
    690                 695                 700
Gln Ile Tyr Gln Glu Pro Phe Lys Asn Leu Lys Thr Gly Lys Tyr Ala
705                 710                 715                 720
Arg Met Arg Gly Ala His Thr Asn Asp Val Lys Gln Leu Thr Glu Ala
                725                 730                 735
Val Gln Lys Ile Thr Thr Glu Ser Ile Val Ile Trp Gly Lys Thr Pro
            740                 745                 750
Lys Phe Lys Leu Pro Ile Gln Lys Glu Thr Trp Glu Thr Trp Trp Thr
        755                 760                 765
Glu Tyr Trp Gln Ala Thr Trp Ile Pro Glu Trp Glu Phe Val Asn Thr
    770                 775                 780
Pro Pro Leu Val Lys Leu Trp Tyr Gln Leu Glu Lys Glu Pro Ile Val
785                 790                 795                 800
Gly Ala Glu Thr Phe Tyr Val Asp Gly Ala Ala Asn Arg Glu Thr Lys
                805                 810                 815
Leu Gly Lys Ala Gly Tyr Val Thr Asn Arg Gly Arg Gln Lys Val Val
            820                 825                 830
Thr Leu Thr Asp Thr Thr Asn Gln Lys Thr Glu Leu Gln Ala Ile Tyr
        835                 840                 845
Leu Ala Leu Gln Asp Ser Gly Leu Glu Val Asn Ile Val Thr Asp Ser
    850                 855                 860
Gln Tyr Ala Leu Gly Ile Ile Gln Ala Gln Pro Asp Gln Ser Glu Ser
865                 870                 875                 880
Glu Leu Val Asn Gln Ile Ile Glu Gln Leu Ile Lys Lys Glu Lys Val
                885                 890                 895
Tyr Leu Ala Trp Val Pro Ala His Lys Gly Ile Gly Gly Asn Glu Gln

900                 905                 910
Val Asp Lys Leu Val Ser Ala Gly Ile Arg Lys Val Leu Phe Leu Asp
        915                 920                 925
Met Val Gly Phe Pro Val Thr Pro Gln Val Pro Leu Arg Pro Met Thr
    930                 935                 940
Tyr Lys Ala Ala Val Asp Leu Ser His Phe Leu Lys Glu Lys Gly Gly
945                 950                 955                 960
Leu Glu Gly Leu Ile His Ser Gln Arg Arg Gln Asp Ile Leu Asp Leu
                965                 970                 975
Trp Ile Tyr His Thr Gln Gly Tyr Phe Pro Asp Trp Gln Asn Tyr Thr
            980                 985                 990
Pro Gly Pro Gly Val Arg Tyr Pro Leu Thr Phe Gly Trp Cys Tyr Lys
        995                 1000                1005
Leu Val Pro Val Glu Pro Asp Lys Val Glu Glu Ala Asn Lys Gly Glu
    1010                1015                1020
Asn Thr Ser Leu Leu His Pro Val Ser Leu His Gly Met Asp Asp Pro
1025                1030                1035                1040
Glu Arg Glu Val Leu Glu Trp Arg Phe Asp Ser His Leu Ala Phe His
                1045                1050                1055
His Val Ala Arg Glu Leu His Pro Glu Tyr Phe Lys Asn Cys
            1060                1065                1070

<210> 66
<211> 3213
<212> DNA
<213> HIV

<400> 66
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300
ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat cctgggcctg aacaagatcg tgcgcatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tgggccccat tagccctatt gagactgtgt cagtaaaatt aaagccagga 1140
atggatggcc caaaagttaa acaatggcca ttgacagaag aaaaaataaa agcattagta 1200
gaaatttgta cagagatgga aaaggaaggg aaaatttcaa aaattgggcc tgaaaatcca 1260
tacaatactc cagtatttgc cataaagaaa aaagacagta ctaaatggag aaaattagta 1320
gatttcagag aacttaataa gagaactcaa gacttctggg aagttcaatt aggaatacca 1380
catcccgcag ggttaaaaaa gaaaaaatca gtaacagtac tggatgtggg tgatgcatat 1440
ttttcagttc ccttagatga agacttcagg aaatatactg catttaccat acctagtata 1500
aacaatgaga caccagggat tagatatcag tacaatgtgc ttccacaggg atggaaagga 1560
tcaccagcaa tattccaaag tagcatgaca aaaatcttag agccttttag aaaacaaaat 1620
ccagacatag ttatctatca atacatggat gatttgtatg taggatctga cttagaaata 1680
gggcagcata gaacaaaaat agaggagctg agacaacatc tgttgaggtg gggacttacc 1740
acaccagaca aaaaacatca gaaagaacct ccattccttt ggatgggtta tgaactccat 1800
cctgataaat ggacagtaca gcctatagtg ctgccagaaa aagacagctg gactgtcaat 1860
gacatacaga agttagtggg gaaattgaat tgggcaagtc agatttaccc agggattaaa 1920
gtaaggcaat tatgtaaact ccttagagga accaaagcac taacagaagt aataccacta 1980
acagaagaag cagagctaga actggcagaa aacagagaga ttctaaaaga accagtacat 2040
ggagtgtatt atgacccatc aaaagactta atagcagaaa tacagaagca ggggcaaggc 2100
caatggacat atcaaattta tcaagagcca tttaaaaatc tgaaaacagg aaaatatgca 2160
agaatgaggg gtgcccacac taatgatgta aaacaattaa cagaggcagt gcaaaaaata 2220
accacagaaa gcatagtaat atggggaaag actcctaaat ttaaactgcc catacaaaag 2280
gaaacatggg aaacatggtg gacagagtat tggcaagcca cctggattcc tgagtgggag 2340
tttgttaata cccctccctt agtgaaatta tggtaccagt tagagaaaga acccatagta 2400
ggagcagaaa ccttctatgt agatggggca gctaacaggg agactaaatt aggaaaagca 2460
ggatatgtta ctaatagagg aagacaaaaa gttgtcaccc taactgacac aacaaatcag 2520
aagactgagt tacaagcaat ttatctagct ttgcaggatt cgggattaga agtaaacata 2580
gtaacagact cacaatatgc attaggaatc attcaagcac aaccagatca aagtgaatca 2640
gagttagtca atcaaataat agagcagtta ataaaaaagg aaaaggtcta tctggcatgg 2700
gtaccagcac acaaaggaat tggaggaaat gaacaagtag ataaattagt cagtgctgga 2760
atcaggaaag tactattttt agatatggtg ggttttccag tcacacctca ggtaccttta 2820

agaccaatga cttacaaggc agctgtagat cttagccact ttttaaaaga aaagggggga 2880
ctggaagggc taattcactc ccaaagaaga caagatatcc ttgatctgtg gatctaccac 2940
acacaaggct acttccctga ttggcagaac tacacaccag ggccaggggt cagatatcca 3000
ctgacctttg gatggtgcta caagctagta ccagttgagc cagataaggt agaagaggcc 3060
aataaaggag agaacaccag cttgttacac cctgtgagcc tgcatgggat ggatgacccg 3120
gagagagaag tgttagagtg gaggtttgac agccacctag catttcatca cgtggcccga 3180
gagctgcatc cggagtactt caagaactgc tga 3213

<210> 67
<211> 1070
<212> PRT
<213> HIV

<400> 67
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Met Gly Pro Ile Ser
        355                 360                 365
Pro Ile Glu Thr Val Ser Val Lys Leu Lys Pro Gly Met Asp Gly Pro
    370                 375                 380
Lys Val Lys Gln Trp Pro Leu Thr Glu Glu Lys Ile Lys Ala Leu Val
385                 390                 395                 400
Glu Ile Cys Thr Glu Met Glu Lys Glu Gly Lys Ile Ser Lys Ile Gly
                405                 410                 415
Pro Glu Asn Pro Tyr Asn Thr Pro Val Phe Ala Ile Lys Lys Lys Asp
            420                 425                 430
Ser Thr Lys Trp Arg Lys Leu Val Asp Phe Arg Glu Leu Asn Lys Arg
        435                 440                 445
Thr Gln Asp Phe Trp Glu Val Gln Leu Gly Ile Pro His Pro Ala Gly
    450                 455                 460
Leu Lys Lys Lys Lys Ser Val Thr Val Leu Asp Val Gly Asp Ala Tyr
465                 470                 475                 480
Phe Ser Val Pro Leu Asp Glu Asp Phe Arg Lys Tyr Thr Ala Phe Thr
                485                 490                 495

Ile Pro Ser Ile Asn Asn Glu Thr Pro Gly Ile Arg Tyr Gln Tyr Asn
                500                 505                 510
Val Leu Pro Gln Gly Trp Lys Gly Ser Pro Ala Ile Phe Gln Ser Ser
            515                 520                 525
Met Thr Lys Ile Leu Glu Pro Phe Arg Lys Gln Asn Pro Asp Ile Val
    530                 535                 540
Ile Tyr Gln Tyr Met Asp Asp Leu Tyr Val Gly Ser Asp Leu Glu Ile
545                 550                 555                 560
Gly Gln His Arg Thr Lys Ile Glu Glu Leu Arg Gln His Leu Leu Arg
                565                 570                 575
Trp Gly Leu Thr Thr Pro Asp Lys Lys His Gln Lys Glu Pro Pro Phe
            580                 585                 590
Leu Trp Met Gly Tyr Glu Leu His Pro Asp Lys Trp Thr Val Gln Pro
        595                 600                 605
Ile Val Leu Pro Glu Lys Asp Ser Trp Thr Val Asn Asp Ile Gln Lys
    610                 615                 620
Leu Val Gly Lys Leu Asn Trp Ala Ser Gln Ile Tyr Pro Gly Ile Lys
625                 630                 635                 640
Val Arg Gln Leu Cys Lys Leu Leu Arg Gly Thr Lys Ala Leu Thr Glu
                645                 650                 655
Val Ile Pro Leu Thr Glu Glu Ala Glu Leu Glu Leu Ala Glu Asn Arg
            660                 665                 670
Glu Ile Leu Lys Glu Pro Val His Gly Val Tyr Tyr Asp Pro Ser Lys
        675                 680                 685
Asp Leu Ile Ala Glu Ile Gln Lys Gln Gly Gln Gly Gln Trp Thr Tyr
    690                 695                 700
Gln Ile Tyr Gln Glu Pro Phe Lys Asn Leu Lys Thr Gly Lys Tyr Ala
705                 710                 715                 720
Arg Met Arg Gly Ala His Thr Asn Asp Val Lys Gln Leu Thr Glu Ala
                725                 730                 735
Val Gln Lys Ile Thr Thr Glu Ser Ile Val Ile Trp Gly Lys Thr Pro
            740                 745                 750
Lys Phe Lys Leu Pro Ile Gln Lys Glu Thr Trp Glu Thr Trp Trp Thr
        755                 760                 765
Glu Tyr Trp Gln Ala Thr Trp Ile Pro Glu Trp Glu Phe Val Asn Thr
    770                 775                 780
Pro Pro Leu Val Lys Leu Trp Tyr Gln Leu Glu Lys Glu Pro Ile Val
785                 790                 795                 800
Gly Ala Glu Thr Phe Tyr Val Asp Gly Ala Ala Asn Arg Glu Thr Lys
                805                 810                 815
Leu Gly Lys Ala Gly Tyr Val Thr Asn Arg Gly Arg Gln Lys Val Val
            820                 825                 830
Thr Leu Thr Asp Thr Thr Asn Gln Lys Thr Glu Leu Gln Ala Ile Tyr
        835                 840                 845
Leu Ala Leu Gln Asp Ser Gly Leu Glu Val Asn Ile Val Thr Asp Ser
    850                 855                 860
Gln Tyr Ala Leu Gly Ile Ile Gln Ala Gln Pro Asp Gln Ser Glu Ser
865                 870                 875                 880
Glu Leu Val Asn Gln Ile Ile Glu Gln Leu Ile Lys Lys Glu Lys Val
                885                 890                 895
Tyr Leu Ala Trp Val Pro Ala His Lys Gly Ile Gly Gly Asn Glu Gln
            900                 905                 910
Val Asp Lys Leu Val Ser Ala Gly Ile Arg Lys Val Leu Phe Leu Asp
        915                 920                 925
Met Val Gly Phe Pro Val Thr Pro Gln Val Pro Leu Arg Pro Met Thr
    930                 935                 940
Tyr Lys Ala Ala Val Asp Leu Ser His Phe Leu Lys Glu Lys Gly Gly
945                 950                 955                 960
Leu Glu Gly Leu Ile His Ser Gln Arg Arg Gln Asp Ile Leu Asp Leu
                965                 970                 975
Trp Ile Tyr His Thr Gln Gly Tyr Phe Pro Asp Trp Gln Asn Tyr Thr
            980                 985                 990
Pro Gly Pro Gly Val Arg Tyr Pro Leu Thr Phe Gly Trp Cys Tyr Lys
        995                 1000                1005
Leu Val Pro Val Glu Pro Asp Lys Val Glu Glu Ala Asn Lys Gly Glu
    1010                1015                1020
Asn Thr Ser Leu Leu His Pro Val Ser Leu His Gly Met Asp Asp Pro
1025                1030                1035                1040
Glu Arg Glu Val Leu Glu Trp Arg Phe Asp Ser His Leu Ala Phe His
                1045                1050                1055
His Val Ala Arg Glu Leu His Pro Glu Tyr Phe Lys Asn Cys
            1060                1065                1070

<210> 68
<211> 3204

<212> DNA
<213> Hiv

<400> 68
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300
ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat cctgggcctg aacaagatcg tgcgcatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tggccccatt cagtcccatc gagaccgtgc cggtgaagct gaaacccggg 1140
atggacggcc ccaaggtcaa gcagtggcca ctcaccgagg agaagatcaa ggccctggtg 1200
gagatctgca ccgagatgga gaaagagggc aagatcagca agatcgggcc tgagaaccca 1260
tacaacaccc ccgtgtttgc catcaagaag aaggacagca ccaagtggcg caagctggtg 1320
gatttccggg agctgaataa gcggacccag gatttctggg aggtccagct gggcatcccc 1380
catccggccg gcctgaagaa gaagaagagc gtgaccgtgc tggacgtggg cgacgcttac 1440
ttcagcgtcc ctctggacga ggactttaga aagtacaccg cctttaccat cccatctatc 1500
aacaacgaga cccctggcat cagatatcag tacaacgtcc tcccccaggg ctggaagggc 1560
tctcccgcca ttttccagag ctccatgacc aagatcctgg agccgtttcg gaagcagaac 1620
cccgatatcg tcatctacca gtacatggac gacctgtacg tgggctctga cctggaaatc 1680
gggcagcatc gcacgaagat tgaggagctg aggcagcatc tgctgagatg gggcctgacc 1740
actccggaca agaagcatca gaaggagccg ccattcctgt ggatgggcta cgagctccat 1800
cccgacaagt ggaccgtgca gcctatcgtc ctccccgaga aggacagctg gaccgtgaac 1860
gacatccaga agctggtggg caagctcaac tgggctagcc agatctatcc cgggatcaag 1920
gtgcgccagc tctgcaagct gctgcgcggc accaaggccc tgaccgaggt gattcccctc 1980
acggaggaag ccgagctcga gctggctgag aaccgggaga tcctgaagga gcccgtgcac 2040
ggcgtgtact atgacccctc caaggacctg atcgccgaaa tccagaagca gggccagggg 2100
cagtggacat accagattta ccaggagcct ttcaagaacc tcaagaccgg caagtacgcc 2160
cgcatgaggg gcgcccacac caacgatgtc aagcagctga ccgaggccgt ccagaagatc 2220
acgaccgagt ccatcgtgat ctgggggaag acacccaagt tcaagctgcc tatccagaag 2280
gagacctggg agacgtggtg gaccgaatat tggcaggcca cctggattcc cgagtgggag 2340
ttcgtgaata cacctcctct ggtgaagctg tggtaccagc tcgagaagga gcccatcgtg 2400
ggcgcggaga cattctacgt ggacggcgcg gccaaccgcg aaacaaagct cgggaaggcc 2460
gggtacgtca ccaaccgggg ccgccagaag gtcgtcaccc tgaccgacac caccaaccag 2520
aagacggagc tgcaggccat ctatctcgct ctccaggact ccggcctgga ggtgaacatc 2580
gtgacggaca gccagtacgc gctgggcatt attcaggccc agccggacca gtccgagagc 2640
gaactggtga accagattat cgagcagctg atcaagaaag agaaggtcta cctcgcctgg 2700
gtcccggccc ataagggcat tggcggcaac gagcaggtcg acaagctggt gagtgcgggg 2760
attagaaagg tgctgatggt gggttttcca gtcacacctc aggtaccttt aagaccaatg 2820
acttacaagg cagctgtaga tcttagccac tttttaaaag aaaagggggg actggaaggg 2880
ctaattcact cccaaagaag acaagatatc cttgatctgt ggatctacca cacacaaggc 2940
tacttccctg attggcagaa ctacacacca gggccagggg tcagatatcc actgaccttt 3000
ggatggtgct acaagctagt accagttgag ccagataagg tagaagaggc caataaagga 3060
gagaacacca gcttgttaca ccctgtgagc ctgcatggga tggatgaccc ggagagagaa 3120
gtgttagagt ggaggtttga cagccgccta gcatttcatc acgtggcccg agagctgcat 3180
ccggagtact tcaagaactg ctga 3204

<210> 69
<211> 1067
<212> PRT
<213> HIV

<400> 69
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Asn Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Val His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                   245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305                 310                 315                 320
   Leu Leu Val Gln Asn Ala Asn Pro Asp Cys Lys Thr Ile Leu Lys Ala
                   325                 330                 335
   Leu Gly Pro Ala Ala Thr Leu Glu Glu Met Met Thr Ala Cys Gln Gly
               340                 345                 350
   Val Gly Gly Pro Gly His Lys Ala Arg Val Leu Met Gly Pro Ile Ser
           355                 360                 365
   Pro Ile Glu Thr Val Ser Val Lys Leu Lys Pro Gly Met Asp Gly Pro
       370                 375                 380
   Lys Val Lys Gln Trp Pro Leu Thr Glu Glu Lys Ile Lys Ala Leu Val
   385                 390                 395                 400
   Glu Ile Cys Thr Glu Met Glu Lys Glu Gly Lys Ile Ser Lys Ile Gly
                   405                 410                 415
   Pro Glu Asn Pro Tyr Asn Thr Pro Val Phe Ala Ile Lys Lys Lys Asp
               420                 425                 430
   Ser Thr Lys Trp Arg Lys Leu Val Asp Phe Arg Glu Leu Asn Lys Arg
           435                 440                 445
   Thr Gln Asp Phe Trp Glu Val Gln Leu Gly Ile Pro His Pro Ala Gly
       450                 455                 460
   Leu Lys Lys Lys Lys Ser Val Thr Val Leu Asp Val Gly Asp Ala Tyr
   465                 470                 475                 480
   Phe Ser Val Pro Leu Asp Glu Asp Phe Arg Lys Tyr Thr Ala Phe Thr
                   485                 490                 495
   Ile Pro Ser Ile Asn Asn Glu Thr Pro Gly Ile Arg Tyr Gln Tyr Asn
               500                 505                 510
   Val Leu Pro Gln Gly Trp Lys Gly Ser Pro Ala Ile Phe Gln Ser Ser
           515                 520                 525
   Met Thr Lys Ile Leu Glu Pro Phe Arg Lys Gln Asn Pro Asp Ile Val
       530                 535                 540
   Ile Tyr Gln Tyr Met Asp Asp Leu Tyr Val Gly Ser Asp Leu Glu Ile
   545                 550                 555                 560
   Gly Gln His Arg Thr Lys Ile Glu Glu Leu Arg Gln His Leu Leu Arg
                   565                 570                 575
   Trp Gly Leu Thr Thr Pro Asp Lys Lys His Gln Lys Glu Pro Pro Phe
               580                 585                 590
   Leu Trp Met Gly Tyr Glu Leu His Pro Asp Lys Trp Thr Val Gln Pro
           595                 600                 605
   Ile Val Leu Pro Glu Lys Asp Ser Trp Thr Val Asn Asp Ile Gln Lys
       610                 615                 620
   Leu Val Gly Lys Leu Asn Trp Ala Ser Gln Ile Tyr Pro Gly Ile Lys
   625                 630                 635                 640
   Val Arg Gln Leu Cys Lys Leu Leu Arg Gly Thr Lys Ala Leu Thr Glu
                   645                 650                 655
   Val Ile Pro Leu Thr Glu Glu Ala Glu Leu Glu Leu Ala Glu Asn Arg
               660                 665                 670
   Glu Ile Leu Lys Glu Pro Val His Gly Val Tyr Tyr Asp Pro Ser Lys
           675                 680                 685
   Asp Leu Ile Ala Glu Ile Gln Lys Gln Gly Gln Gly Gln Trp Thr Tyr
   690                 695                 700
   Gln Ile Tyr Gln Glu Pro Phe Lys Asn Leu Lys Thr Gly Lys Tyr Ala
   705                 710                 715                 720
   Arg Met Arg Gly Ala His Thr Asn Asp Val Lys Gln Leu Thr Glu Ala
                   725                 730                 735
   Val Gln Lys Ile Thr Thr Glu Ser Ile Val Ile Trp Gly Lys Thr Pro
               740                 745                 750
   Lys Phe Lys Leu Pro Ile Gln Lys Glu Thr Trp Glu Thr Trp Trp Thr
           755                 760                 765
   Glu Tyr Trp Gln Ala Thr Trp Ile Pro Glu Trp Glu Phe Val Asn Thr
       770                 775                 780
   Pro Pro Leu Val Lys Leu Trp Tyr Gln Leu Glu Lys Glu Pro Ile Val
   785                 790                 795                 800
   Gly Ala Glu Thr Phe Tyr Val Asp Gly Ala Ala Asn Arg Glu Thr Lys
                   805                 810                 815
   Leu Gly Lys Ala Gly Tyr Val Thr Asn Arg Gly Arg Gln Lys Val Val
               820                 825                 830
   Thr Leu Thr Asp Thr Thr Asn Gln Lys Thr Glu Leu Gln Ala Ile Tyr
           835                 840                 845
   Leu Ala Leu Gln Asp Ser Gly Leu Glu Val Asn Ile Val Thr Asp Ser
       850                 855                 860
   Gln Tyr Ala Leu Gly Ile Ile Gln Ala Gln Pro Asp Gln Ser Glu Ser
   865                 870                 875                 880
   Glu Leu Val Asn Gln Ile Ile Glu Gln Leu Ile Lys Lys Glu Lys Val
                   885                 890                 895
   Tyr Leu Ala Trp Val Pro Ala His Lys Gly Ile Gly Gly Asn Glu Gln
               900                 905                 910
   Val Asp Lys Leu Val Ser Ala Gly Ile Arg Lys Val Leu Met Val Gly
           915                 920                 925
   Phe Pro Val Thr Pro Gln Val Pro Leu Arg Pro Met Thr Tyr Lys Ala
       930                 935                 940
   Ala Val Asp Leu Ser His Phe Leu Lys Glu Lys Gly Gly Leu Glu Gly
   945                 950                 955                 960
   Leu Ile His Ser Gln Arg Arg Gln Asp Ile Leu Asp Leu Trp Ile Tyr
                   965                 970                 975
   His Thr Gln Gly Tyr Phe Pro Asp Trp Gln Asn Tyr Thr Pro Gly Pro
               980                 985                 990
   Gly Val Arg Tyr Pro Leu Thr Phe Gly Trp Cys Tyr Lys Leu Val Pro
           995                 1000                1005
   Val Glu Pro Asp Lys Val Glu Glu Ala Asn Lys Gly Glu Asn Thr Ser
       1010                1015                1020
   Leu Leu His Pro Val Ser Leu His Gly Met Asp Asp Pro Glu Arg Glu
   1025                1030                1035                1040
   Val Leu Glu Trp Arg Phe Asp Ser Arg Leu Ala Phe His His Val Ala
                   1045                1050                1055
   Arg Glu Leu His Pro Glu Tyr Phe Lys Asn Cys
               1060                1065

<210> 70
<211> 1518
<212> DNA
<213> HIV

<400> 70
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300
ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat tctcggtctc aataaaattg ttagaatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tggtgggttt tccagtcaca cctcaggtac ctttaagacc aatgacttac 1140
aaggcagctg tagatcttag ccacttttta aaagaaaagg ggggactgga agggctaatt 1200
cactcccaaa gaagacaaga tatccttgat ctgtggatct accacacaca aggctacttc 1260

cctgattggc agaactacac accagggcca ggggtcagat atccactgac ctttggatgg 1320
tgctacaagc tagtaccagt tgagccagat aaggtagaag aggccaataa aggagagaac 1380
accagcttgt tacaccctgt gagcctgcat gggatggatg acccggagag agaagtgtta 1440
gagtggaggt ttgacagccg cctagcattt catcacgtgg cccgagagct gcatccggag 1500
tacttcaaga actgctga 1518

<210> 71
<211> 505
<212> PRT
<213> HIV

<400> 71
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Met Val Gly Phe Pro
        355                 360                 365
Val Thr Pro Gln Val Pro Leu Arg Pro Met Thr Tyr Lys Ala Ala Val
    370                 375                 380
Asp Leu Ser His Phe Leu Lys Glu Lys Gly Gly Leu Glu Gly Leu Ile
385                 390                 395                 400
His Ser Gln Arg Arg Gln Asp Ile Leu Asp Leu Trp Ile Tyr His Thr
                405                 410                 415
Gln Gly Tyr Phe Pro Asp Trp Gln Asn Tyr Thr Pro Gly Pro Gly Val
            420                 425                 430
Arg Tyr Pro Leu Thr Phe Gly Trp Cys Tyr Lys Leu Val Pro Val Glu
        435                 440                 445
Pro Asp Lys Val Glu Glu Ala Asn Lys Gly Glu Asn Thr Ser Leu Leu
    450                 455                 460
His Pro Val Ser Leu His Gly Met Asp Asp Pro Glu Arg Glu Val Leu
465                 470                 475                 480
Glu Trp Arg Phe Asp Ser Arg Leu Ala Phe His His Val Ala Arg Glu
                485                 490                 495
Leu His Pro Glu Tyr Phe Lys Asn Cys
            500                 505

<210> 72
<211> 1689
<212> DNA
<213> HIV

<400> 72
atgggcccca tcagtcccat cgagaccgtg ccggtgaagc tgaaacccgg gatggacggc 60
cccaaggtca agcagtggcc actcaccgag gagaagatca aggccctggt ggagatctgc 120
accgagatgg agaaagaggg caagatcagc aagatcgggc ctgagaaccc atacaacacc 180
cccgtgtttg ccatcaagaa gaaggacagc accaagtggc gcaagctggt ggatttccgg 240
gagctgaata agcggaccca ggatttctgg gaggtccagc tgggcatccc ccatccggcc 300
ggcctgaaga agaagaagag cgtgaccgtg ctggacgtgg gcgacgctta cttcagcgtc 360
cctctggacg aggactttag aaagtacacc gcctttacca tcccatctat caacaacgag 420
acccctggca tcagatatca gtacaacgtc ctcccccagg gctggaaggg ctctcccgcc 480
attttccaga gctccatgac caagatcctg gagccgtttc ggaagcagaa ccccgatatc 540
gtcatctacc agtacatgga cgacctgtac gtgggctctg acctggaaat cgggcagcat 600
cgcacgaaga ttgaggagct gaggcagcat ctgctgagat ggggcctgac cactccggac 660
aagaagcatc agaaggagcc gccattcctg aagatgggct acgagctcca tcccgacaag 720
tggaccgtgc agcctatcgt cctccccgag aaggacagct ggaccgtgaa cgacatccag 780
aagctggtgg gcaagctcaa ctgggctagc cagatctatc ccgggatcaa ggtgcgccag 840
ctctgcaagc tgctgcgcgg caccaaggcc ctgaccgagg tgattcccct cacggaggaa 900
gccgagctcg agctggctga gaaccgggag atcctgaagg agcccgtgca cggcgtgtac 960
tatgacccct ccaaggacct gatcgccgaa atccagaagc agggccaggg gcagtggaca 1020
taccagattt accaggagcc tttcaagaac ctcaagaccg gcaagtacgc ccgcatgagg 1080
ggcgcccaca ccaacgatgt caagcagctg accgaggccg tccagaagat cacgaccgag 1140
tccatcgtga tctgggggaa gacacccaag ttcaagctgc ctatccagaa ggagacctgg 1200
gagacgtggt ggaccgaata ttggcaggcc acctggattc ccgagtggga gttcgtgaat 1260
acacctcctc tggtgaagct gtggtaccag ctcgagaagg agcccatcgt gggcgcggag 1320
acattctacg tggacggcgc ggccaaccgc gaaacaaagc tcgggaaggc cgggtacgtc 1380
accaaccggg gccgccagaa ggtcgtcacc ctgaccgaca ccaccaacca gaagacggag 1440
ctgcaggcca tctatctcgc tctccaggac tccggcctgg aggtgaacat cgtgacggac 1500
agccagtacg cgctgggcat tattcaggcc cagccggacc agtccgagag cgaactggtg 1560
aaccagatta tcgagcagct gatcaagaaa gagaaggtct acctcgcctg ggtcccggcc 1620
cataagggca ttggcggcaa cgagcaggtc gacaagctgg tgagtgcggg gattagaaag 1680
gtgctgtaa 1689

<210> 73
<211> 3204
<212> DNA
<213> HIV

<400> 73
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300
ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat cctgggcctg aacaagatcg tgcgcatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tgggccccat cagtcccatc gagaccgtgc cggtgaagct gaaacccggg 1140
atggacggcc ccaaggtcaa gcagtggcca ctcaccgagg agaagatcaa ggccctggtg 1200
gagatctgca ccgagatgga gaaagagggc aagatcagca agatcgggcc tgagaaccca 1260
tacaacaccc ccgtgtttgc catcaagaag aaggacagca ccaagtggcg caagctggtg 1320
gatttccggg agctgaataa gcggacccag gatttctggg aggtccagct gggcatcccc 1380
catccggccg gcctgaagaa gaagaagagc gtgaccgtgc tggacgtggg cgacgcttac 1440
ttcagcgtcc ctctggacga ggactttaga aagtacaccg cctttaccat cccatctatc 1500
aacaacgaga cccctggcat cagatatcag tacaacgtcc tcccccaggg ctggaagggc 1560
tctcccgcca ttttccagag ctccatgacc aagatcctgg agccgtttcg gaagcagaac 1620
cccgatatcg tcatctacca gtacatggac gacctgtacg tgggctctga cctggaaatc 1680
gggcagcatc gcacgaagat tgaggagctg aggcagcatc tgctgagatg gggcctgacc 1740
actccggaca agaagcatca gaaggagccg ccattcctga agatgggcta cgagctccat 1800
cccgacaagt ggaccgtgca gcctatcgtc ctccccgaga aggacagctg gaccgtgaac 1860
gacatccaga agctggtggg caagctcaac tgggctagcc agatctatcc cgggatcaag 1920

gtgcgccagc tctgcaagct gctgcgcggc accaaggccc tgaccgaggt gattcccctc 1980
acggaggaag ccgagctcga gctggctgag aaccgggaga tcctgaagga gcccgtgcac 2040
ggcgtgtact atgacccctc caaggacctg atcgccgaaa tccagaagca gggccagggg 2100
cagtggacat accagattta ccaggagcct ttcaagaacc tcaagaccgg caagtacgcc 2160
cgcatgaggg gcgcccacac caacgatgtc aagcagctga ccgaggccgt ccagaagatc 2220
acgaccgagt ccatcgtgat ctgggggaag acacccaagt tcaagctgcc tatccagaag 2280
gagacctggg agacgtggtg gaccgaatat tggcaggcca cctggattcc cgagtgggag 2340
ttcgtgaata cacctcctct ggtgaagctg tggtaccagc tcgagaagga gcccatcgtg 2400
ggcgcggaga cattctacgt ggacggcgcg gccaaccgcg aaacaaagct cgggaaggcc 2460
gggtacgtca ccaaccgggg ccgccagaag gtcgtcaccc tgaccgacac caccaaccag 2520
aagacggagc tgcaggccat ctatctcgct ctccaggact ccggcctgga ggtgaacatc 2580
gtgacggaca gccagtacgc gctgggcatt attcaggccc agccggacca gtccgagagc 2640
gaactggtga accagattat cgagcagctg atcaagaaag agaaggtcta cctcgcctgg 2700
gtcccggccc ataagggcat tggcggcaac gagcaggtcg acaagctggt gagtgcgggg 2760
attagaaagg tgctgatggt gggttttcca gtcacacctc aggtaccttt aagaccaatg 2820
acttacaagg cagctgtaga tcttagccac tttttaaaag aaaagggggg actggaaggg 2880
ctaattcact cccaaagaag acaagatatc cttgatctgt ggatctacca cacacaaggc 2940
tacttccctg attggcagaa ctacacacca gggccagggg tcagatatcc actgaccttt 3000
ggatggtgct acaagctagt accagttgag ccagataagg tagaagaggc caataaagga 3060
gagaacacca gcttgttaca ccctgtgagc ctgcatggga tggatgaccc ggagagagaa 3120
gtgttagagt ggaggtttga cagccgccta gcatttcatc acgtggcccg agagctgcat 3180
ccggagtact tcaagaactg ctga 3204

<210> 74
<211> 1067
<212> PRT
<213> HIV

<400> 74
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160
Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Met Gly Pro Ile Ser
        355                 360                 365
Pro Ile Glu Thr Val Ser Val Lys Leu Lys Pro Gly Met Asp Gly Pro

370                 375                 380
Lys Val Lys Gln Trp Pro Leu Thr Glu Glu Lys Ile Lys Ala Leu Val
385                 390                 395                 400
Glu Ile Cys Thr Glu Met Glu Lys Glu Gly Lys Ile Ser Lys Ile Gly
                405                 410                 415
Pro Glu Asn Pro Tyr Asn Thr Pro Val Phe Ala Ile Lys Lys Lys Asp
            420                 425                 430
Ser Thr Lys Trp Arg Lys Leu Val Asp Phe Arg Glu Leu Asn Lys Arg
        435                 440                 445
Thr Gln Asp Phe Trp Glu Val Gln Leu Gly Ile Pro His Pro Ala Gly
    450                 455                 460
Leu Lys Lys Lys Lys Ser Val Thr Val Leu Asp Val Gly Asp Ala Tyr
465                 470                 475                 480
Phe Ser Val Pro Leu Asp Glu Asp Phe Arg Lys Tyr Thr Ala Phe Thr
                485                 490                 495
Ile Pro Ser Ile Asn Asn Glu Thr Pro Gly Ile Arg Tyr Gln Tyr Asn
            500                 505                 510
Val Leu Pro Gln Gly Trp Lys Gly Ser Pro Ala Ile Phe Gln Ser Ser
        515                 520                 525
Met Thr Lys Ile Leu Glu Pro Phe Arg Lys Gln Asn Pro Asp Ile Val
    530                 535                 540
Ile Tyr Gln Tyr Met Asp Asp Leu Tyr Val Gly Ser Asp Leu Glu Ile
545                 550                 555                 560
Gly Gln His Arg Thr Lys Ile Glu Glu Leu Arg Gln His Leu Leu Arg
                565                 570                 575
Trp Gly Leu Thr Thr Pro Asp Lys Lys His Gln Lys Glu Pro Pro Phe
            580                 585                 590
Leu Trp Met Gly Tyr Glu Leu His Pro Asp Lys Trp Thr Val Gln Pro
        595                 600                 605
Ile Val Leu Pro Glu Lys Asp Ser Trp Thr Val Asn Asp Ile Gln Lys
    610                 615                 620
Leu Val Gly Lys Leu Asn Trp Ala Ser Gln Ile Tyr Pro Gly Ile Lys
625                 630                 635                 640
Val Arg Gln Leu Cys Lys Leu Leu Arg Gly Thr Lys Ala Leu Thr Glu
                645                 650                 655
Val Ile Pro Leu Thr Glu Glu Ala Glu Leu Glu Leu Ala Glu Asn Arg
            660                 665                 670
Glu Ile Leu Lys Glu Pro Val His Gly Val Tyr Tyr Asp Pro Ser Lys
        675                 680                 685
Asp Leu Ile Ala Glu Ile Gln Lys Gln Gly Gln Gly Gln Trp Thr Tyr
    690                 695                 700
Gln Ile Tyr Gln Glu Pro Phe Lys Asn Leu Lys Thr Gly Lys Tyr Ala
705                 710                 715                 720
Arg Met Arg Gly Ala His Thr Asn Asp Val Lys Gln Leu Thr Glu Ala
                725                 730                 735
Val Gln Lys Ile Thr Thr Glu Ser Ile Val Ile Trp Gly Lys Thr Pro
            740                 745                 750
Lys Phe Lys Leu Pro Ile Gln Lys Glu Thr Trp Glu Thr Trp Trp Thr
        755                 760                 765
Glu Tyr Trp Gln Ala Thr Trp Ile Pro Glu Trp Glu Phe Val Asn Thr
    770                 775                 780
Pro Pro Leu Val Lys Leu Trp Tyr Gln Leu Glu Lys Glu Pro Ile Val
785                 790                 795                 800
Gly Ala Glu Thr Phe Tyr Val Asp Gly Ala Ala Asn Arg Glu Thr Lys
                805                 810                 815
Leu Gly Lys Ala Gly Tyr Val Thr Asn Arg Gly Arg Gln Lys Val Val
            820                 825                 830
Thr Leu Thr Asp Thr Thr Asn Gln Lys Thr Glu Leu Gln Ala Ile Tyr
        835                 840                 845
Leu Ala Leu Gln Asp Ser Gly Leu Glu Val Asn Ile Val Thr Asp Ser
    850                 855                 860
Gln Tyr Ala Leu Gly Ile Ile Gln Ala Gln Pro Asp Gln Ser Glu Ser
865                 870                 875                 880
Glu Leu Val Asn Gln Ile Ile Glu Gln Leu Ile Lys Lys Glu Lys Val
                885                 890                 895
Tyr Leu Ala Trp Val Pro Ala His Lys Gly Ile Gly Gly Asn Glu Gln
            900                 905                 910
Val Asp Lys Leu Val Ser Ala Gly Ile Arg Lys Val Leu Met Val Gly
        915                 920                 925
Phe Pro Val Thr Pro Gln Val Pro Leu Arg Pro Met Thr Tyr Lys Ala
    930                 935                 940
Ala Val Asp Leu Ser His Phe Leu Lys Glu Lys Gly Gly Leu Glu Gly
945                 950                 955                 960
Leu Ile His Ser Gln Arg Arg Gln Asp Ile Leu Asp Leu Trp Ile Tyr
                965                 970                 975
His Thr Gln Gly Tyr Phe Pro Asp Trp Gln Asn Tyr Thr Pro Gly Pro

980                 985                 990
Gly Val Arg Tyr Pro Leu Thr Phe Gly Trp Cys Tyr Lys Leu Val Pro
        995                 1000                1005
Val Glu Pro Asp Lys Val Glu Glu Ala Asn Lys Gly Glu Asn Thr Ser
    1010                1015                1020
Leu Leu His Pro Val Ser Leu His Gly Met Asp Asp Pro Glu Arg Glu
1025                1030                1035                1040
Val Leu Glu Trp Arg Phe Asp Ser Arg Leu Ala Phe His His Val Ala
                1045                1050                1055
Arg Glu Leu His Pro Glu Tyr Phe Lys Asn Cys
                1060                1065

<210> 75
<211> 3204
<212> DNA
<213> HIV

<400> 75
   atggtgggtt ttccagtcac acctcaggta cctttaagac caatgactta caaggcagct 60
   gtagatctta gccacttttt aaaagaaaag gggggactgg aagggctaat tcactcccaa 120
   agaagacaag atatccttga tctgtggatc taccacacac aaggctactt ccctgattgg 180
   cagaactaca caccagggcc aggggtcaga tatccactga cctttggatg gtgctacaag 240
   ctagtaccag ttgagccaga taaggtagaa gaggccaata aaggagagaa caccagcttg 300
   ttacaccctg tgagcctgca tgggatggat gacccggaga gagaagtgtt agagtggagg 360
   tttgacagcc gcctagcatt tcatcacgtg gcccgagagc tgcatccgga gtacttcaag 420
   aactgcatgg gccccatcag tcccatcgag accgtgccgg tgaagctgaa acccgggatg 480
   gacggcccca aggtcaagca gtggccactc accgaggaga agatcaaggc cctggtggag 540
   atctgcaccg agatggagaa agagggcaag atcagcaaga tcgggcctga gaacccatac 600
   aacacccccg tgtttgccat caagaagaag gacagcacca agtggcgcaa gctggtggat 660
   ttccgggagc tgaataagcg gacccaggat ttctgggagg tccagctggg catcccccat 720
   ccggccggcc tgaagaagaa gaagagcgtg accgtgctgg acgtgggcga cgcttacttc 780
   agcgtccctc tggacgagga ctttagaaag tacaccgcct ttaccatccc atctatcaac 840
   aacgagaccc ctggcatcag atatcagtac aacgtcctcc cccagggctg gaagggctct 900
   cccgccattt tccagagctc catgaccaag atcctggagc cgtttcggaa gcagaacccc 960
   gatatcgtca tctaccagta catggacgac ctgtacgtgg gctctgacct ggaaatcggg 1020
   cagcatcgca cgaagattga ggagctgagg cagcatctgc tgagatgggg cctgaccact 1080
   ccggacaaga agcatcagaa ggagccgcca ttcctgaaga tgggctacga gctccatccc 1140
   gacaagtgga ccgtgcagcc tatcgtcctc cccgagaagg acagctggac cgtgaacgac 1200
   atccagaagc tggtgggcaa gctcaactgg gctagccaga tctatcccgg gatcaaggtg 1260
   cgccagctct gcaagctgct gcgcggcacc aaggccctga ccgaggtgat tcccctcacg 1320
   gaggaagccg agctcgagct ggctgagaac cgggagatcc tgaaggagcc cgtgcacggc 1380
   gtgtactatg acccctccaa ggacctgatc gccgaaatcc agaagcaggg ccaggggcag 1440
   tggacatacc agatttacca ggagcctttc aagaacctca agaccggcaa gtacgcccgc 1500
   atgaggggcg cccacaccaa cgatgtcaag cagctgaccg aggccgtcca gaagatcacg 1560
   accgagtcca tcgtgatctg ggggaagaca cccaagttca agctgcctat ccagaaggag 1620
   acctgggaga cgtggtggac cgaatattgg caggccacct ggattcccga gtgggagttc 1680
   gtgaatacac ctcctctggt gaagctgtgg taccagctcg agaaggagcc catcgtgggc 1740
   gcggagacat tctacgtgga cggcgcggcc aaccgcgaaa caaagctcgg gaaggccggg 1800
   tacgtcacca accgggggcg ccagaaggtc gtcaccctga ccgacaccac caaccagaag 1860
   acggagctgc aggccatcta tctcgctctc caggactccg gcctggaggt gaacatcgtg 1920
   acggacagcc agtacgcgct gggcattatt caggcccagc cggaccagtc cgagagcgaa 1980
   ctggtgaacc agattatcga gcagctgatc aagaaagaga aggtctacct cgcctgggtc 2040
   ccggcccata agggcattgg cggcaacgag caggtcgaca agctggtgag tgcggggatt 2100
   agaaaggtgc tgatgggtgc ccgagcttcg gtactgtctg gtggagagct ggacagatgg 2160
   gagaaaatta ggctgcgccc gggaggcaaa aagaaataca agctcaagca tatcgtgtgg 2220
   gcctcgaggg agcttgaacg gtttgccgtg aacccaggcc tgctggaaac atctgaggga 2280
   tgtcgccaga tcctggggca attgcagcca tccctccaga ccgggagtga agagctgagg 2340
   tccttgtata acacagtggc taccctctac tgcgtacacc agaggatcga gattaaggat 2400
   accaaggagg ccttggacaa aattgaggag gagcaaaaca agagcaagaa gaaggcccag 2460
   caggcagctg ctgacactgg gcatagcaac caggtatcac agaactatcc tattgtccaa 2520
   aacattcagg gccagatggt tcatcaggcc atcagccccc ggacgctcaa tgcctgggtg 2580
   aaggttgtcg aagagaaggc cttttctcct gaggttatcc ccatgttctc cgctttgagt 2640
   gagggggcca ctcctcagga cctcaataca atgcttaata ccgtgggcgg ccatcaggcc 2700
   gccatgcaaa tgttgaagga gactatcaac gaggaggcag ccgagtggga cagagtgcat 2760
   cccgtccacg ctggcccaat cgcgcccgga cagatgcggg agcctcgcgg ctctgacatt 2820
   gccggcacca cctctacact gcaagagcaa atcggatgga tgaccaacaa tcctcccatc 2880
   ccagttggag aaatctataa acggtggatc atcctgggcc tgaacaagat cgtgcgcatg 2940
   tactctccga catccatcct tgacattaga cagggaccca aagagccttt tagggattac 3000
   gtcgaccggt tttataagac cctgcgagca gagcaggcct ctcaggaggt caaaaactgg 3060
   atgacggaga cactcctggt acagaacgct aaccccgact gcaaaacaat cttgaaggca 3120
   ctaggcccgg ctgccaccct ggaagagatg atgaccgcct gtcagggagt aggcggaccc 3180
   ggacacaaag ccagagtgtt gtga 3204

<210> 76
<211> 1067

<212> PRT
<213> HIV

<400> 76
Met Val Gly Phe Pro Val Thr Pro Gln Val Pro Leu Arg Pro Met Thr
1               5                   10                  15
Tyr Lys Ala Ala Val Asp Leu Ser His Phe Leu Lys Glu Lys Gly Gly
            20                  25                  30
Leu Glu Gly Leu Ile His Ser Gln Arg Arg Gln Asp Ile Leu Asp Leu
        35                  40                  45
Trp Ile Tyr His Thr Gln Gly Tyr Phe Pro Asp Trp Gln Asn Tyr Thr
    50                  55                  60
Pro Gly Pro Gly Val Arg Tyr Pro Leu Thr Phe Gly Trp Cys Tyr Lys
65                  70                  75                  80
Leu Val Pro Val Glu Pro Asp Lys Val Glu Glu Ala Asn Lys Gly Glu
                85                  90                  95
Asn Thr Ser Leu Leu His Pro Val Ser Leu His Gly Met Asp Asp Pro
            100                 105                 110
Glu Arg Glu Val Leu Glu Trp Arg Phe Asp Ser Arg Leu Ala Phe His
        115                 120                 125
His Val Ala Arg Glu Leu His Pro Glu Tyr Phe Lys Asn Cys Met Gly
    130                 135                 140
Pro Ile Ser Pro Ile Glu Thr Val Ser Val Lys Leu Lys Pro Gly Met
145                 150                 155                 160
Asp Gly Pro Lys Val Lys Gln Trp Pro Leu Thr Glu Glu Lys Ile Lys
                165                 170                 175
Ala Leu Val Glu Ile Cys Thr Glu Met Glu Lys Glu Gly Lys Ile Ser
            180                 185                 190
Lys Ile Gly Pro Glu Asn Pro Tyr Asn Thr Pro Val Phe Ala Ile Lys
        195                 200                 205
Lys Lys Asp Ser Thr Lys Trp Arg Lys Leu Val Asp Phe Arg Glu Leu
    210                 215                 220
Asn Lys Arg Thr Gln Asp Phe Trp Glu Val Gln Leu Gly Ile Pro His
225                 230                 235                 240
Pro Ala Gly Leu Lys Lys Lys Lys Ser Val Thr Val Leu Asp Val Gly
                245                 250                 255
Asp Ala Tyr Phe Ser Val Pro Leu Asp Glu Asp Phe Arg Lys Tyr Thr
            260                 265                 270
Ala Phe Thr Ile Pro Ser Ile Asn Asn Glu Thr Pro Gly Ile Arg Tyr
        275                 280                 285
Gln Tyr Asn Val Leu Pro Gln Gly Trp Lys Gly Ser Pro Ala Ile Phe
    290                 295                 300
Gln Ser Ser Met Thr Lys Ile Leu Glu Pro Phe Arg Lys Gln Asn Pro
305                 310                 315                 320
Asp Ile Val Ile Tyr Gln Tyr Met Asp Asp Leu Tyr Val Gly Ser Asp
                325                 330                 335
Leu Glu Ile Gly Gln His Arg Thr Lys Ile Glu Glu Leu Arg Gln His
            340                 345                 350
Leu Leu Arg Trp Gly Leu Thr Thr Pro Asp Lys Lys His Gln Lys Glu
        355                 360                 365
Pro Pro Phe Leu Trp Met Gly Tyr Glu Leu His Pro Asp Lys Trp Thr
    370                 375                 380
Val Gln Pro Ile Val Leu Pro Glu Lys Asp Ser Trp Thr Val Asn Asp
385                 390                 395                 400
Ile Gln Lys Leu Val Gly Lys Leu Asn Trp Ala Ser Gln Ile Tyr Pro
                405                 410                 415
Gly Ile Lys Val Arg Gln Leu Cys Lys Leu Leu Arg Gly Thr Lys Ala
            420                 425                 430
Leu Thr Glu Val Ile Pro Leu Thr Glu Glu Ala Glu Leu Glu Leu Ala
        435                 440                 445
Glu Asn Arg Glu Ile Leu Lys Glu Pro Val His Gly Val Tyr Tyr Asp
    450                 455                 460
Pro Ser Lys Asp Leu Ile Ala Glu Ile Gln Lys Gln Gly Gln Gly Gln
465                 470                 475                 480
Trp Thr Tyr Gln Ile Tyr Gln Glu Pro Phe Lys Asn Leu Lys Thr Gly
                485                 490                 495
Lys Tyr Ala Arg Met Arg Gly Ala His Thr Asn Asp Val Lys Gln Leu
            500                 505                 510
Thr Glu Ala Val Gln Lys Ile Thr Thr Glu Ser Ile Val Ile Trp Gly
        515                 520                 525
Lys Thr Pro Lys Phe Lys Leu Pro Ile Gln Lys Glu Thr Trp Glu Thr
    530                 535                 540
Trp Trp Thr Glu Tyr Trp Gln Ala Thr Trp Ile Pro Glu Trp Glu Phe
545                 550                 555                 560
Val Asn Thr Pro Pro Leu Val Lys Leu Trp Tyr Gln Leu Glu Lys Glu
                565                 570                 575

Pro Ile Val Gly Ala Glu Thr Phe Tyr Val Asp Gly Ala Ala Asn Arg
            580                 585                 590
Glu Thr Lys Leu Gly Lys Ala Gly Tyr Val Thr Asn Arg Gly Arg Gln
        595                 600                 605
Lys Val Val Thr Leu Thr Asp Thr Thr Asn Gln Lys Thr Glu Leu Gln
    610                 615                 620
Ala Ile Tyr Leu Ala Leu Gln Asp Ser Gly Leu Glu Val Asn Ile Val
625                 630                 635                 640
Thr Asp Ser Gln Tyr Ala Leu Gly Ile Ile Gln Ala Gln Pro Asp Gln
                645                 650                 655
Ser Glu Ser Glu Leu Val Asn Gln Ile Ile Glu Gln Leu Ile Lys Lys
            660                 665                 670
Glu Lys Val Tyr Leu Ala Trp Val Pro Ala His Lys Gly Ile Gly Gly
        675                 680                 685
Asn Glu Gln Val Asp Lys Leu Val Ser Ala Gly Ile Arg Lys Val Leu
    690                 695                 700
Met Gly Ala Arg Ala Ser Val Leu Ser Gly Gly Glu Leu Asp Arg Trp
705                 710                 715                 720
Glu Lys Ile Arg Leu Arg Pro Gly Gly Lys Lys Lys Tyr Lys Leu Lys
                725                 730                 735
His Ile Val Trp Ala Ser Arg Glu Leu Glu Arg Phe Ala Val Asn Pro
            740                 745                 750
Gly Leu Leu Glu Thr Ser Glu Gly Cys Arg Gln Ile Leu Gly Gln Leu
        755                 760                 765
Gln Pro Ser Leu Gln Thr Gly Ser Glu Glu Leu Arg Ser Leu Tyr Asn
    770                 775                 780
Thr Val Ala Thr Leu Tyr Cys Val His Gln Arg Ile Glu Ile Lys Asp
785                 790                 795                 800
Thr Lys Glu Ala Leu Asp Lys Ile Glu Glu Glu Gln Asn Lys Ser Lys
                805                 810                 815
Lys Lys Ala Gln Gln Ala Ala Ala Asp Thr Gly His Ser Asn Gln Val
            820                 825                 830
Ser Gln Asn Tyr Pro Ile Val Gln Asn Ile Gln Gly Gln Met Val His
        835                 840                 845
Gln Ala Ile Ser Pro Arg Thr Leu Asn Ala Trp Val Lys Val Val Glu
    850                 855                 860
Glu Lys Ala Phe Ser Pro Glu Val Ile Pro Met Phe Ser Ala Leu Ser
865                 870                 875                 880
Glu Gly Ala Thr Pro Gln Asp Leu Asn Thr Met Leu Asn Thr Val Gly
                885                 890                 895
Gly His Gln Ala Ala Met Gln Met Leu Lys Glu Thr Ile Asn Glu Glu
            900                 905                 910
Ala Ala Glu Trp Asp Arg Val His Pro Val His Ala Gly Pro Ile Ala
        915                 920                 925
Pro Gly Gln Met Arg Glu Pro Arg Gly Ser Asp Ile Ala Gly Thr Thr
    930                 935                 940
Ser Thr Leu Gln Glu Gln Ile Gly Trp Met Thr Asn Asn Pro Pro Ile
945                 950                 955                 960
Pro Val Gly Glu Ile Tyr Lys Arg Trp Ile Ile Leu Gly Leu Asn Lys
                965                 970                 975
Ile Val Arg Met Tyr Ser Pro Thr Ser Ile Leu Asp Ile Arg Gln Gly
            980                 985                 990
Pro Lys Glu Pro Phe Arg Asp Tyr Val Asp Arg Phe Tyr Lys Thr Leu
        995                 1000                1005
Arg Ala Glu Gln Ala Ser Gln Glu Val Lys Asn Trp Met Thr Glu Thr
    1010                1015                1020
Leu Leu Val Gln Asn Ala Asn Pro Asp Cys Lys Thr Ile Leu Lys Ala
1025                1030                1035                1040
Leu Gly Pro Ala Ala Thr Leu Glu Glu Met Met Thr Ala Cys Gln Gly
                1045                1050                1055
Val Gly Gly Pro Gly His Lys Ala Arg Val Leu
            1060                1065

<210> 77
<211> 3204
<212> DNA
<213> HIV

<400> 77
atggtgggtt ttccagtcac acctcaggta cctttaagac caatgactta caaggcagct 60
gtagatctta gccacttttt aaaagaaaag gggggactgg aagggctaat tcactcccaa 120
agaagacaag atatccttga tctgtggatc taccacacac aaggctactt ccctgattgg 180
cagaactaca caccagggcc aggggtcaga tatccactga cctttggatg gtgctacaag 240
ctagtaccag ttgagccaga taaggtagaa gaggccaata aaggagagaa caccagcttg 300
ttacaccctg tgagcctgca tgggatggat gacccggaga gagaagtgtt agagtggagg 360

tttgacagcc gcctagcatt tcatcacgtg gcccgagagc tgcatccgga gtacttcaag 420
aactgcatgg gtgcccgagc ttcggtactg tctggtggag agctggacag atgggagaaa 480
attaggctgc gcccgggagg caaaaagaaa tacaagctca agcatatcgt gtgggcctcg 540
agggagcttg aacggtttgc cgtgaaccca ggcctgctgg aaacatctga gggatgtcgc 600
cagatcctgg ggcaattgca gccatccctc cagaccggga gtgaagagct gaggtccttg 660
tataacacag tggctaccct ctactgcgta caccagagga tcgagattaa ggataccaag 720
gaggccttgg acaaaattga ggaggagcaa aacaagagca agaagaaggc ccagcaggca 780
gctgctgaca ctgggcatag caaccaggta tcacagaact atcctattgt ccaaaacatt 840
cagggccaga tggttcatca ggccatcagc ccccggacgc tcaatgcctg ggtgaaggtt 900
gtcgaagaga aggccttttc tcctgaggtt atccccatgt tctccgcttt gagtgagggg 960
gccactcctc aggacctcaa tacaatgctt aataccgtgg gcggccatca ggccgccatg 1020
caaatgttga aggagactat caacgaggag gcagccgagt gggacagagt gcatcccgtc 1080
cacgctggcc caatcgcgcc cggacagatg cgggagcctc gcggctctga cattgccggc 1140
accacctcta cactgcaaga gcaaatcgga tggatgacca acaatcctcc catcccagtt 1200
ggagaaatct ataaacggtg gatcatcctg ggcctgaaca agatcgtgcg catgtactct 1260
ccgacatcca tccttgacat tagacaggga cccaaagagc cttttaggga ttacgtcgac 1320
cggttttata agaccctgcg agcagagcag gcctctcagg aggtcaaaaa ctggatgacg 1380
gagacactcc tggtacagaa cgctaacccc gactgcaaaa caatcttgaa ggcactaggc 1440
ccggctgcca ccctggaaga gatgatgacc gcctgtcagg gagtaggcgg acccggacac 1500
aaagccagag tgttgatggg ccccatcagt cccatcgaga ccgtgccggt gaagctgaaa 1560
cccgggatgg acggccccaa ggtcaagcag tggccactca ccgaggagaa gatcaaggcc 1620
ctggtggaga tctgcaccga gatggagaaa gagggcaaga tcagcaagat cgggcctgag 1680
aacccataca acacccccgt gtttgccatc aagaagaagg acagcaccaa gtggcgcaag 1740
ctggtggatt tccgggagct gaataagcgg acccaggatt tctgggaggt ccagctgggc 1800
atcccccatc cggccggcct gaagaagaag aagagcgtga ccgtgctgga cgtgggcgac 1860
gcttacttca gcgtccctct ggacgaggac tttagaaagt acaccgcctt taccatccca 1920
tctatcaaca acgagacccc tggcatcaga tatcagtaca acgtcctccc ccagggctgg 1980
aagggctctc ccgccatttt ccagagctcc atgaccaaga tcctggagcc gtttcggaag 2040
cagaaccccg atatcgtcat ctaccagtac atggacgacc tgtacgtggg ctctgacctg 2100
gaaatcgggc agcatcgcac gaagattgag gagctgaggc agcatctgct gagatggggc 2160
ctgaccactc cggacaagaa gcatcagaag gagccgccat tcctgaagat gggctacgag 2220
ctccatcccg acaagtggac cgtgcagcct atcgtcctcc ccgagaagga cagctggacc 2280
gtgaacgaca tccagaagct ggtgggcaag ctcaactggg ctagccagat ctatcccggg 2340
atcaaggtgc gccagctctg caagctgctg cgcggcacca aggccctgac cgaggtgatt 2400
cccctcacgg aggaagccga gctcgagctg gctgagaacc gggagatcct gaaggagccc 2460
gtgcacggcg tgtactatga cccctccaag gacctgatcg ccgaaatcca gaagcagggc 2520
caggggcagt ggacatacca gatttaccag gagcctttca agaacctcaa gaccggcaag 2580
tacgcccgca tgaggggcgc ccacaccaac gatgtcaagc agctgaccga ggccgtccag 2640
aagatcacga ccgagtccat cgtgatctgg gggaagacac ccaagttcaa gctgcctatc 2700
cagaaggaga cctgggagac gtggtggacc gaatattggc aggccacctg gattcccgag 2760
tgggagttcg tgaatacacc tcctctggtg aagctgtggt accagctcga gaaggagccc 2820
atcgtgggcg cggagacatt ctacgtggac ggcgcggcca accgcgaaac aaagctcggg 2880
aaggccgggt acgtcaccaa ccggggccgc cagaaggtcg tcaccctgac cgacaccacc 2940
aaccagaaga cggagctgca ggccatctat ctcgctctcc aggactccgg cctggaggtg 3000
aacatcgtga cggacagcca gtacgcgctg ggcattattc aggcccagcc ggaccagtcc 3060
gagagcgaac tggtgaacca gattatcgag cagctgatca agaaagagaa ggtctacctc 3120
gcctgggtcc cggcccataa gggcattggc ggcaacgagc aggtcgacaa gctggtgagt 3180
gcggggatta gaaaggtgct gtaa 3204

<210> 78
<211> 1067
<212> PRT
<213> HIV

<400> 78
Met Val Gly Phe Pro Val Thr Pro Gln Val Pro Leu Arg Pro Met Thr
1               5                   10                  15
Tyr Lys Ala Ala Val Asp Leu Ser His Phe Leu Lys Glu Lys Gly Gly
            20                  25                  30
Leu Glu Gly Leu Ile His Ser Gln Arg Arg Gln Asp Ile Leu Asp Leu
        35                  40                  45
Trp Ile Tyr His Thr Gln Gly Tyr Phe Pro Asp Trp Gln Asn Tyr Thr
    50                  55                  60
Pro Gly Pro Gly Val Arg Tyr Pro Leu Thr Phe Gly Trp Cys Tyr Lys
65                  70                  75                  80
Leu Val Pro Val Glu Pro Asp Lys Val Glu Glu Ala Asn Lys Gly Glu
                85                  90                  95
Asn Thr Ser Leu Leu His Pro Val Ser Leu His Gly Met Asp Asp Pro
            100                 105                 110
Glu Arg Glu Val Leu Glu Trp Arg Phe Asp Ser Arg Leu Ala Phe His
        115                 120                 125
His Val Ala Arg Glu Leu His Pro Glu Tyr Phe Lys Asn Cys Met Gly
    130                 135                 140
Ala Arg Ala Ser Val Leu Ser Gly Gly Glu Leu Asp Arg Trp Glu Lys
145                 150                 155                 160
Ile Arg Leu Arg Pro Gly Gly Lys Lys Lys Tyr Lys Leu Lys His Ile

                    165                 170                 175
Val Trp Ala Ser Arg Glu Leu Glu Arg Phe Ala Val Asn Pro Gly Leu
                180                 185                 190
Leu Glu Thr Ser Glu Gly Cys Arg Gln Ile Leu Gly Gln Leu Gln Pro
            195                 200                 205
Ser Leu Gln Thr Gly Ser Glu Glu Leu Arg Ser Leu Tyr Asn Thr Val
        210                 215                 220
Ala Thr Leu Tyr Cys Val His Gln Arg Ile Glu Ile Lys Asp Thr Lys
225                 230                 235                 240
Glu Ala Leu Asp Lys Ile Glu Glu Glu Gln Asn Lys Ser Lys Lys Lys
                245                 250                 255
Ala Gln Gln Ala Ala Ala Asp Thr Gly His Ser Asn Gln Val Ser Gln
            260                 265                 270
Asn Tyr Pro Ile Val Gln Asn Ile Gln Gly Gln Met Val His Gln Ala
        275                 280                 285
Ile Ser Pro Arg Thr Leu Asn Ala Trp Val Lys Val Val Glu Glu Lys
    290                 295                 300
Ala Phe Ser Pro Glu Val Ile Pro Met Phe Ser Ala Leu Ser Glu Gly
305                 310                 315                 320
Ala Thr Pro Gln Asp Leu Asn Thr Met Leu Asn Thr Val Gly Gly His
                325                 330                 335
Gln Ala Ala Met Gln Met Leu Lys Glu Thr Ile Asn Glu Glu Ala Ala
            340                 345                 350
Glu Trp Asp Arg Val His Pro Val His Ala Gly Pro Ile Ala Pro Gly
        355                 360                 365
Gln Met Arg Glu Pro Arg Gly Ser Asp Ile Ala Gly Thr Thr Ser Thr
    370                 375                 380
Leu Gln Glu Gln Ile Gly Trp Met Thr Asn Asn Pro Pro Ile Pro Val
385                 390                 395                 400
Gly Glu Ile Tyr Lys Arg Trp Ile Ile Leu Gly Leu Asn Lys Ile Val
                405                 410                 415
Arg Met Tyr Ser Pro Thr Ser Ile Leu Asp Ile Arg Gln Gly Pro Lys
            420                 425                 430
Glu Pro Phe Arg Asp Tyr Val Asp Arg Phe Tyr Lys Thr Leu Arg Ala
        435                 440                 445
Glu Gln Ala Ser Gln Glu Val Lys Asn Trp Met Thr Glu Thr Leu Leu
    450                 455                 460
Val Gln Asn Ala Asn Pro Asp Cys Lys Thr Ile Leu Lys Ala Leu Gly
465                 470                 475                 480
Pro Ala Ala Thr Leu Glu Glu Met Met Thr Ala Cys Gln Gly Val Gly
                485                 490                 495
Gly Pro Gly His Lys Ala Arg Val Leu Met Gly Pro Ile Ser Pro Ile
            500                 505                 510
Glu Thr Val Ser Val Lys Leu Lys Pro Gly Met Asp Gly Pro Lys Val
        515                 520                 525
Lys Gln Trp Pro Leu Thr Glu Glu Lys Ile Lys Ala Leu Val Glu Ile
    530                 535                 540
Cys Thr Glu Met Glu Lys Glu Gly Lys Ile Ser Lys Ile Gly Pro Glu
545                 550                 555                 560
Asn Pro Tyr Asn Thr Pro Val Phe Ala Ile Lys Lys Lys Asp Ser Thr
                565                 570                 575
Lys Trp Arg Lys Leu Val Asp Phe Arg Glu Leu Asn Lys Arg Thr Gln
            580                 585                 590
Asp Phe Trp Glu Val Gln Leu Gly Ile Pro His Pro Ala Gly Leu Lys
        595                 600                 605
Lys Lys Lys Ser Val Thr Val Leu Asp Val Gly Asp Ala Tyr Phe Ser
    610                 615                 620
Val Pro Leu Asp Glu Asp Phe Arg Lys Tyr Thr Ala Phe Thr Ile Pro
625                 630                 635                 640
Ser Ile Asn Asn Glu Thr Pro Gly Ile Arg Tyr Gln Tyr Asn Val Leu
                645                 650                 655
Pro Gln Gly Trp Lys Gly Ser Pro Ala Ile Phe Gln Ser Ser Met Thr
            660                 665                 670
Lys Ile Leu Glu Pro Phe Arg Lys Gln Asn Pro Asp Ile Val Ile Tyr
        675                 680                 685
Gln Tyr Met Asp Asp Leu Tyr Val Gly Ser Asp Leu Glu Ile Gly Gln
    690                 695                 700
His Arg Thr Lys Ile Glu Glu Leu Arg Gln His Leu Leu Arg Trp Gly
705                 710                 715                 720
Leu Thr Thr Pro Asp Lys Lys His Gln Lys Glu Pro Pro Phe Leu Trp
                725                 730                 735
Met Gly Tyr Glu Leu His Pro Asp Lys Trp Thr Val Gln Pro Ile Val
            740                 745                 750
Leu Pro Glu Lys Asp Ser Trp Thr Val Asn Asp Ile Gln Lys Leu Val
        755                 760                 765
Gly Lys Leu Asn Trp Ala Ser Gln Ile Tyr Pro Gly Ile Lys Val Arg

770                 775                 780
Gln Leu Cys Lys Leu Leu Arg Gly Thr Lys Ala Leu Thr Glu Val Ile
785                 790                 795                 800
Pro Leu Thr Glu Glu Ala Glu Leu Glu Leu Ala Glu Asn Arg Glu Ile
                805                 810                 815
Leu Lys Glu Pro Val His Gly Val Tyr Tyr Asp Pro Ser Lys Asp Leu
            820                 825                 830
Ile Ala Glu Ile Gln Lys Gln Gly Gln Gly Gln Trp Thr Tyr Gln Ile
        835                 840                 845
Tyr Gln Glu Pro Phe Lys Asn Leu Lys Thr Gly Lys Tyr Ala Arg Met
    850                 855                 860
Arg Gly Ala His Thr Asn Asp Val Lys Gln Leu Thr Glu Ala Val Gln
865                 870                 875                 880
Lys Ile Thr Thr Glu Ser Ile Val Ile Trp Gly Lys Thr Pro Lys Phe
                885                 890                 895
Lys Leu Pro Ile Gln Lys Glu Thr Trp Glu Thr Trp Trp Thr Glu Tyr
            900                 905                 910
Trp Gln Ala Thr Trp Ile Pro Glu Trp Glu Phe Val Asn Thr Pro Pro
        915                 920                 925
Leu Val Lys Leu Trp Tyr Gln Leu Glu Lys Glu Pro Ile Val Gly Ala
    930                 935                 940
Glu Thr Phe Tyr Val Asp Gly Ala Ala Asn Arg Glu Thr Lys Leu Gly
945                 950                 955                 960
Lys Ala Gly Tyr Val Thr Asn Arg Gly Arg Gln Lys Val Val Thr Leu
                965                 970                 975
Thr Asp Thr Thr Asn Gln Lys Thr Glu Leu Gln Ala Ile Tyr Leu Ala
            980                 985                 990
Leu Gln Asp Ser Gly Leu Glu Val Asn Ile Val Thr Asp Ser Gln Tyr
        995                 1000                1005
Ala Leu Gly Ile Ile Gln Ala Gln Pro Asp Gln Ser Glu Ser Glu Leu
    1010                1015                1020
Val Asn Gln Ile Ile Glu Gln Leu Ile Lys Lys Glu Lys Val Tyr Leu
1025                1030                1035                1040
Ala Trp Val Pro Ala His Lys Gly Ile Gly Gly Asn Glu Gln Val Asp
                1045                1050                1055
Lys Leu Val Ser Ala Gly Ile Arg Lys Val Leu
            1060                1065

<210> 79
<211> 3204
<212> DNA
<213> HIV

<400> 79
atgggcccca tcagtcccat cgagaccgtg ccggtgaagc tgaaacccgg gatggacggc 60
cccaaggtca agcagtggcc actcaccgag gagaagatca aggccctggt ggagatctgc 120
accgagatgg agaaagaggg caagatcagc aagatcgggc ctgagaaccc atacaacacc 180
cccgtgtttg ccatcaagaa gaaggacagc accaagtggc gcaagctggt ggatttccgg 240
gagctgaata agcggaccca ggatttctgg gaggtccagc tgggcatccc ccatccggcc 300
ggcctgaaga agaagaagag cgtgaccgtg ctggacgtgg gcgacgctta cttcagcgtc 360
cctctggacg aggactttag aaagtacacc gcctttacca tcccatctat caacaacgag 420
acccctggca tcagatatca gtacaacgtc ctcccccagg gctggaaggg ctctcccgcc 480
attttccaga gctccatgac caagatcctg gagccgtttc ggaagcagaa ccccgatatc 540
gtcatctacc agtacatgga cgacctgtac gtgggctctg acctggaaat cgggcagcat 600
cgcacgaaga ttgaggagct gaggcagcat ctgctgagat ggggcctgac cactccggac 660
aagaagcatc agaaggagcc gccattcctg aagatgggct acgagctcca tcccgacaag 720
tggaccgtgc agcctatcgt cctccccgag aaggacagct ggaccgtgaa cgacatccag 780
aagctggtgg gcaagctcaa ctgggctagc cagatctatc ccgggatcaa ggtgcgccag 840
ctctgcaagc tgctgcgcgg caccaaggcc ctgaccgagg tgattcccct cacggaggaa 900
gccgagctcg agctggctga gaaccgggag atcctgaagg agcccgtgca cggcgtgtac 960
tatgacccct ccaaggacct gatcgccgaa atccagaagc agggccaggg gcagtggaca 1020
taccagattt accaggagcc tttcaagaac ctcaagaccg gcaagtacgc ccgcatgagg 1080
ggcgcccaca ccaacgatgt caagcagctg accgaggccg tccagaagat cacgaccgag 1140
tccatcgtga tctgggggaa gacacccaag ttcaagctgc ctatccagaa ggagacctgg 1200
gagacgtggt ggaccgaata ttggcaggcc acctggattc ccgagtggga gttcgtgaat 1260
acacctcctc tggtgaagct gtggtaccag ctcgagaagg agcccatcgt gggcgcggag 1320
acattctacg tggacggcgc ggccaaccgc gaaacaaagc tcgggaaggc cgggtacgtc 1380
accaaccggg gccgccagaa ggtcgtcacc ctgaccgaca ccaccaacca gaagacggag 1440
ctgcaggcca tctatctcgc tctccaggac tccggcctgg aggtgaacat cgtgacggac 1500
agccagtacg cgctgggcat tattcaggcc cagccggacc agtccgagag cgaactggtg 1560
aaccagatta tcgagcagct gatcaagaaa gagaaggtct acctcgcctg ggtcccggcc 1620
cataagggca ttggcggcaa cgagcaggtc gacaagctgg tgagtgcggg gattagaaag 1680
gtgctgatgg gtgcccgagc ttcggtactg tctggtggag agctggacag atgggagaaa 1740
attaggctgc gcccgggagg caaaaagaaa tacaagctca agcatatcgt gtgggcctcg 1800
agggagcttg aacggtttgc cgtgaaccca ggcctgctgg aaacatctga gggatgtcgc 1860

cagatcctgg ggcaattgca gccatccctc cagaccggga gtgaagagct gaggtccttg 1920
tataacacag tggctaccct ctactgcgta caccagagga tcgagattaa ggataccaag 1980
gaggccttgg acaaaattga ggaggagcaa aacaagagca agaagaaggc ccagcaggca 2040
gctgctgaca ctgggcatag caaccaggta tcacagaact atcctattgt ccaaaacatt 2100
cagggccaga tggttcatca ggccatcagc ccccggacgc tcaatgcctg ggtgaaggtt 2160
gtcgaagaga aggccttttc tcctgaggtt atccccatgt tctccgcttt gagtgagggg 2220
gccactcctc aggacctcaa tacaatgctt aataccgtgg gcggccatca ggccgccatg 2280
caaatgttga aggagactat caacgaggag gcagccgagt gggacagagt gcatcccgtc 2340
cacgctggcc caatcgcgcc cggacagatg cgggagcctc gcggctctga cattgccggc 2400
accacctcta cactgcaaga gcaaatcgga tggatgacca acaatcctcc catcccagtt 2460
ggagaaatct ataaacggtg gatcatcctg ggcctgaaca agatcgtgcg catgtactct 2520
ccgacatcca tccttgacat tagacaggga cccaaagagc cttttaggga ttacgtcgac 2580
cggttttata agaccctgcg agcagagcag gcctctcagg aggtcaaaaa ctggatgacg 2640
gagacactcc tggtacagaa cgctaacccc gactgcaaaa caatcttgaa ggcactaggc 2700
ccggctgcca ccctggaaga gatgatgacc gcctgtcagg gagtaggcgg acccggacac 2760
aaagccagag tgttgatggt gggttttcca gtcacacctc aggtaccttt aagaccaatg 2820
acttacaagg cagctgtaga tcttagccac tttttaaaag aaaagggggg actggaaggg 2880
ctaattcact cccaaagaag acaagatatc cttgatctgt ggatctacca cacacaaggc 2940
tacttccctg attggcagaa ctacacacca gggccagggg tcagatatcc actgaccttt 3000
ggatggtgct acaagctagt accagttgag ccagataagg tagaagaggc caataaagga 3060
gagaacacca gcttgttaca ccctgtgagc ctgcatggga tggatgaccc ggagagagaa 3120
gtgttagagt ggaggtttga cagccgccta gcatttcatc acgtggcccg agagctgcat 3180
ccggagtact tcaagaactg ctga 3204

<210> 80
<211> 1067
<212> PRT
<213> HIV

<400> 80
Met Gly Pro Ile Ser Pro Ile Glu Thr Val Ser Val Lys Leu Lys Pro
1               5                   10                  15
Gly Met Asp Gly Pro Lys Val Lys Gln Trp Pro Leu Thr Glu Glu Lys
            20                  25                  30
Ile Lys Ala Leu Val Glu Ile Cys Thr Glu Met Glu Lys Glu Gly Lys
        35                  40                  45
Ile Ser Lys Ile Gly Pro Glu Asn Pro Tyr Asn Thr Pro Val Phe Ala
    50                  55                  60
Ile Lys Lys Lys Asp Ser Thr Lys Trp Arg Lys Leu Val Asp Phe Arg
65                  70                  75                  80
Glu Leu Asn Lys Arg Thr Gln Asp Phe Trp Glu Val Gln Leu Gly Ile
                85                  90                  95
Pro His Pro Ala Gly Leu Lys Lys Lys Lys Ser Val Thr Val Leu Asp
            100                 105                 110
Val Gly Asp Ala Tyr Phe Ser Val Pro Leu Asp Glu Asp Phe Arg Lys
        115                 120                 125
Tyr Thr Ala Phe Thr Ile Pro Ser Ile Asn Asn Glu Thr Pro Gly Ile
    130                 135                 140
Arg Tyr Gln Tyr Asn Val Leu Pro Gln Gly Trp Lys Gly Ser Pro Ala
145                 150                 155                 160
Ile Phe Gln Ser Ser Met Thr Lys Ile Leu Glu Pro Phe Arg Lys Gln
                165                 170                 175
Asn Pro Asp Ile Val Ile Tyr Gln Tyr Met Asp Asp Leu Tyr Val Gly
            180                 185                 190
Ser Asp Leu Glu Ile Gly Gln His Arg Thr Lys Ile Glu Glu Leu Arg
        195                 200                 205
Gln His Leu Leu Arg Trp Gly Leu Thr Thr Pro Asp Lys Lys His Gln
    210                 215                 220
Lys Glu Pro Pro Phe Leu Trp Met Gly Tyr Glu Leu His Pro Asp Lys
225                 230                 235                 240
Trp Thr Val Gln Pro Ile Val Leu Pro Glu Lys Asp Ser Trp Thr Val
                245                 250                 255
Asn Asp Ile Gln Lys Leu Val Gly Lys Leu Asn Trp Ala Ser Gln Ile
            260                 265                 270
Tyr Pro Gly Ile Lys Val Arg Gln Leu Cys Lys Leu Leu Arg Gly Thr
        275                 280                 285
Lys Ala Leu Thr Glu Val Ile Pro Leu Thr Glu Glu Ala Glu Leu Glu
    290                 295                 300
Leu Ala Glu Asn Arg Glu Ile Leu Lys Glu Pro Val His Gly Val Tyr
305                 310                 315                 320
Tyr Asp Pro Ser Lys Asp Leu Ile Ala Glu Ile Gln Lys Gln Gly Gln
                325                 330                 335
Gly Gln Trp Thr Tyr Gln Ile Tyr Gln Glu Pro Phe Lys Asn Leu Lys
            340                 345                 350
Thr Gly Lys Tyr Ala Arg Met Arg Gly Ala His Thr Asn Asp Val Lys
        355                 360                 365

Gln Leu Thr Glu Ala Val Gln Lys Ile Thr Thr Glu Ser Ile Val Ile
        370                 375                 380
Trp Gly Lys Thr Pro Lys Phe Lys Leu Pro Ile Gln Lys Glu Thr Trp
385                 390                 395                 400
Glu Thr Trp Trp Thr Glu Tyr Trp Gln Ala Thr Trp Ile Pro Glu Trp
                405                 410                 415
Glu Phe Val Asn Thr Pro Pro Leu Val Lys Leu Trp Tyr Gln Leu Glu
            420                 425                 430
Lys Glu Pro Ile Val Gly Ala Glu Thr Phe Tyr Val Asp Gly Ala Ala
        435                 440                 445
Asn Arg Glu Thr Lys Leu Gly Lys Ala Gly Tyr Val Thr Asn Arg Gly
    450                 455                 460
Arg Gln Lys Val Val Thr Leu Thr Asp Thr Thr Asn Gln Lys Thr Glu
465                 470                 475                 480
Leu Gln Ala Ile Tyr Leu Ala Leu Gln Asp Ser Gly Leu Glu Val Asn
                485                 490                 495
Ile Val Thr Asp Ser Gln Tyr Ala Leu Gly Ile Ile Gln Ala Gln Pro
            500                 505                 510
Asp Gln Ser Glu Ser Glu Leu Val Asn Gln Ile Ile Glu Gln Leu Ile
        515                 520                 525
Lys Lys Glu Lys Val Tyr Leu Ala Trp Val Pro Ala His Lys Gly Ile
    530                 535                 540
Gly Gly Asn Glu Gln Val Asp Lys Leu Val Ser Ala Gly Ile Arg Lys
545                 550                 555                 560
Val Leu Met Gly Ala Arg Ala Ser Val Leu Ser Gly Gly Glu Leu Asp
                565                 570                 575
Arg Trp Glu Lys Ile Arg Leu Arg Pro Gly Gly Lys Lys Lys Tyr Lys
            580                 585                 590
Leu Lys His Ile Val Trp Ala Ser Arg Glu Leu Glu Arg Phe Ala Val
        595                 600                 605
Asn Pro Gly Leu Leu Glu Thr Ser Glu Gly Cys Arg Gln Ile Leu Gly
    610                 615                 620
Gln Leu Gln Pro Ser Leu Gln Thr Gly Ser Glu Glu Leu Arg Ser Leu
625                 630                 635                 640
Tyr Asn Thr Val Ala Thr Leu Tyr Cys Val His Gln Arg Ile Glu Ile
                645                 650                 655
Lys Asp Thr Lys Glu Ala Leu Asp Lys Ile Glu Glu Glu Gln Asn Lys
            660                 665                 670
Ser Lys Lys Lys Ala Gln Gln Ala Ala Ala Asp Thr Gly His Ser Asn
        675                 680                 685
Gln Val Ser Gln Asn Tyr Pro Ile Val Gln Asn Ile Gln Gly Gln Met
    690                 695                 700
Val His Gln Ala Ile Ser Pro Arg Thr Leu Asn Ala Trp Val Lys Val
705                 710                 715                 720
Val Glu Glu Lys Ala Phe Ser Pro Glu Val Ile Pro Met Phe Ser Ala
                725                 730                 735
Leu Ser Glu Gly Ala Thr Pro Gln Asp Leu Asn Thr Met Leu Asn Thr
            740                 745                 750
Val Gly Gly His Gln Ala Ala Met Gln Met Leu Lys Glu Thr Ile Asn
        755                 760                 765
Glu Glu Ala Ala Glu Trp Asp Arg Val His Pro Val His Ala Gly Pro
    770                 775                 780
Ile Ala Pro Gly Gln Met Arg Glu Pro Arg Gly Ser Asp Ile Ala Gly
785                 790                 795                 800
Thr Thr Ser Thr Leu Gln Glu Gln Ile Gly Trp Met Thr Asn Asn Pro
                805                 810                 815
Pro Ile Pro Val Gly Glu Ile Tyr Lys Arg Trp Ile Ile Leu Gly Leu
            820                 825                 830
Asn Lys Ile Val Arg Met Tyr Ser Pro Thr Ser Ile Leu Asp Ile Arg
        835                 840                 845
Gln Gly Pro Lys Glu Pro Phe Arg Asp Tyr Val Asp Arg Phe Tyr Lys
    850                 855                 860
Thr Leu Arg Ala Glu Gln Ala Ser Gln Glu Val Lys Asn Trp Met Thr
865                 870                 875                 880
Glu Thr Leu Leu Val Gln Asn Ala Asn Pro Asp Cys Lys Thr Ile Leu
                885                 890                 895
Lys Ala Leu Gly Pro Ala Ala Thr Leu Glu Glu Met Met Thr Ala Cys
            900                 905                 910
Gln Gly Val Gly Gly Pro Gly His Lys Ala Arg Val Leu Met Val Gly
        915                 920                 925
Phe Pro Val Thr Pro Gln Val Pro Leu Arg Pro Met Thr Tyr Lys Ala
    930                 935                 940
Ala Val Asp Leu Ser His Phe Leu Lys Glu Lys Gly Gly Leu Glu Gly
945                 950                 955                 960
Leu Ile His Ser Gln Arg Arg Gln Asp Ile Leu Asp Leu Trp Ile Tyr
                965                 970                 975

His Thr Gln Gly Tyr Phe Pro Asp Trp Gln Asn Tyr Thr Pro Gly Pro
                980                 985                 990
Gly Val Arg Tyr Pro Leu Thr Phe Gly Trp Cys Tyr Lys Leu Val Pro
        995                 1000                1005
Val Glu Pro Asp Lys Val Glu Glu Ala Asn Lys Gly Glu Asn Thr Ser
    1010                1015                1020
Leu Leu His Pro Val Ser Leu His Gly Met Asp Asp Pro Glu Arg Glu
1025                1030                1035                1040
Val Leu Glu Trp Arg Phe Asp Ser Arg Leu Ala Phe His His Val Ala
                1045                1050                1055
Arg Glu Leu His Pro Glu Tyr Phe Lys Asn Cys
                1060                1065

<210> 81
<211> 3204
<212> DNA
<213> HIV

<400> 81
atgggcccca tcagtcccat cgagaccgtg ccggtgaagc tgaaacccgg gatggacggc 60
cccaaggtca agcagtggcc actcaccgag gagaagatca aggccctggt ggagatctgc 120
accgagatgg agaaagaggg caagatcagc aagatcgggc cggagaaccc atacaacacc 180
cccgtgtttg ccatcaagaa gaaggacagc accaagtggc gcaagctggt ggatttccgg 240
gagctgaata agcggaccca ggatttctgg gaggtccagc tgggcatccc ccatccggcc 300
ggcctgaaga agaagaagag cgtgaccgtg ctggacgtgg gcgacgctta cttcagcgtc 360
cctctggacg aggactttag aaagtacacc gcctttacca tcccatctat caacaacgag 420
acccctggca tcagatatca gtacaacgtc ctcccccagg gctggaaggg ctctcccgcc 480
attttccaga gctccatgac caagatcctg gagccgtttc ggaagcagaa ccccgatatc 540
gtcatctacc agtacatgga cgacctgtac gtgggctctg acctggaaat cgggcagcat 600
cgcacgaaga ttgaggagct gaggcagcat ctgctgagat ggggcctgac cactccggac 660
aagaagcatc agaaggagcc gccattcctg aagatgggct acgagctcca tcccgacaag 720
tggaccgtgc agcctatcgt cctccccgag aaggacagct ggaccgtgaa cgacatccag 780
aagctggtgg gcaagctcaa ctgggctagc cagatctatc ccgggatcaa ggtgcgccag 840
ctctgcaagc tgctgcgcgg caccaaggcc ctgaccgagg tgattcccct cacggaggaa 900
gccgagctcg agctggctga gaaccgggag atcctgaagg agcccgtgca cggcgtgtac 960
tatgacccct ccaaggacct gatcgccgaa atccagaagc agggccaggg gcagtggaca 1020
taccagattt accaggagcc tttcaagaac ctcaagaccg gcaagtacgc ccgcatgagg 1080
ggcgcccaca ccaacgatgt caagcagctg accgaggccg tccagaagat cacgaccgag 1140
tccatcgtga tctgggggaa gacacccaag ttcaagctgc ctatccagaa ggagacctgg 1200
gagacgtggt ggaccgaata ttggcaggcc acctggattc ccgagtggga gttcgtgaat 1260
acacctcctc tggtgaagct gtggtaccag ctcgagaagg agcccatcgt gggcgcggag 1320
acattctacg tggacggcgc ggccaaccgc gaaacaaagc tcgggaaggc cgggtacgtc 1380
accaaccggg gccgccagaa ggtcgtcacc ctgaccgaca ccaccaacca gaagacggag 1440
ctgcaggcca tctatctcgc tctccaggac tccggcctgg aggtgaacat cgtgacggac 1500
agccagtacg cgctgggcat tattcaggcc cagccggacc agtccgagag cgaactggtg 1560
aaccagatta tcgagcagct gatcaagaaa gagaaggtct acctcgcctg ggtcccggcc 1620
cataaggcca ttggcggcaa cgagcaggtc gacaagctgg tgagtgcggg gattagaaag 1680
gtgctgatgg tgggttttcc agtcacacct caggtacctt taagaccaat gacttacaag 1740
gcagctgtag atcttagcca ctttttaaaa gaaaaggggg gactggaagg gctaattcac 1800
tcccaaagaa gacaagatat ccttgatctg tggatctacc acacacaagg ctacttccct 1860
gattggcaga actacacacc agggccaggg gtcagatatc cactgacctt tggatggtgc 1920
tacaagctag taccagttga gccagataag gtagaagagg ccaataaagg agagaacacc 1980
agcttgttac accctgtgag cctgcatggg atggatgacc cggagagaga agtgttagag 2040
tggaggtttg acagccgcct agcatttcat cacgtggccc gagagctgca tccggagtac 2100
ttcaagaact gctgaatggg tgcccgagct tcggtactgt ctggtggaga gctggacaga 2160
tgggagaaaa ttaggctgcg cccgggaggc aaaaagaaat acaagctcaa gcatatcgtg 2220
tgggcctcga gggagcttga acggtttgcc gtgaacccag gcctgctgga aacatctgag 2280
ggatgtcgcc agatcctggg gcaattgcag ccatccctcc agaccgggag tgaagagctg 2340
aggtccttgt ataacacagt ggctaccctc tactgcgtac accagaggat cgagattaag 2400
gataccaagg aggccttgga caaaattgag gaggagcaaa acaagagcaa gaagaaggcc 2460
cagcaggcag ctgctgacac tgggcatagc aaccaggtat cacagaacta tcctattgtc 2520
caaaacattc agggccagat ggttcatcag gccatcagcc cccggacgct caatgcctgg 2580
gtgaaggttg tcgaagagaa ggccttttct cctgaggtta tccccatgtt ctccgctttg 2640
agtgaggggg ccactcctca ggacctcaat acaatgctta ataccgtggg cggccatcag 2700
gccgccatgc aaatgttgaa ggagactatc aacgaggagg cagccgagtg ggacagagtg 2760
catcccgtcc acgctggccc aatcgcgccc ggacagatgc gggagcctcg cggctctgac 2820
attgccggca ccacctctac actgcaagag caaatcggat ggatgaccaa caatcctccc 2880
atcccagttg gagaaatcta taaacggtgg atcatcctgg gcctgaacaa gatcgtgcgc 2940
atgtactctc cgacatccat ccttgacatt agacagggac ccaaagagcc ttttagggat 3000
tacgtcgacc ggttttataa gaccctgcga gcagagcagg cctctcagga ggtcaaaaac 3060
tggatgacgg agacactcct ggtacagaac gctaaccccg actgcaaaac aatcttgaag 3120
gcactaggcc cggctgccac cctggaagag atgatgaccg cctgtcaggg agtaggcgga 3180
cccggacaca aagccagagt gttg 3204

<210> 82

<211> 1067
<212> PRT
<213> HIV

<400> 82
Met Gly Pro Ile Ser Pro Ile Glu Thr Val Ser Val Lys Leu Lys Pro
1               5                   10                  15
Gly Met Asp Gly Pro Lys Val Lys Gln Trp Pro Leu Thr Glu Glu Lys
            20                  25                  30
Ile Lys Ala Leu Val Glu Ile Cys Thr Glu Met Glu Lys Glu Gly Lys
        35                  40                  45
Ile Ser Lys Ile Gly Pro Glu Asn Pro Tyr Asn Thr Pro Val Phe Ala
    50                  55                  60
Ile Lys Lys Lys Asp Ser Thr Lys Trp Arg Lys Leu Val Asp Phe Arg
65                  70                  75                  80
Glu Leu Asn Lys Arg Thr Gln Asp Phe Trp Glu Val Gln Leu Gly Ile
                85                  90                  95
Pro His Pro Ala Gly Leu Lys Lys Lys Lys Ser Val Thr Val Leu Asp
            100                 105                 110
Val Gly Asp Ala Tyr Phe Ser Val Pro Leu Asp Glu Asp Phe Arg Lys
        115                 120                 125
Tyr Thr Ala Phe Thr Ile Pro Ser Ile Asn Asn Glu Thr Pro Gly Ile
    130                 135                 140
Arg Tyr Gln Tyr Asn Val Leu Pro Gln Gly Trp Lys Gly Ser Pro Ala
145                 150                 155                 160
Ile Phe Gln Ser Ser Met Thr Lys Ile Leu Glu Pro Phe Arg Lys Gln
                165                 170                 175
Asn Pro Asp Ile Val Ile Tyr Gln Tyr Met Asp Asp Leu Tyr Val Gly
            180                 185                 190
Ser Asp Leu Glu Ile Gly Gln His Arg Thr Lys Ile Glu Glu Leu Arg
        195                 200                 205
Gln His Leu Leu Arg Trp Gly Leu Thr Thr Pro Asp Lys Lys His Gln
    210                 215                 220
Lys Glu Pro Pro Phe Leu Trp Met Gly Tyr Glu Leu His Pro Asp Lys
225                 230                 235                 240
Trp Thr Val Gln Pro Ile Val Leu Pro Glu Lys Asp Ser Trp Thr Val
                245                 250                 255
Asn Asp Ile Gln Lys Leu Val Gly Lys Leu Asn Trp Ala Ser Gln Ile
            260                 265                 270
Tyr Pro Gly Ile Lys Val Arg Gln Leu Cys Lys Leu Leu Arg Gly Thr
        275                 280                 285
Lys Ala Leu Thr Glu Val Ile Pro Leu Thr Glu Glu Ala Glu Leu Glu
    290                 295                 300
Leu Ala Glu Asn Arg Glu Ile Leu Lys Glu Pro Val His Gly Val Tyr
305                 310                 315                 320
Tyr Asp Pro Ser Lys Asp Leu Ile Ala Glu Ile Gln Lys Gln Gly Gln
                325                 330                 335
Gly Gln Trp Thr Tyr Gln Ile Tyr Gln Glu Pro Phe Lys Asn Leu Lys
            340                 345                 350
Thr Gly Lys Tyr Ala Arg Met Arg Gly Ala His Thr Asn Asp Val Lys
        355                 360                 365
Gln Leu Thr Glu Ala Val Gln Lys Ile Thr Thr Glu Ser Ile Val Ile
    370                 375                 380
Trp Gly Lys Thr Pro Lys Phe Lys Leu Pro Ile Gln Lys Glu Thr Trp
385                 390                 395                 400
Glu Thr Trp Trp Thr Glu Tyr Trp Gln Ala Thr Trp Ile Pro Glu Trp
                405                 410                 415
Glu Phe Val Asn Thr Pro Pro Leu Val Lys Leu Trp Tyr Gln Leu Glu
            420                 425                 430
Lys Glu Pro Ile Val Gly Ala Glu Thr Phe Tyr Val Asp Gly Ala Ala
        435                 440                 445
Asn Arg Glu Thr Lys Leu Gly Lys Ala Gly Tyr Val Thr Asn Arg Gly
    450                 455                 460
Arg Gln Lys Val Val Thr Leu Thr Asp Thr Thr Asn Gln Lys Thr Glu
465                 470                 475                 480
Leu Gln Ala Ile Tyr Leu Ala Leu Gln Asp Ser Gly Leu Glu Val Asn
                485                 490                 495
Ile Val Thr Asp Ser Gln Tyr Ala Leu Gly Ile Ile Gln Ala Gln Pro
            500                 505                 510
Asp Gln Ser Glu Ser Glu Leu Val Asn Gln Ile Ile Glu Gln Leu Ile
        515                 520                 525
Lys Lys Glu Lys Val Tyr Leu Ala Trp Val Pro Ala His Lys Gly Ile
    530                 535                 540
Gly Gly Asn Glu Gln Val Asp Lys Leu Val Ser Ala Gly Ile Arg Lys
545                 550                 555                 560
Val Leu Met Val Gly Phe Pro Val Thr Pro Gln Val Pro Leu Arg Pro

                565                 570                 575
Met Thr Tyr Lys Ala Ala Val Asp Leu Ser His Phe Leu Lys Glu Lys
            580                 585                 590
Gly Gly Leu Glu Gly Leu Ile His Ser Gln Arg Arg Gln Asp Ile Leu
        595                 600                 605
Asp Leu Trp Ile Tyr His Thr Gln Gly Tyr Phe Pro Asp Trp Gln Asn
    610                 615                 620
Tyr Thr Pro Gly Pro Gly Val Arg Tyr Pro Leu Thr Phe Gly Trp Cys
625                 630                 635                 640
Tyr Lys Leu Val Pro Val Glu Pro Asp Lys Val Glu Glu Ala Asn Lys
                645                 650                 655
Gly Glu Asn Thr Ser Leu Leu His Pro Val Ser Leu His Gly Met Asp
            660                 665                 670
Asp Pro Glu Arg Glu Val Leu Glu Trp Arg Phe Asp Ser Arg Leu Ala
        675                 680                 685
Phe His His Val Ala Arg Glu Leu His Pro Glu Tyr Phe Lys Asn Cys
    690                 695                 700
Met Gly Ala Arg Ala Ser Val Leu Ser Gly Gly Glu Leu Asp Arg Trp
705                 710                 715                 720
Glu Lys Ile Arg Leu Arg Pro Gly Gly Lys Lys Lys Tyr Lys Leu Lys
                725                 730                 735
His Ile Val Trp Ala Ser Arg Glu Leu Glu Arg Phe Ala Val Asn Pro
            740                 745                 750
Gly Leu Leu Glu Thr Ser Glu Gly Cys Arg Gln Ile Leu Gly Gln Leu
        755                 760                 765
Gln Pro Ser Leu Gln Thr Gly Ser Glu Glu Leu Arg Ser Leu Tyr Asn
    770                 775                 780
Thr Val Ala Thr Leu Tyr Cys Val His Gln Arg Ile Glu Ile Lys Asp
785                 790                 795                 800
Thr Lys Glu Ala Leu Asp Lys Ile Glu Glu Glu Gln Asn Lys Ser Lys
                805                 810                 815
Lys Lys Ala Gln Gln Ala Ala Ala Asp Thr Gly His Ser Asn Gln Val
            820                 825                 830
Ser Gln Asn Tyr Pro Ile Val Gln Asn Ile Gln Gly Gln Met Val His
        835                 840                 845
Gln Ala Ile Ser Pro Arg Thr Leu Asn Ala Trp Val Lys Val Val Glu
    850                 855                 860
Glu Lys Ala Phe Ser Pro Glu Val Ile Pro Met Phe Ser Ala Leu Ser
865                 870                 875                 880
Glu Gly Ala Thr Pro Gln Asp Leu Asn Thr Met Leu Asn Thr Val Gly
                885                 890                 895
Gly His Gln Ala Ala Met Gln Met Leu Lys Glu Thr Ile Asn Glu Glu
            900                 905                 910
Ala Ala Glu Trp Asp Arg Val His Pro Val His Ala Gly Pro Ile Ala
        915                 920                 925
Pro Gly Gln Met Arg Glu Pro Arg Gly Ser Asp Ile Ala Gly Thr Thr
    930                 935                 940
Ser Thr Leu Gln Glu Gln Ile Gly Trp Met Thr Asn Asn Pro Pro Ile
945                 950                 955                 960
Pro Val Gly Glu Ile Tyr Lys Arg Trp Ile Ile Leu Gly Leu Asn Lys
                965                 970                 975
Ile Val Arg Met Tyr Ser Pro Thr Ser Ile Leu Asp Ile Arg Gln Gly
            980                 985                 990
Pro Lys Glu Pro Phe Arg Asp Tyr Val Asp Arg Phe Tyr Lys Thr Leu
        995                 1000                1005
Arg Ala Glu Gln Ala Ser Gln Glu Val Lys Asn Trp Met Thr Glu Thr
    1010                1015                1020
Leu Leu Val Gln Asn Ala Asn Pro Asp Cys Lys Thr Ile Leu Lys Ala
1025                1030                1035                1040
Leu Gly Pro Ala Ala Thr Leu Glu Glu Met Met Thr Ala Cys Gln Gly
                1045                1050                1055
Val Gly Gly Pro Gly His Lys Ala Arg Val Leu
            1060                1065

<210> 83
<211> 3204
<212> DNA
<213> HIV

<400> 83
atgggtgccc gagcttcggt actgtctggt ggagagctgg acagatggga gaaaattagg 60
ctgcgcccgg gaggcaaaaa gaaatacaag ctcaagcata tcgtgtgggc ctcgagggag 120
cttgaacggt ttgccgtgaa cccaggcctg ctggaaacat ctgagggatg tcgccagatc 180
ctggggcaat tgcagccatc cctccagacc gggagtgaag agctgaggtc cttgtataac 240
acagtggcta ccctctactg cgtacaccag aggatcgaga ttaaggatac caaggaggcc 300

ttggacaaaa ttgaggagga gcaaaacaag agcaagaaga aggcccagca ggcagctgct 360
gacactgggc atagcaacca ggtatcacag aactatccta ttgtccaaaa cattcagggc 420
cagatggttc atcaggccat cagcccccgg acgctcaatg cctgggtgaa ggttgtcgaa 480
gagaaggcct tttctcctga ggttatcccc atgttctccg ctttgagtga gggggccact 540
cctcaggacc tcaatacaat gcttaatacc gtgggcggcc atcaggccgc catgcaaatg 600
ttgaaggaga ctatcaacga ggaggcagcc gagtgggaca gagtgcatcc cgtccacgct 660
ggcccaatcg cgcccggaca gatgcgggag cctcgcggct ctgacattgc cggcaccacc 720
tctacactgc aagagcaaat cggatggatg accaacaatc ctcccatccc agttggagaa 780
atctataaac ggtggatcat cctgggcctg aacaagatcg tgcgcatgta ctctccgaca 840
tccatccttg acattagaca gggacccaaa gagcctttta gggattacgt cgaccggttt 900
tataagaccc tgcgagcaga gcaggcctct caggaggtca aaaactggat gacggagaca 960
ctcctggtac agaacgctaa ccccgactgc aaaacaatct tgaaggcact aggcccggct 1020
gccaccctgg aagagatgat gaccgcctgt cagggagtag gcggacccgg acacaaagcc 1080
agagtgttga tggtgggttt tccagtcaca cctcaggtac ctttaagacc aatgacttac 1140
aaggcagctg tagatcttag ccacttttta aaagaaaagg ggggactgga agggctaatt 1200
cactcccaaa gaagacaaga tatccttgat ctgtggatct accacacaca aggctacttc 1260
cctgattggc agaactacac accagggcca ggggtcagat atccactgac ctttggatgg 1320
tgctacaagc tagtaccagt tgagccagat aaggtagaag aggccaataa aggagagaac 1380
accagcttgt tacaccctgt gagcctgcat gggatggatg acccggagag agaagtgtta 1440
gagtggaggt ttgacagccg cctagcattt catcacgtgg cccgagagct gcatccggag 1500
tacttcaaga actgcatggg ccccatcagt cccatcgaga ccgtgccggt gaagctgaaa 1560
cccgggatgg acggccccaa ggtcaagcag tggccactca ccgaggagaa gatcaaggcc 1620
ctggtggaga tctgcaccga gatggagaaa gagggcaaga tcagcaagat cgggcctgag 1680
aacccataca acacccccgt gtttgccatc aagaagaagg acagcaccaa gtggcgcaag 1740
ctggtggatt tccgggagct gaataagcgg acccaggatt tctgggaggt ccagctgggc 1800
atcccccatc cggccggcct gaagaagaag aagagcgtga ccgtgctgga cgtgggcgac 1860
gcttacttca gcgtccctct ggacgaggac tttagaaagt acaccgcctt taccatccca 1920
tctatcaaca acgagacccc tggcatcaga tatcagtaca acgtcctccc ccagggctgg 1980
aagggctctc ccgccatttt ccagagctcc atgaccaaga tcctggagcc gtttcggaag 2040
cagaaccccg atatcgtcat ctaccagtac atggacgacc tgtacgtggg ctctgacctg 2100
gaaatcgggc agcatcgcac gaagattgag gagctgaggc agcatctgct gagatggggc 2160
ctgaccactc cggacaagaa gcatcagaag gagccgccat tcctgaagat gggctacgag 2220
ctccatcccg acaagtggac cgtgcagcct atcgtcctcc ccgagaagga cagctggacc 2280
gtgaacgaca tccagaagct ggtgggcaag ctcaactggg ctagccagat ctatcccggg 2340
atcaaggtgc gccagctctg caagctgctg cgcggcacca aggccctgac cgaggtgatt 2400
cccctcacgg aggaagccga gctcgagctg gctgagaacc gggagatcct gaaggagccc 2460
gtgcacggcg tgtactatga cccctccaag gacctgatcg ccgaaatcca gaagcagggc 2520
caggggcagt ggacatacca gatttaccag gagcctttca agaacctcaa gaccggcaag 2580
tacgcccgca tgaggggcgc ccacaccaac gatgtcaagc agctgaccga ggccgtccag 2640
aagatcacga ccgagtccat cgtgatctgg gggaagacac ccaagttcaa gctgcctatc 2700
cagaaggaga cctgggagac gtggtggacc gaatattggc aggccacctg gattcccgag 2760
tgggagttcg tgaatacacc tcctctggtg aagctgtggt accagctcga gaaggagccc 2820
atcgtgggcg cggagacatt ctacgtggac ggcgcggcca accgcgaaac aaagctcggg 2880
aaggccgggt acgtcaccaa ccggggccgc cagaaggtcg tcaccctgac cgacaccacc 2940
aaccagaaga cggagctgca ggccatctat ctcgctctcc aggactccgg cctggaggtg 3000
aacatcgtga cggacagcca gtacgcgctg ggcattattc aggcccagcc ggaccagtcc 3060
gagagcgaac tggtgaacca gattatcgag cagctgatca agaaagagaa ggtctacctc 3120
gcctgggtcc cggcccataa gggcattggc ggcaacgagc aggtcgacaa gctggtgagt 3180
gcggggatta gaaaggtgct gtaa 3204

<210> 84
<211> 1067
<212> PRT
<213> HIV

<400> 84
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Asn Gln Val
        115                 120                 125
Ser Gln Asn Tyr Pro Ile Val Gln Asn Ile Gln Gly Gln Met Val His
    130                 135                 140
Gln Ala Ile Ser Pro Arg Thr Leu Asn Ala Trp Val Lys Val Val Glu
145                 150                 155                 160

Glu Lys Ala Phe Ser Pro Glu Val Ile Pro Met Phe Ser Ala Leu Ser
                165                 170                 175
Glu Gly Ala Thr Pro Gln Asp Leu Asn Thr Met Leu Asn Thr Val Gly
            180                 185                 190
Gly His Gln Ala Ala Met Gln Met Leu Lys Glu Thr Ile Asn Glu Glu
        195                 200                 205
Ala Ala Glu Trp Asp Arg Val His Pro Val His Ala Gly Pro Ile Ala
    210                 215                 220
Pro Gly Gln Met Arg Glu Pro Arg Gly Ser Asp Ile Ala Gly Thr Thr
225                 230                 235                 240
Ser Thr Leu Gln Glu Gln Ile Gly Trp Met Thr Asn Asn Pro Pro Ile
                245                 250                 255
Pro Val Gly Glu Ile Tyr Lys Arg Trp Ile Ile Leu Gly Leu Asn Lys
            260                 265                 270
Ile Val Arg Met Tyr Ser Pro Thr Ser Ile Leu Asp Ile Arg Gln Gly
        275                 280                 285
Pro Lys Glu Pro Phe Arg Asp Tyr Val Asp Arg Phe Tyr Lys Thr Leu
    290                 295                 300
Arg Ala Glu Gln Ala Ser Gln Glu Val Lys Asn Trp Met Thr Glu Thr
305                 310                 315                 320
Leu Leu Val Gln Asn Ala Asn Pro Asp Cys Lys Thr Ile Leu Lys Ala
                325                 330                 335
Leu Gly Pro Ala Ala Thr Leu Glu Glu Met Met Thr Ala Cys Gln Gly
            340                 345                 350
Val Gly Gly Pro Gly His Lys Ala Arg Val Leu Met Val Gly Phe Pro
        355                 360                 365
Val Thr Pro Gln Val Pro Leu Arg Pro Met Thr Tyr Lys Ala Ala Val
    370                 375                 380
Asp Leu Ser His Phe Leu Lys Glu Lys Gly Gly Leu Glu Gly Leu Ile
385                 390                 395                 400
His Ser Gln Arg Arg Gln Asp Ile Leu Asp Leu Trp Ile Tyr His Thr
                405                 410                 415
Gln Gly Tyr Phe Pro Asp Trp Gln Asn Tyr Thr Pro Gly Pro Gly Val
            420                 425                 430
Arg Tyr Pro Leu Thr Phe Gly Trp Cys Tyr Lys Leu Val Pro Val Glu
        435                 440                 445
Pro Asp Lys Val Glu Glu Ala Asn Lys Gly Glu Asn Thr Ser Leu Leu
    450                 455                 460
His Pro Val Ser Leu His Gly Met Asp Asp Pro Glu Arg Glu Val Leu
465                 470                 475                 480
Glu Trp Arg Phe Asp Ser Arg Leu Ala Phe His His Val Ala Arg Glu
                485                 490                 495
Leu His Pro Glu Tyr Phe Lys Asn Cys Met Gly Pro Ile Ser Pro Ile
            500                 505                 510
Glu Thr Val Ser Val Lys Leu Lys Pro Gly Met Asp Gly Pro Lys Val
        515                 520                 525
Lys Gln Trp Pro Leu Thr Glu Glu Lys Ile Lys Ala Leu Val Glu Ile
    530                 535                 540
Cys Thr Glu Met Glu Lys Glu Gly Lys Ile Ser Lys Ile Gly Pro Glu
545                 550                 555                 560
Asn Pro Tyr Asn Thr Pro Val Phe Ala Ile Lys Lys Lys Asp Ser Thr
                565                 570                 575
Lys Trp Arg Lys Leu Val Asp Phe Arg Glu Leu Asn Lys Arg Thr Gln
            580                 585                 590
Asp Phe Trp Glu Val Gln Leu Gly Ile Pro His Pro Ala Gly Leu Lys
        595                 600                 605
Lys Lys Lys Ser Val Thr Val Leu Asp Val Gly Asp Ala Tyr Phe Ser
    610                 615                 620
Val Pro Leu Asp Glu Asp Phe Arg Lys Tyr Thr Ala Phe Thr Ile Pro
625                 630                 635                 640
Ser Ile Asn Asn Glu Thr Pro Gly Ile Arg Tyr Gln Tyr Asn Val Leu
                645                 650                 655
Pro Gln Gly Trp Lys Gly Ser Pro Ala Ile Phe Gln Ser Ser Met Thr
            660                 665                 670
Lys Ile Leu Glu Pro Phe Arg Lys Gln Asn Pro Asp Ile Val Ile Tyr
        675                 680                 685
Gln Tyr Met Asp Asp Leu Tyr Val Gly Ser Asp Leu Glu Ile Gly Gln
    690                 695                 700
His Arg Thr Lys Ile Glu Glu Leu Arg Gln His Leu Leu Arg Trp Gly
705                 710                 715                 720
Leu Thr Thr Pro Asp Lys Lys His Gln Lys Glu Pro Pro Phe Leu Trp
                725                 730                 735
Met Gly Tyr Glu Leu His Pro Asp Lys Trp Thr Val Gln Pro Ile Val
            740                 745                 750
Leu Pro Glu Lys Asp Ser Trp Thr Val Asn Asp Ile Gln Lys Leu Val
        755                 760                 765

Gly Lys Leu Asn Trp Ala Ser Gln Ile Tyr Pro Gly Ile Lys Val Arg
    770                 775                 780
Gln Leu Cys Lys Leu Leu Arg Gly Thr Lys Ala Leu Thr Glu Val Ile
785                 790                 795                 800
Pro Leu Thr Glu Glu Ala Glu Leu Glu Leu Ala Glu Asn Arg Glu Ile
                805                 810                 815
Leu Lys Glu Pro Val His Gly Val Tyr Tyr Asp Pro Ser Lys Asp Leu
            820                 825                 830
Ile Ala Glu Ile Gln Lys Gln Gly Gln Gly Gln Trp Thr Tyr Gln Ile
        835                 840                 845
Tyr Gln Glu Pro Phe Lys Asn Leu Lys Thr Gly Lys Tyr Ala Arg Met
    850                 855                 860
Arg Gly Ala His Thr Asn Asp Val Lys Gln Leu Thr Glu Ala Val Gln
865                 870                 875                 880
Lys Ile Thr Thr Glu Ser Ile Val Ile Trp Gly Lys Thr Pro Lys Phe
                885                 890                 895
Lys Leu Pro Ile Gln Lys Glu Thr Trp Glu Thr Trp Trp Thr Glu Tyr
            900                 905                 910
Trp Gln Ala Thr Trp Ile Pro Glu Trp Glu Phe Val Asn Thr Pro Pro
        915                 920                 925
Leu Val Lys Leu Trp Tyr Gln Leu Glu Lys Glu Pro Ile Val Gly Ala
    930                 935                 940
Glu Thr Phe Tyr Val Asp Gly Ala Ala Asn Arg Glu Thr Lys Leu Gly
945                 950                 955                 960
Lys Ala Gly Tyr Val Thr Asn Arg Gly Arg Gln Lys Val Val Thr Leu
                965                 970                 975
Thr Asp Thr Thr Asn Gln Lys Thr Glu Leu Gln Ala Ile Tyr Leu Ala
            980                 985                 990
Leu Gln Asp Ser Gly Leu Glu Val Asn Ile Val Thr Asp Ser Gln Tyr
        995                 1000                1005
Ala Leu Gly Ile Ile Gln Ala Gln Pro Asp Gln Ser Glu Ser Glu Leu
    1010                1015                1020
Val Asn Gln Ile Ile Glu Gln Leu Ile Lys Lys Glu Lys Val Tyr Leu
1025                1030                1035                1040
Ala Trp Val Pro Ala His Lys Gly Ile Gly Gly Asn Glu Gln Val Asp
                1045                1050                1055
Lys Leu Val Ser Ala Gly Ile Arg Lys Val Leu
            1060                1065